# EUROPEAN PATENT SPECIFICATION

(11) **EP 3 342 370 B1**
(45) Date of publication and mention of the grant of the patent: **14.10.2020**
(21) Application number: 16839364.3
(22) Date of filing: 26.08.2016
(51) Int. Cl.: A61C 13/01, A61C 13/113, A61C 13/007, A61C 13/00, A61C 19/04

(54) **REFERENCE DENTAL PLATE, REFERENCE DENTURE, DENTURE FABRICATION KIT AND DENTURE FABRICATION METHOD**
REFERENZZAHNPLATTE, REFERENZZAHNPROTHESE ZAHNPROTHESENHERSTELLUNGSKIT UND ZAHNPROTHESENHERSTELLUNGSVERFAHREN
PLAQUE DENTALE DE RÉFÉRENCE, PROTHÈSE DENTAIRE DE RÉFÉRENCE, TROUSSE DE FABRICATION DE PROTHÈSE DENTAIRE ET PROCÉDÉ DE FABRICATION DE PROTHÈSE DENTAIRE

(30) Priority: 27.08.2015 JP 2015167534; 27.08.2015 JP 2015167535; 31.08.2015 JP 2015169995; 31.08.2015 JP 2015169996; 31.08.2015 JP 2015169997; 31.08.2015 JP 2015169998
(43) Date of publication of application: 04.07.2018
(73) Proprietor: Tokuyama Dental Corporation, Tokyo 110-0016 (JP)
(72) Inventor: NAKASHIMA, Kei, Tokyo 110-0016 (JP); YAMAZAKI, Tatsuya, Tokyo 110-0016 (JP); GYAKUSHI, Ayumu, Tokyo 110-0016 (JP); HITOMI, Miki, Tokyo 110-0016 (JP); MATSUSHIGE, Koji, Tokyo 110-0016 (JP); HASHIGUCHI, Masanao, Tokyo 110-0016 (JP)
(74) Representative: Gulde & Partner
(86) International application number: PCT/JP2016/074934
(87) International publication number: WO 2017/034017

(56) References cited:
- DE-A1-102012 201 744
- JP-A- H05 192 353
- JP-A- 2001 276 098
- JP-A- 2003 079 645
- JP-U- 3 132 554
- JP-U- 3 172 628
- JP-U- S59 100 409
- US-A- 3 644 996
- US-A- 4 017 971
- KEIZO SUGIYAMA et al.: Kogosho no Seisaku, Koza Sika Giko Atlas, 10 September 1982 (1982-09-10), pages 241-249, XP009509004,
- SHOGO MINAGI et al.: "Measurement of Denture Base Size to Provide Standard Width for Occluso-Swallow Prosthesis and Masticato- Swallow Prosthesis : Proposition of the Standard Size of Lower Complete Denture Base for the Occlusal and Masticatory Functional Rehabilitation", Japanese Journal of Gerodontology, vol. 27, no. 1, 2012, pages 3-9, XP055365795,
- TADASHI SOTOKAWA: "Measurement of Distances between Anatomic Landmarks on Casts for Edentulous Patients : Trays Fitting to Edentulous Alveolus Ridges", The Journal of the Japan Prosthodontic Society, vol. 37, no. 3, 1993, pages 469-479, XP055365801,
- FUMIO NINOMIYA et al.: "A Study on the Dental Arch and Palate of a Population in Kyushu", Kyushu Shikaishi, vol. 22, no. 2, 1968, pages 119-136, XP009508856,
- DELLAVIA CLAUDIA et al.: "Three-dimensional hard tissue palatal size and shape in Down syndrome subjects", European Journal of Orthodontics, vol. 29, 2007, pages 417-422, XP055365808,

## Description

### Technical Field

The present invention relates to a ready-made standard dental plate and standard denture having a predetermined shape. The present invention also relates to a denture fabrication kit including the standard dental plate or the standard denture. The present invention further relates to a denture fabrication method using the denture fabrication kit.

### Background Art

Conventionally, a denture has been mainly fabricated by the following procedure:
(1) An impression material is used to take an impression in the oral cavity of a patient.
(2) The impression is used to fabricate a plaster model.
(3) A patient is fitted with a bite plate to take the interocclusal relation.
(4) An artificial tooth is arranged on the bite plate to fabricate a wax denture.
(5) A patient is made to conduct try-in of the wax denture to adjust dental occlusion.
(6) The wax denture is embedded into plaster to fabricate a template.
(7) Resin is packed into the template, and cured to fabricate a denture.

In this way, the denture is fabricated one by one by manual work in conformity with a shape of the oral cavity of a patient, and accordingly, an abundance of time is required, and the burden of a dental clinic, a dental technician's office, and a patient is large. Additionally, since a method of fabricating this denture has a plurality of times of a transfer step, an error of a shape is easily generated. Furthermore, quality easily varies depending on the skill of a dentist and a dental technician.

Patent Literature 1 discloses a method of fabricating a denture using a dental plate which has been manufactured using a mold or the like at a large scale. Patent Document 2 discloses a method of fabricating a denture using a prosthetic denture precursor in which at least one artificial tooth is arranged, for a dental plate member consisting of a plastic photopolymerizable composition. These methods can simplify a part of steps, but since it is still necessary to fabricate a plaster model, it is hardly said that the steps have been sufficiently simplified. Additionally, since a form of an alveolar crest of an edentulous jaw person is different for every patient, it is difficult to determine a shape of a dental plate in advance to manufacture the dental plate at a large scale.

Patent Literature 3 discloses a method of fabricating a denture using a standard dental plate which has been fabricated in advance, and has a concave part at an alveolar crest. However, Patent Literature 3 does not disclose a shape of the standard dental plate. Additionally, it is necessary to arrange an artificial tooth, and thus, the method is troublesome.

In the previous denture fabrication method, it is general that a denture is fabricated based on an interalveolar crest line rule, but the method is troublesome as described above. Additionally, in modern times when the population of patients has aged, when alveolar bone involution is remarkable, it becomes difficult to obtain the normal bite with the denture which has been fabricated by the above-mentioned method, and a problem such as cross bite has generated. Furthermore, in a patient of such case, an occlusal force per unit area which is loaded on an alveolar bone is great, and the alveolar ridge mucous membrane also becomes thin due to senile atrophy. For that reason, impact of a biting or chewing pressure is directly transmitted to the alveolar bone without being alleviated, and pain is generated in some cases. Then, in recent years, a method of fabricating a denture based on the denture space theory of determining a denture shape in conformity with the neutral zone of muscle pressure in which the dental arch is considered to exist in jaw with teeth has been proposed. However, in order to learn the method of fabricating the denture based on the denture space theory, an abundance of time is required. Additionally, even in the method of fabricating the denture based on the denture space theory, a denture fabrication method itself is troublesome as before. For that reason, a denture based on the denture space theory, which can be fabricated by a simple method has been demanded.

Patent Literature 4 discloses a workpiece system for a dental milling machine. The invention further relates to a workpiece holder of a workpiece system and a shaped body for processing by a dental milling machine and a set of a dental milling machine and a workpiece system or workpiece holder of the type mentioned.

Patent Literature 1: JP 6-63065 A
Patent Literature 2: JP 5-192353 A
Patent Literature 3: JP 4-218151 A
Patent Literature 4: DE102012201744 A1

### Summary of Invention

### Technical Problem

An object of the present invention is to provide a ready-made standard dental plate or standard denture which is adaptable to many patients. Another object thereof is to simplify a denture fabrication step to shorten the time for fabrication of a denture.

### Solution to Problem

In order to solve the above-mentioned problems, the present inventors conceived that a shape of a dental plate is determined based on an oral cavity shape of not only an edentulous jaw person but also a person having the jaw with teeth, and a dental plate is fabricated based on the shape. The present inventors thus found out that the dental plate of a predetermined shape which is fabricated by this method can be applied to many edentulous jaw persons. Further, the present inventors found out that by using a denture fabrication kit including this standard dental plate or standard denture, a denture fabrication step can be simplified while the high matching rate is maintained, resulting in completion of the present invention.

The present invention solving the above-mentioned problems is as described below. In addition, in the present description, a standard dental plate refers to a ready-made dental plate, or a dental plate in which at least one tooth has been arranged on the ready-made dental plate in advance, and a standard denture refers to a ready-made dental plate in which artificial teeth of a whole teeth row have been arranged in advance. Additionally, in the present description, a whole teeth row refers to a tooth row including 14 teeth from the left second molar to the right second molar.

A maxillary standard dental plate characterized in that when a length of a line segment PQ connecting a point P corresponding to the left pterygomaxillary notch and a point Q corresponding to the right pterygomaxillary notch of the plate posterior border is set to be 1, a length of a line segment OM connecting a point O corresponding to the superior labial frenulum which comes under the median of the labial denture border and a middle point M of the line segment PQ is 0.76 to 0.98, and
when respective points dividing a line segment OP connecting the point O and the point P into four equal portions are defined as a point P1, a point P2, and a point P3 from the point O side, points at which vertical lines from the point P1, the point P2, and the point P3 on the line segment OP are intersected with the left buccal denture border are defined as a point D1, a point D2, and a point D3,
respective points dividing a line segment OQ connecting the point O and the point Q into four equal portions are defined as a point Q1, a point Q2, and a point Q3 from the point O side, points at which vertical lines from the point Q1, the point Q2, and the point Q3 on the line segment OQ are intersected with the right buccal denture border are defined as a point E1, a point E2, and a point E3, and
lengths of a line segment P1D1 and a line segment Q1E1 are 0.11 to 0.36, respectively, lengths of a line segment P2D2 and a line segment Q2E2 are 0.19 to 0.45, respectively, and lengths of a line segment P3D3 and a line segment Q3E3 are 0.16 to 0.45, respectively.

The inventions described in [1] and [2] are a maxillary standard dental plate of a predetermined shape. This maxillary standard dental plate has a predetermined shape based on the denture space theory.
[3] The maxillary standard dental plate according to [1], wherein
   when a length of the line segment PQ is set to be 1, a length of a line segment NM connecting a point N corresponding to the plate posterior border on the line segment OM and the middle point M is 0.05 to 0.10.
   In the invention described in [3], particularly, a shape of the plate posterior border part is limited to a predetermined range.
[4] The maxillary standard dental plate according to [1], wherein the maxillary standard dental plate has a notched part at a part covering the palate.
   The invention described in [4] is a non-palate maxillary standard dental plate having no palate-covering part.
[5] The maxillary standard dental plate according to [1], wherein a guide showing an arrangement position of an artificial tooth is formed.
   The invention described in [5] is a maxillary standard dental plate in which a guide for arranging at least one artificial tooth is formed.
[6] The maxillary standard dental plate according to [1], wherein at least one artificial tooth has been arranged in advance.
   The invention described in [6] is a maxillary standard dental plate in which at least one artificial tooth is arranged on a maxillary standard dental plate having a predetermined shape. When this standard dental plate is used, since a tooth row to be a standard has been arranged in advance, the time for arranging a tooth row is shortened.
[7] A maxillary standard denture characterized in that artificial teeth of a whole teeth row have been arranged on the maxillary standard dental plate as defined in [1] in advance.
   The invention described in [7] is a maxillary standard denture in which artificial teeth of a whole teeth row are arranged on a maxillary standard dental plate of a predetermined shape. When this standard denture is used, since the whole teeth row has been arranged in advance, arrangement of a tooth row can be omitted.
[8] A maxillary denture fabrication kit consisting of :
   the maxillary standard dental plate as defined in [1]; and
   a lining material.
[9] A maxillary denture fabrication kit consisting of :
   the maxillary standard dental plate as defined in [1];
   connected artificial teeth in which two or more artificial teeth are connected and integrated; and
   a lining material.
[10] A maxillary denture fabrication kit consisting of :
   the maxillary standard denture as defined in [7] ; and
   a lining material.
[11] The maxillary denture fabrication kit according to any one of [8] to [10], wherein said lining material is a lining material having the durometer A hardness of 55 or less.
[12] The maxillary denture fabrication kit according to any one of [8] to [10], wherein said lining material is a hard lining material.
   The inventions described in [8] to [12] are a denture fabrication kit for selecting a maxillary standard dental plate or a maxillary standard denture in conformity with the oral cavity size of a patient, from a plurality of maxillary standard dental plates or maxillary standard dentures having the different sizes. By the methods of the following [13] and [14] using this denture fabrication kit, a maxillary denture having high matching rate can be fabricated in a short time.
[13] A maxillary denture fabrication method characterized by having:
   a step of selecting a maxillary standard dental plate in conformity with the size of the mouse of a patient, from the maxillary denture fabrication kit as defined in [8] or [9];
   a step of arranging an artificial tooth on said maxillary standard dental plate; and
   a step of building up a lining material on said maxillary standard dental plate.
[14] A maxillary denture fabrication method characterized by having:
   a step of selecting a maxillary standard denture in conformity with the size of the mouse of a patient, from the maxillary denture fabrication kit as defined in [10]; and
   a step of building up a lining material on said maxillary standard denture.
[15] A mandibular standard dental plate characterized in that
   when a length of a line segment pq connecting a point p corresponding to the left retromolar pad and a point q corresponding to the right retromolar pad of the plate posterior border is set to be 1,
   a length of a line segment om connecting a point o corresponding to the inferior labial frenulum which comes under the median of the labial denture border and a middle point m of the line segment pq is 0.74 to 0.94.
[16] The mandibular standard dental plate according to [15], wherein
   when respective points dividing a line segment op connecting the point o and the point p into four equal portions are defined as a point p1, a point p2, and a point p3 from the point o side, points at which vertical lines from the point p1, the point p2, and the point p3 on the line segment op are intersected with the left buccal denture border are defined as a point d1, a point d2, and a point d3,
   respective points dividing a line segment oq connecting the point o and the point q into four equal portions are defined as a point q1, a point q2, and a point q3 from the point o side, points at which vertical lines from the point q1, the point q2, and the point q3 on the line segment oq are intersected with the right buccal denture border are defined as a point e1, a point e2 and a point e3, and
a length of the line segment pq is set to be 1, lengths of a line segment p1d1 and a line segment q1e1 are 0.11 to 0.32, respectively,
   lengths of a line segment p2d2 and a line segment q2e2 are 0.13 to 0.34, respectively, and
   lengths of a line segment p3d3 and a line segment q3e3 are 0.14 to 0.33, respectively.
   The inventions described in [15] and [16] are a mandibular standard dental plate of a predetermined shape. This mandibular standard dental plate has a predetermined shape based on the denture space theory.
[17] The mandibular standard dental plate according to [15], wherein a guide showing an arrangement position of an artificial tooth is formed.
   The invention described in [17] is a mandibular standard dental plate in which a guide for arranging at least one artificial tooth is formed.
[18] The mandibular standard dental plate according to [15], wherein at least one artificial tooth has been arranged in advance.
   The invention described in [18] is a mandibular standard dental plate in which at least one artificial tooth is arranged on a mandibular standard dental plate of a predetermined shape. When this standard dental plate is used, since a tooth row to be a standard has been arranged in advance, the time for arranging a tooth row is shortened.
[19] A mandibular standard denture characterized in that artificial teeth of a whole teeth row have been arranged on the mandibular standard dental plate as defined in [15] in advance.
   The invention described in [19] is a mandibular standard denture in which artificial teeth of a whole teeth row are arranged on a mandibular standard dental plate of a predetermined shape. When this standard denture is used, since the whole teeth row has been arranged in advance, arrangement of a tooth row can be omitted.
[20] A mandibular denture fabrication kit consisting of:
   the mandibular standard dental plate as defined in [15]; and
   a lining material.
[21] A mandibular denture fabrication kit consisting of:
   the mandibular standard dental plate as defined in [15];
   connected artificial teeth in which two or more artificial teeth are connected and integrated; and
   a lining material.
[22] A mandibular denture fabrication kit consisting of:
   the mandibular standard denture as defined in [19] ; and
   a lining material.
[23] The mandibular denture fabrication kit according to any one of [20] to [22], wherein said lining material is a lining material having the durometer A hardness of 55 or less.
[24] The mandibular denture fabrication kit according to any one of [20] to [22], wherein said lining material is a hard lining material.
   The inventions described in [20] to [24] relate to a denture fabrication kit for selecting a mandibular standard dental plate or a mandibular standard denture which is compatible with the oral cavity size of a patient, from a plurality of mandibular standard dental plates or mandibular standard dentures having the different sizes. By the methods of the following [25] and [26] using this denture fabrication kit, a mandibular denture having the high matching rate can be fabricated in a short time.
[25] A mandibular denture fabrication method characterized by having:
   a step of selecting a mandibular standard dental plate in conformity with the size of the mouth of a patient, from the mandibular denture fabrication kit as defined in [20] or [21];
   a step of arranging an artificial tooth on said mandibular standard dental plate; and
   a step of building up a lining material on said mandibular standard dental plate.
[26] A mandibular denture fabrication method characterized by having:
   a step of selecting a mandibular standard denture in conformity with the size of the mouth of a patient, from the mandibular denture fabrication kit as defined in [22]; and
   a step of building up a lining material on said mandibular standard denture.

### Advantageous Effects of Invention

Since the standard dental plate of the present invention has a predetermined shape, it can be easily adapted to an oral cavity shape of a patient only by building up a lining material. That is, by using a ready-made standard dental plate, the time for fabricating the denture can be shortened. Additionally, in the standard denture of the present invention, since artificial teeth of a whole teeth row have been arranged on a standard dental plate of a predetermined shape in advance, the denture can be easily fabricated only by building up a lining material. That is, by using the ready-made standard dental plate and, at the same time, arranging the whole teeth row in advance in the previous step of fabricating the denture, the time for fabricating the denture can be considerably shortened. Thereby, the burden of a dentist, a dental technician and a patient can be considerably reduced.

The standard dental plate of the present invention is not fabricated by taking an impression of an oral cavity shape of individual patients as before, but is fabricated based on the denture space theory by statistically determining an oral cavity shape of a person having the jaw with teeth and an edentulous jaw person. For that reason, when a few kinds of standard dental plates are prepared, the denture having the high matching rate to almost all patients can be fabricated. Additionally, the standard dental plate can also be applied to a patient of intractable cases in which resorption of an alveolar crest is intense.

### Brief Description of Drawings

Figs. 1(a) and 1(b) are illustrations showing the state where a patient is fitted with the standard dental plate of the present invention.
Fig. 2 is a plan view showing one example of a maxillary standard dental plate 100 of the present invention.
Fig. 3 is a plan view showing one example of a shape of a non-palate maxillary standard dental plate 100a of the present invention.
Fig. 4 is illustration showing a position of an artificial tooth which is arranged on the maxillary standard dental plate of the present invention or a position of a guide.
Fig. 5 is a plan view showing one example of a maxillary standard denture in which all artificial teeth are arranged on the maxillary standard dental plate of the present invention.
Fig. 6 is illustration showing an arrangement height of an artificial tooth which is arranged on the maxillary standard dental plate of the present invention.
Figs. 7(a) and 7(b) are illustrations showing a height of an artificial tooth in the state where a patient is fitted with the denture.
Fig. 8 is a plan view showing one example of a shape of a mandibular standard dental plate 200 of the present invention.
Fig. 9 is illustration showing a position of an artificial tooth which is arranged on the mandibular standard dental plate of the present invention or a position of a guide.
Fig. 10 is a plan view showing one example of a mandibular standard denture in which all artificial teeth are arranged on the mandibular standard dental plate of the present invention.
Fig. 11 is illustration showing an arrangement height of an artificial tooth which is arranged on the mandibular standard dental plate of the present invention.

### Description of Embodiments

The present invention will be described in detail below referring to the drawings.

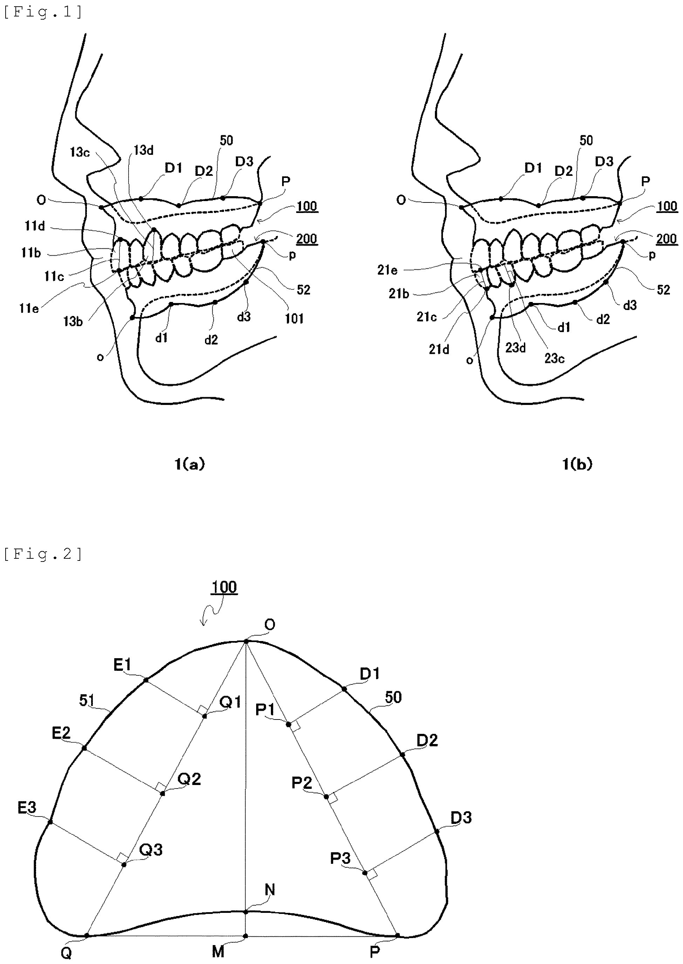

Figs. 1(a) and 1(b) are illustrations showing the state where a patient is fitted with the standard dental plate of the present invention. In Figs. 1(a) and 1(b), 100 is a maxillary standard dental plate, and 200 is a mandibular standard dental plate (hereinafter, these are also simply referred to as "standard dental plate"). 101 is an artificial tooth, and when all tooth rows are arranged on the standard dental plate, a standard denture consisting of these artificial teeth and standard dental plate is completed.

In Figs. 1(a) and 1(b), a point O is a point corresponding to the superior labial frenulum which comes under the median of the labial denture border, and a point P is a point corresponding to the left pterygomaxillary notch of the plate posterior border. The left buccal denture border 50 of the maxillary standard dental plate 100 has points D1 to D3 (described later). A point o is a point corresponding to the inferior labial frenulum which comes under the median of the labial denture border. A point p is a point corresponding to the left retromolar pad of the plate posterior border. The left buccal denture border 52 of the mandibular standard dental plate 200 has points d1 to d3 (described later). The standard dental plate of the present invention is characterized in that the relative positional relationship of these points is in a predetermined range.

### 1. With Regard to Maxillary Standard Dental Plate (1) Form of Maxillary Standard Dental Plate

Fig. 2 is a plan view showing one example of the maxillary standard dental plate 100 of the present invention. In Fig. 2, a point O is a point corresponding to the superior labial frenulum which comes under the median of the labial denture border. A point P is a point corresponding to the left pterygomaxillary notch of the plate posterior border, and a point Q is a point corresponding to the right pterygomaxillary notch of the plate posterior border. A point M is a middle point of a line segment PQ connecting the point P and the point Q. In the standard dental plate of the present invention, when a length of the line segment PQ is set to be 1, a length of a line segment OM connecting the point O and the point M is in a range of 0.76 to 0.98, preferably in a range of 0.81 to 0.93, and more preferably in a range of 0.86 to 0.88. The maxillary standard dental plate having this shape can be adapted to many edentulous jaw persons by building up a lining material. In addition, right and left of the maxillary standard dental plate are left and right when a patient is fitted therewith, and are reverted from left and right on a paper surface.

In Fig. 2, points P1 to P3 are points dividing a line segment OP into four equal portions. That is, lengths of a line segment OP1, a line segment P1P2, a line segment P2P3, and a line segment P3P are equal. Points D1 to D3 are points at which vertical lines to the line segment OP from each of the points P1 to P3 are intersected with a left buccal denture border 50. Points Q1 to Q3 are points dividing a line segment OQ into four equal portions. That is, lengths of a line segment OQ1, a line segment Q1Q2, a line segment Q2Q3, and a line segment Q3Q are equal. Points E1 to E3 are points at which vertical lines to the line segment OQ from each of the points Q1 to Q3 are intersected with a right buccal denture border 51.

In the maxillary standard dental plate of the present invention, when a length of the line segment PQ is set to be 1, lengths of a line segment P1D1 and a line segment Q1E1 are preferably in a range of 0.11 to 0.36, more preferably in a range of 0.16 to 0.31, and particularly preferably in a range of 0.21 to 0.26, respectively. Additionally, lengths of a line segment P2D2 and a line segment Q2E2 are preferably in a range of 0.19 to 0.45, more preferably in a range of 0.24 to 0.40, and particularly preferably in a range of 0.29 to 0.35, respectively. Furthermore, lengths of a line segment P3D3 and a line segment Q3E3 are preferably in a range of 0.16 to 0.45, more preferably in a range of 0.21 to 0.40, and particularly preferably in a range of 0.26 to 0.35. The maxillary standard dental plate having this shape can be adapted to edentulous jaw persons with much higher matching rate.

### (2) Form of Plate Posterior Border Part

In Fig. 2, a point N is a point corresponding to the plate posterior border, and is a point at which a line passing through the middle point M and orthogonal to the line segment PQ is intersected with the plate posterior border part. A length of a line segment NM is preferably 0.05 to 0.10, and more preferably 0.06 to 0.09. This shape is a shape which is obtained by cutting mainly a central part of the plate posterior border of the dental plate while both ends thereof are left. By forming the dental plate in this shape, fittability can be improved while sealability of the plate posterior border part is maintained. When the length is less than 0.05, it is necessary to greatly cut the plate posterior border part in order to improve fittability in some cases, and the time for fabricating the denture becomes long. On the other hand, when the length is more than 0.10, sealability of the plate posterior border part is reduced.

### (3) Non-Palate Maxillary Standard Dental Plate

The maxillary standard dental plate of the present invention may be a non-palate maxillary standard dental plate not having a part or all of a portion covering the palate. The non-palate maxillary standard dental plate can be adapted to many edentulous jaw persons by forming a palate-covering part at the part covering the palate using a lining material or the like, and building up the lining material or the like on a whole mucous membrane contact surface of the dental plate. That is, by not only using a ready-made dental plate to shorten the time for fabricating the denture, but also fabricating a palate-covering part for every oral cavity shape of a patient, the palate-covering part having the high matching rate between the palate-covering part and the mucous membrane, and a small thickness can be formed.

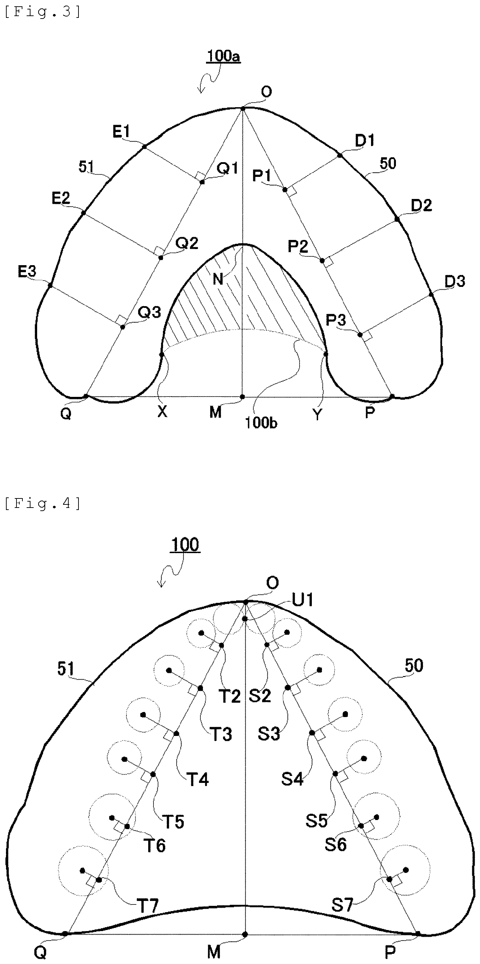

Fig. 3 is a plan view showing one example of a shape of a non-palate maxillary standard dental plate 100a of the present invention. 100a is a non-palate maxillary standard dental plate, and 100b is a notched part corresponding to a palate part of the maxillary standard dental plate. In addition, right and left of the non-palate maxillary standard dental plate are left and right when a patient is fitted therewith, and are inverted from left and right on a paper surface.

In Fig. 3, a point N is an intersection between a vertical line to a line segment PQ from a middle point M and the plate posterior border. A length of a line segment NM connecting the point N and the middle point M is preferably 0.40 to 0.85, and more preferably 0.50 to 0.75. By forming the notched part 100b with this length, the palate-covering part can be formed thin, and the matching rate to the mucous membrane can be sufficiently improved. When the length is less than 0.40, the matching rate to the mucous membrane cannot be sufficiently improved in some cases. On the other hand, when the length is more than 0.85, the matching rate to the mucous membrane can be improved, but the fabrication time becomes long.

In Fig. 3, a point X is an end on the right buccal side of the notched part 100b, and a point Y is an end on the left buccal side. The point X is such a point that when a line connecting the central fossa of the right maxillary second molar (right upper 7^{th}) and the central fossa of the left maxillary second molar (left upper 7^{th}) is translated parallel in a tooth axis direction of the right maxillary second molar and the left maxillary second molar, the line and the right buccal denture border part are intersected. Y is similarly such a point that when a line connecting the central fossa of the right maxillary second molar (right upper 7^{th}) and the central fossa of the left maxillary second molar (left upper 7^{th}) is translated parallel in a tooth axis direction of the right maxillary second molar and the left maxillary second molar, the line and the left buccal denture border part are intersected. In the present invention, a notch width of the notched part 100b means a length of a line segment XY.

When a length of the line segment PQ is set to be 1, the line segment XY is preferably 0.4 to 0.7, and more preferably 0.5 to 0.6. By forming the notched part 100b with this notch width, the matching rate to the mucous membrane can be sufficiently improved. In addition, it is preferable that the point X and the point Y are formed symmetrically with a line segment OM.

Additionally, an area of the notched part 100b is preferably 1 to 5 cm², and more preferably 2 to 4 cm². In addition, herein, an area of the notched part 100b means an area corresponding to the palate-covering part which is formed using a lining material at a post step.

For forming the notched part 100b, it may be formed by notching the standard dental plate having the palate, or may be formed, for example, using a mold in which the notched part 100b is formed. That is, it is not essential to manufacture the notched part by actual notching.

### (4) Arrangement of Artificial Tooth

It is preferable that at least one artificial tooth has been arranged on the maxillary standard dental plate of the present invention in advance. When the artificial tooth has been arranged in advance, by arranging other tooth rows so as to form any dental arch of square, rounded square, rounded, rounded V shape types based on the Thompson's classification standard employing the artificial tooth as a standard, the time for arranging the artificial tooth can be shortened. It is preferable that the artificial tooth to be arranged in advance is one or more teeth including the first molar (6^{th}), the central incisor (1^{st}), and the canine (3^{rd}).

The maxillary standard dental plate of the present invention may have a guide showing an arrangement position of the artificial tooth. Examples of the guide include a method of forming a through-hole for arranging the artificial tooth on the standard dental plate, a method of providing a concave part corresponding to a shape of the artificial tooth, a method of providing a notched concave part such as flaw and groove, a method of printing a mark by printing or transference, and a method of attaching a member to be a mark.

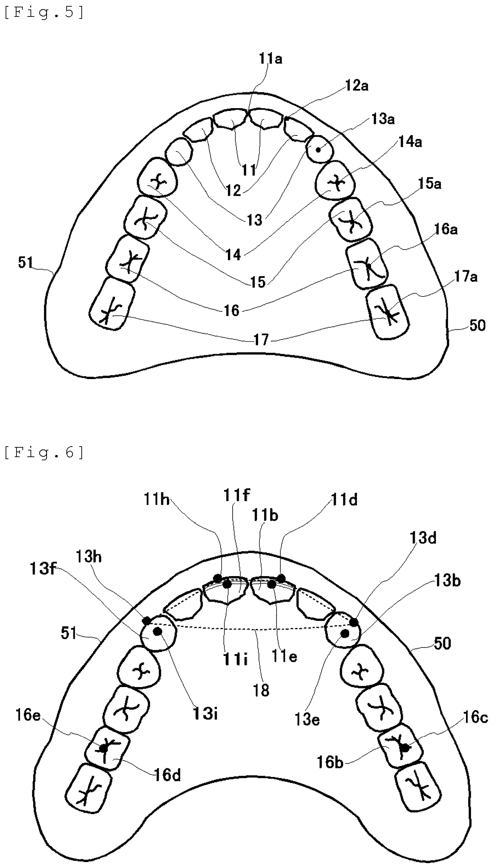

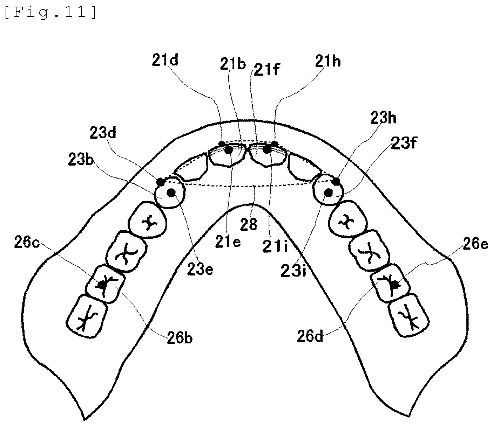

Fig. 4 is illustration showing a position of an artificial tooth which is arranged on the maxillary standard dental plate of the present invention or a position of a guide. Fig. 5 is a plan view showing one example of a maxillary standard denture in which all artificial teeth are arranged on the maxillary standard dental plate of the present invention. In Fig. 5, 11 is the maxillary central incisor (1^{st}), and 11a is the interdental part between the left maxillary central incisor and the right maxillary central incisor. 12 is the maxillary lateral incisor (2^{nd}), 13 is the maxillary canine (3^{rd}), 14 is the maxillary first premolar (4^{th}), 15 is the maxillary second premolar (5^{th}), 16 is the maxillary first molar (6^{th}), and 17 is the maxillary second molar (7^{th}). 16a is the central fossa of the maxillary first molar.

### (4-1) Arrangement Position of Artificial Tooth on Maxillary Standard Dental Plate or Position of Guide

### (a) Arrangement Position of Left Maxillary First Molar (left upper 6^{th}) and Position of Guide

In Fig. 4, a point S6 is on a line segment OP, and when a length of a line segment PQ is set to be 1, is positioned at a distance of 0.74 from a point O. A point T6 is on a line segment OQ, and when a length of the line segment PQ is set to be 1, is positioned at a distance of 0.74 from the point O. It is preferable that the left maxillary first molar is arranged so as to have the central fossa thereof in a circle of a radius 0.14, which is on a vertical line to the line segment OP from the point S6 to the left buccal denture border 50 side, and has a central point at a distance of 0.11 from the point S6 to the left buccal denture border 50 side, and it is particularly preferable that it is arranged in a circle of a radius 0.08.

When a guide for arranging an artificial tooth is formed without arranging an artificial tooth, a position thereof is a position as described above. In addition, in arrangement of other artificial teeth which will be described below, an arrangement position of the artificial tooth and a formation position of the guide are the same. For that reason, description of the formation position of the guide is omitted.

### (b) Arrangement Position of Right Maxillary First Molar (right upper 6^{th})

Similarly, it is preferable that the right maxillary first molar is arranged so as to have the central fossa thereof in a circle of a radius 0.14, which is on a vertical line to the line segment OQ from the point T6 to the right buccal denture border 51 side, and has a central point at a distance of 0.11 from the point T6 to the right buccal denture border 51 side, and it is particularly preferable that it is arranged in a circle of a radius 0.08.

### (c) Arrangement Position of Maxillary Central incisor (upper 1^{st})

In Fig. 4, the maxillary central incisor is arranged so as to have the interdental part between the left maxillary central incisor and the right maxillary central incisor, on a straight line OM, and at a distance of 0.76 to 1.14 from a point M to a labial side.

It is preferable that the left maxillary central incisor is arranged so that the central mamelon of the left central incisor is positioned in a circle of a diameter 0.17, which is on the straight line OM, has the circumference coming in contact with a point U1 at a distance of 0.76 to 1.14 from the point M to a labial side when a length of the line segment PQ is set to be 1, and at the same time, has a central point on a straight line orthogonal to the straight line OM at a point U1, and on the left buccal denture border 50 side.

Similarly, it is preferable that the right maxillary central incisor is arranged so that the central mamelon of the right central incisor is positioned in a circle of a diameter 0.17, which is on the straight line OM, has the circumference coming in contact with a point U1 at a distance of 0.76 to 1.14 from the point M to a labial side, and at the same time, has a central point on a straight line orthogonal to the straight line OM at the point U1, and on the right buccal denture border 51 side.

The point U1 is preferably at a distance of 0.86 to 1.04 from the point M, and particularly preferably at a distance of 0.91 to 0.99. Additionally, it is preferable that the left maxillary central incisor and the right maxillary central incisor are arranged bilaterally symmetrical with the straight line OM.

### (d) Arrangement Position of Left Maxillary Canine (left upper 3^{rd})

In Fig. 4, a point S3 is on the line segment OP, and when a length of the line segment PQ is set to be 1, is at a distance of 0.30 from the point O. A point T3 is on the line segment OQ, and when a length of the line segment PQ is set to be 1, is at a distance of 0.30 from the point O. It is preferable that the left maxillary canine is arranged so that the pointed cuspid of the left maxillary canine is positioned in a circle of a radius 0.12, which is on a vertical line to the line segment OP from the point S3 to the left buccal denture border 50 side, and has a central point at a distance of 0.25 from the point S3 to the left buccal denture border 50 side, and it is particularly preferable that it is arranged in a circle of a radius 0.07.

### (e) Arrangement Position of Right Maxillary Canine (right upper 3^{rd})

Similarly, it is preferable that the right maxillary canine is arranged so that the pointed cuspid of the right maxillary canine is positioned in a circle of a radius 0.12, which is on a vertical line to the line segment OQ from the point T3 to the right buccal denture border 51 side, and has a central point at a distance of 0.25 from the point T3 to the right buccal denture border 51 side, and it is particularly preferable that it is arranged in a circle of a radius 0.07.

### (f) Arrangement Position of Left Maxillary First Premolar (left upper 4^{th})

In Fig. 4, a point S4 is on the line segment OP, and when a length of the line segment PQ is set to be 1, is at a distance of 0.44 from the point O. A point T4 is on the line segment OQ, and when a length of the line segment PQ is set to be 1, is at a distance of 0.44 from the point O. It is preferable that the left maxillary first premolar is arranged so that an intersection between the central groove thereof and the central ridge is positioned in a circle of a radius 0.13, which is on a vertical line to the line segment OP from the point S4 to the left buccal denture border 50 side, and has a central point at a distance of 0.24 from the point S4 to the left buccal denture border 50 side, and it is particularly preferable that it is arranged in a circle of a radius 0.08.

### (g) Arrangement Position of Right Maxillary First Premolar (right upper 4^{th})

Similarly, it is preferable that the right maxillary first premolar is arranged so that an intersection between the central groove thereof and the central ridge is positioned in a circle of a radius 0.13, which is on a vertical line to the line segment OQ from the point T4 to the right buccal denture border 51 side, and has a central point at a distance of 0.24 from the point T4 to the right buccal denture border 51 side, and it is particularly preferable that it is arranged in a circle of a radius 0.08.

### (h) Arrangement Position of Left Maxillary Second Premolar (left upper 5^{th})

In Fig. 4, a point S5 is on the line segment OP, and when a length of the line segment PQ is set to be 1, is at a distance of 0.58 from the point O. A point T5 is on the line segment OQ, and when a length of the line segment PQ is set to be 1, is at a distance of 0.58 from the point O. It is preferable that the left maxillary second premolar is arranged so that an intersection between the central groove thereof and the central ridge is positioned in a circle of a radius 0.13, which is on a vertical line to the line segment OP from the point S5 to the left buccal denture border 50 side, and has a central point at a distance of 0.19 from the point S5 to the left buccal denture border 50 side, and it is particularly preferable that it is arranged in a circle of a radius 0.08.

### (i) Arrangement Position of Right Maxillary Second Premolar (right upper 5^{th})

Similarly, it is preferable that the right maxillary second premolar is arranged so that an intersection between the central groove thereof and the central ridge is positioned in a circle of a radius 0.13, which is on a vertical line to the line segment OQ from a point T5 to the right buccal denture border 51 side, and has a central point at a distance of 0.19 from the point T5 to the right buccal denture border 51 side, and it is particularly preferable that it is arranged in a circle of a radius 0.08.

### (j) Arrangement Position of Left Maxillary Second Molar (left upper 7^{th})

In Fig. 4, a point S7 is on the line segment OP, and when a length of the line segment PQ is set to be 1, is at a distance of 0.90 from the point O. A point T7 is on the line segment OQ, and when a length of the line segment PQ is set to be 1, is at a distance of 0.90 from the point O. It is preferable that the left maxillary second molar is arranged so that the central fossa thereof is positioned in a circle of a radius 0.12, which is on a vertical line to the line segment OP from the point S7 to the left buccal denture border 50 side, and has a central point at a distance of 0.08 from the point S7 to the left buccal denture border 50 side, and it is particularly preferable that it is arranged in a circle of a radius 0.08.

### (k) Arrangement Position of Right Maxillary Second Molar (right upper 7^{th})

Similarly, it is preferable that the right maxillary second molar is arranged so that the central fossa thereof is positioned in a circle of a radius 0.12, which is on a vertical line to the line segment OQ from the point T7 to the right buccal denture border 51 side, and has a central point at a distance of 0.08 from the point T7 to the right buccal denture border 51 side, and it is particularly preferable that it is arranged in a circle of a radius 0.08.

### (l) Arrangement Position of Left Maxillary Lateral incisor (left upper 2^{nd})

In Fig. 4, a point S2 is on the line segment OP, and when a length of the line segment PQ is set to be 1, is at a distance of 0.12 from the point O. A point T2 is on the line segment OQ, and when a length of the line segment PQ is set to be 1, is at a distance of 0.12 from the point O. It is preferable that the left maxillary lateral incisor is arranged so that the mesioincisal angle thereof is positioned in a circle of a radius 0.11, which is on a vertical line to the line segment OP from the point S2 to the left buccal denture border 50 side, and has a central point at a distance of 0.14 from the point S2 to the left buccal denture border 50 side, and it is particularly preferable that it is arranged in a circle of a radius 0.08.

### (m) Arrangement Position of Right Maxillary Lateral incisor (right upper 2^{nd})

Similarly, it is preferable that the right maxillary lateral incisor is arranged so that the mesioincisal angle thereof is positioned in a circle of a radius 0.11, which is on a vertical line to the line segment OQ from the point T2 to the right buccal denture border 51 side, and has a central point at a distance of 0.14 from the point T2 to the right buccal denture border 51 side, and it is particularly preferable that it is arranged in a circle of a radius 0.08.

### (n) Arrangement Height of Artificial Tooth

In Fig. 1(a), 11b is the left maxillary central incisor (left upper 1^{st}), and 11c is the centrolabial ridge of the left maxillary central incisor (left upper 1^{st}). 11d is an intersection between the centrolabial ridge 11c of the left maxillary central incisor (left upper 1^{st}) and the gingival margin. 13c is the centrolabial ridge of the left maxillary canine (left upper 3^{rd}), and 13d is an intersection between the centrolabial ridge 13c of the left maxillary canine (left upper 3^{rd}) and the gingival margin. Fig. 6 is illustration showing an arrangement height of an artificial tooth which is arranged on the maxillary standard dental plate of the present invention. In Fig. 6, 11b is the left maxillary central incisor (left upper 1^{st}) , and 11d is an intersection between the centrolabial ridge of the left maxillary central incisor (left upper 1^{st}) and the gingival margin. 11f is the right maxillary central incisor (right upper 1^{st}), and 11h is an intersection between the centrolabial ridge of the right maxillary central incisor (right upper 1^{st}) and the gingival margin. 13b is the left maxillary canine (left upper 3^{rd}), and 13d is an intersection between the centrolabial ridge of the left maxillary canine (left upper 3^{rd}) and the gingival margin. 13f is the right maxillary canine (right upper 3^{rd}), and 13h is an intersection between the centrolabial ridge of the right maxillary canine (right upper 3^{rd}) and the gingival margin. In the present invention, a planar surface connecting the intersections 11d, 11h, 13d, and 13h between the centrolabial ridge in each tooth and the gingival margin is defined as a standard planar surface 18.

In Fig. 6, 16b is the left maxillary first molar (left upper 6^{th}), and 16c is the distobuccal cusp tip of the left maxillary first molar (left upper 6^{th}). 16d is the right maxillary first molar (right upper 6^{th}), and 16e is the distobuccal cusp tip of the right maxillary first molar (right upper 6^{th}) .

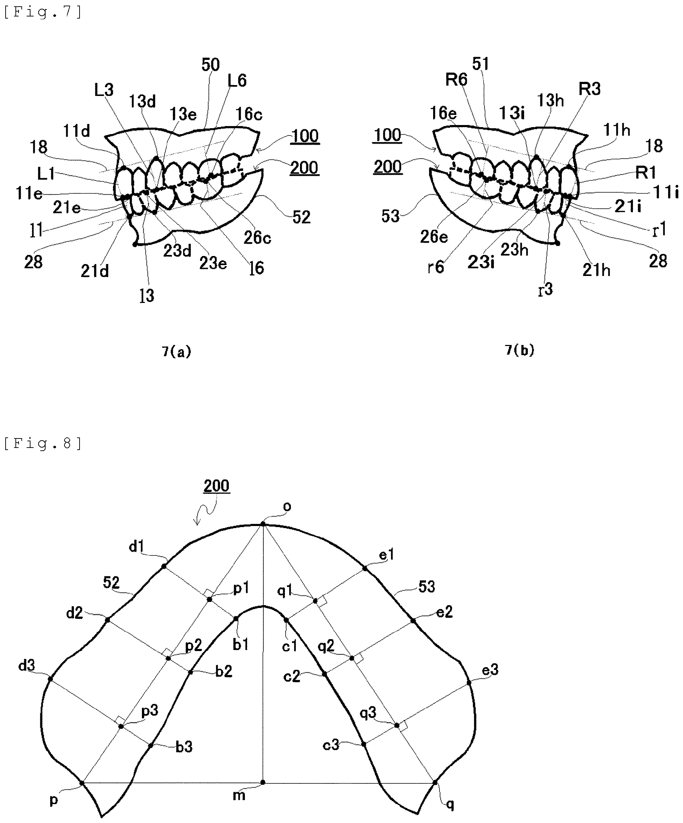

When a length of the line segment PQ in Fig. 2 is set to be 1, a vertical distance L6 to a standard planar surface 18 from the standard planar surface 18 to the distobuccal cusp tip 16c, and a vertical distance R6 to a standard planar surface 18 from the standard planar surface 18 to the distobuccal cusp tip 16e are preferably 0.16 to 0.24, and more preferably 0.18 to 0.22, respectively (see Figs. 7(a) and 7(b)). When the distance is outside a range of 0.16 to 0.24, an occlusal force is reduced in some cases.

In Fig. 6, 11b is the left maxillary central incisor (left upper 1^{st}), and 11e is the center of incisal edge of the left maxillary central incisor (left upper 1^{st}). 11f is the right maxillary central incisor (right upper 1^{st}), and 11i is the center of incisal edge of the right maxillary central incisor (right upper 1^{st}).

When a length of the line segment PQ in Fig. 2 is set to be 1, a vertical distance L1 to a standard planar surface 18 from the standard planar surface 18 to the center of incisal edge 11e of the left maxillary central incisor (left upper 1^{st}), and a vertical distance R1 to a standard planar surface 18 from the standard planar surface 18 to the center of incisal edge 11i of the right maxillary central incisor (right upper 1^{st})are preferably 0.16 to 0.24, and more preferably 0.18 to 0.22, respectively (see Figs. 7(a) and 7(b)). When the distance is outside a range of 0.16 to 0.24, an occlusal force is reduced in some cases.

In Fig. 6, 13b is the left maxillary canine (left upper 3^{rd}), and 13e is the pointed cuspid of the left maxillary canine (left upper 3^{rd}). 13f is the left maxillary canine (left upper 3^{rd}), and 13i is the pointed cuspid of the left maxillary canine (left upper 3^{rd}).

When a length of the line segment PQ in Fig. 2 is set to be 1, a vertical distance L3 to a standard planar surface 18 from the standard planar surface 18 to the pointed cuspid 13e of the left maxillary canine (left upper 3^{rd}), and a vertical distance R3 to a standard planar surface 18 from the standard planar surface 18 to the pointed cuspid 13i of the right maxillary canine (right upper 3^{rd}) are preferably 0.16 to 0.24, and more preferably 0.18 to 0.22, respectively (see Figs. 7(a) and 7(b)). When the distance is outside a range of 0.16 to 0.24, an occlusal force is reduced in some cases.

### 2. With Regard to Maxillary Standard Denture

The maxillary standard denture of the present invention is such that artificial teeth of a whole teeth row have been arranged on the above-mentioned maxillary standard dental plate in advance. Positions at which these artificial teeth are arranged are not particularly limited, but it is preferable that the teeth are arranged at the positions described above.

### 3. With Regard to Mandibular Standard Dental Plate

### (1) Form of Mandibular Standard Dental Plate

Fig. 8 is a plan view showing one example of a shape of the mandibular standard dental plate 200 of the present invention. In Fig. 8, a point o is a point corresponding to the inferior labial frenulum which comes under the median of the labial denture border. A point p is a point corresponding to the left retromolar pad of the plate posterior border, and a point q is a point corresponding to the right retromolar pad of the plate posterior border. A point m is a middle point of a line segment pq connecting the point p and the point q. In this mandibular standard dental plate, when a length of the line segment pq is set to be 1, a length of a line segment om connecting the point o and the point m is in a range of 0.76 to 0.94, preferably in a range of 0.80 to 0.90, and more preferably in a range of 0.84 to 0.86. The mandibular standard dental plate having this shape can be adapted to many edentulous jaw persons by building up a lining material.

In Fig. 8, points p1 to p3 are points dividing a line segment op into four equal portions. That is, lengths of a line segment op1, a line segment p1p2, a line segment p2p3, and a line segment p3p are equal. Points d1 to d3 are points at which vertical lines to the line segment op from each of the points p1 to p3 are intersected with the left buccal denture border 52. Points q1 to q3 are points dividing a line segment oq into four equal portions. That is, lengths of a line segment oq1, a line segment q1q2, a line segment q2q3, and a line segment q3q are equal. Points e1 to e3 are points at which vertical lines to the line segment oq from each of the points q1 to q3 are intersected with the right buccal denture border 53.

In the mandibular standard dental plate of the present invention, when a length of the line segment pq is set to be 1, lengths of a line segment p1d1 and a line segment q1e1 are preferably in a range of 0.11 to 0.32, more preferably in a range of 0.15 to 0.28, and particularly preferably in a range of 0.19 to 0.24, respectively. Additionally, lengths of a line segment p2d2 and a line segment q2e2 are preferably in a range of 0.13 to 0.34, more preferably in a range of 0.17 to 0.30, and particularly preferably in a range of 0.21 to 0.26, respectively. Furthermore, lengths of a line segment p3d3 and a line segment q3e3 are preferably in a range of 0.14 to 0.33, more preferably in a range of 0.18 to 0.29, and particularly preferably in a range of 0.22 to 0.25, respectively. The mandibular standard dental plate having this shape can be adapted to an edentulous jaw person with higher matching rate.

In Fig. 8, points b1 to b3 are points at which vertical lines to the line segment op from each of the points p1 to p3 are intersected with the lingual denture border. Points c1 to c3 are points at which vertical lines to the line segment oq from each of the points q1 to q3 are intersected with the lingual denture border.

In the mandibular standard dental plate of the present invention, when a length of the line segment pq is set to be 1, lengths of a line segment d1b1 and a line segment e1c1 are preferably in a range of 0.14 to 0.40, more preferably in a range of 0.19 to 0.35, and particularly preferably in a range of 0.24 to 0.30, respectively. Additionally, lengths of a line segment d2b2 and a line segment e2c2 are preferably in a range of 0.19 to 0.41, more preferably in a range of 0.24 to 0.36, and particularly preferably in a range of 0.29 to 0.31, respectively. Furthermore, lengths of a line segment d3b3 and a line segment e3c3 are preferably in a range of 0.21 to 0.42, more preferably in a range of 0.25 to 0.38, and particularly preferably in a range of 0.30 to 0.34, respectively. The mandibular standard dental plate having this shape can be adapted to an edentulous jaw person with much higher matching rate.

### (2) Arrangement of Artificial Tooth

It is preferable that at least one artificial tooth has been arranged on the mandibular standard dental plate of the present invention in advance. The artificial tooth to be arranged in advance is preferably one or more teeth including the first molar (6^{th}), the central incisor (1^{st}), and the canine (3^{d}).

The mandibular standard dental plate of the present invention may have a guide showing an arrangement position of the artificial tooth. Formation of the guide is the same as that of the maxillary standard dental plate as described above.

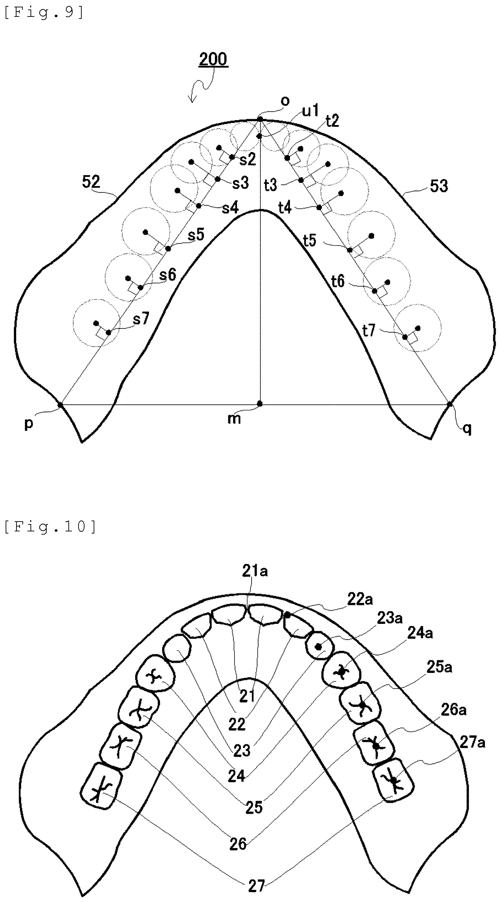

Fig. 9 is illustration showing a position of an artificial tooth which is arranged on the mandibular standard dental plate of the present invention or a position of a guide. Fig. 10 is a plan view showing one example of a mandibular standard denture in which all artificial teeth are arranged on the mandibular standard dental plate of the present invention. In Fig. 10, 21 is the mandibular central incisor (1^{st}), and 21a is an interdental part between the left mandibular central incisor and the right mandibular central incisor. 22 is the mandibular lateral incisor (2^{nd}), and 22a is the mesioincisal angle of the mandibular lateral incisor. 23 is the mandibular canine (3^{rd}), and 23a is the pointed cuspid of the mandibular canine. 24 is the mandibular first premolar (4^{th}), and 24a is an intersection between the central groove in the mandibular first premolar and the central ridge. 25 is the mandibular second premolar (5^{th}), and 25a is an intersection between the central groove in the mandibular second premolar and the central ridge. 26 is the mandibular first molar (6^{th}), and 26a is the central fossa of the mandibular first molar. 27 is the mandibular second molar (7^{th}), and 27a is the central fossa of the mandibular second molar.

### (2-1) Arrangement Position of Artificial Tooth in Mandibular Standard Dental Plate or Position of Guide

### (o) Arrangement Position of Left Mandibular First Molar (left lower 6^{th}) or Position of Guide

In Fig. 9, a point s6 is on a line segment op, and when a length of a line segment pq is set to be 1, is at a distance of 0.65 from a point o. A point t6 is on a line segment oq, and when a length of the line segment pq is set to be 1, is at a distance of 0.65 from the point o. It is preferable that the left mandibular first molar (left lower 6^{th}) is arranged so that the central fossa of the left first molar is positioned in a circle of a radius 0.12, which is on a vertical line to the line segment op from the point s6 to the left buccal denture border 52 side, and has a central point at a distance of 0.08 from the point s6 to the left buccal denture border 52 side, and it is particularly preferable that it is arranged in a circle of a radius 0.08.

### (p) Arrangement Position of Right Mandibular First Molar (right lower 6^{th})

Similarly, it is preferable that the right mandibular first molar is arranged so that the central fossa of the right mandibular first molar is positioned in a circle of a radius 0.12, which is on a vertical line to the line segment oq from a point t6 to the right buccal denture border 53 side, and has a central point at a distance of 0.08 from the point t6 to the right buccal denture border 53 side, and it is particularly preferable that it is arranged in a circle of a radius 0.08.

### (q) Arrangement Position of Mandibular Central incisor (lower 1^{st})

The mandibular central incisor is on a straight line om in Fig. 9, and when a length of the line segment pq is set to be 1, is arranged so as to have an interdental part between the left mandibular central incisor and the right mandibular central incisor at a distance of 0.74 to 0.94 from a point m to a labial side.

It is preferable that the left mandibular central incisor is arranged so that the central mamelon of the left central incisor is positioned in a circle of a diameter 0.12, which is on a straight line om, has the circumference coming in contact with a point u1 at a distance of 0.74 to 0.94 from the point m to the labial side when a length of the line segment pq is set to be 1, and at the same time, has a central point on a straight line orthogonal to the straight line om at the point u1, and on the left buccal denture border 52 side.

Similarly, it is preferable that the right mandibular central incisor is arranged so that the central mamelon of the right central incisor is positioned in a circle of a diameter 0.12, which is on the straight line om, has the circumference coming in contact with the point u1 at a distance of 0.74 to 0.94 from the point m to the labial side when a length of the line segment pq is set to be 1, and at the same time, has a central point on a straight line orthogonal to the straight line om at the point u1, and on the right buccal denture border 53 side.

The point u1 is more preferably at a distance of 0.79 to 0.89, and particularly preferably at a distance of 0.83 to 0.85 from the point m.

Additionally, it is preferable that the left mandibular central incisor and the right mandibular central incisor are arranged bilaterally symmetric with the straight line om.

### (r) Arrangement Position of Left Mandibular Canine (left lower 3^{rd})

In Fig. 9, a point s3 is on the line segment op, and when a length of the line segment pq is set to be 1, is at a distance of 0.23 from the point o. A point t3 is on the line segment oq, and when a length of the line segment pq is set to be 1, is at a distance of 0.23 from the point o. It is preferable that the left mandibular canine is arranged so that the pointed cuspid of the left mandibular canine is positioned in a circle of a radius 0.11, which is on a vertical line to the line segment op from the point s3 to the left buccal denture border 52 side, and has a central point at a distance of 0.17 from the point s3 to the left buccal denture border 52 side, and it is particularly preferable that it is arranged in a circle of a radius 0.08.

### (s) Arrangement Position of Right Mandibular Canine (right lower 3^{rd})

Similarly, it is preferable that the right mandibular canine is arranged so that the pointed cuspid of the right mandibular canine is positioned in a circle of a radius 0.11, which is on a vertical line to the line segment oq from the point t3 to the right buccal denture border 53 side, and has a central point at a distance of 0.17 from the point t3 to the right buccal denture border 53 side, and it is particularly preferable that it is arranged in a circle of a radius 0.08.

### (t) Arrangement Position of Left Mandibular First Premolar (left lower 4^{th})

In Fig. 9, a point s4 is on the line segment op, and when a length of the line segment pq is set to be 1, is at a distance of 0.38 from the point o. A point t4 is on the line segment oq, and when a length of the line segment pq is set to be 1, is at a distance of 0.38 from the point o. It is preferable that the left mandibular first premolar is arranged so that an intersection between the central groove thereof and the central ridge is positioned in a circle of a radius 0.12, which is on a vertical line to the line segment op from the point s4 to the left buccal denture border 52 side, and has a central point at a distance of 0.15 from the point s4 to the left buccal denture border 52 side, and it is particularly preferable that it is arranged in a circle of a radius 0.08.

### (u) Arrangement Position of Right Mandibular First Premolar (right lower 4^{th})

Similarly, it is preferable that the right mandibular first premolar is arranged so that an intersection between the central groove thereof and the central ridge is positioned in a circle of a radius 0.12, which is on a vertical line to the line segment oq from the point t4 to the right buccal denture border 53 side, and has a central point at a distance of 0.15 from the point t4 to the right buccal denture border 53 side, and it is particularly preferable that it is arranged in a circle of a radius 0.08.

### (v) Arrangement Position of Left Mandibular Second Premolar (left lower 5^{th})

In Fig. 9, a point s5 is on the line segment op, and when a length of the line segment pq is set to be 1, is at a distance of 0.50 from the point o. A point t5 is on the line segment oq, and when a length of the line segment pq is set to be 1, is at a distance of 0.50 from the point o. It is preferable that the left mandibular second premolar is arranged so that an intersection between the central groove thereof and the central ridge is positioned in a circle of a radius 0.12, which is on a vertical line to the line segment op from the point s5 to the left buccal denture border 52 side, and has a central point at a distance of 0.14 from the point s5 to the left buccal denture border 52 side, and it is particularly preferable that it is arranged in a circle of a radius 0.08.

### (w) Arrangement Position of Right Mandibular Second Premolar (right lower 5^{th})

Similarly, it is preferable that the right mandibular second premolar is arranged so that an intersection between the central groove thereof and the central ridge is positioned in a circle of a radius 0.12, which is on a vertical line to the line segment oq from the point t5 to the right buccal denture border 53 side, and has a central point at a distance of 0.14 from the point t5 to the right buccal denture border 53 side, and it is particularly preferable that it is arranged in a circle of a radius 0.08.

### (x) Arrangement Position of Left Mandibular Second Molar (left lower 7^{th})

In Fig. 9, a point s7 is on the line segment op, and when a length of the line segment pq is set to be 1, is at a distance of 0.82 from the point o. A point t7 is on the line segment oq, and when a length of the line segment pq is set to be 1, is at a distance of 0.82 from the point o. It is preferable that the left mandibular second molar is arranged so that the central fossa thereof is positioned in a circle of a radius 0.12, which is on a vertical line to the line segment op from the point s7 to the left buccal denture border 52 side, and has a central point at a distance of 0.08 from the point s7 to the left buccal denture border 52 side, and it is particularly preferable that it is arranged in a circle of a radius 0.08.

### (y) Arrangement Position of Right Mandibular Second Molar (right lower 7^{th})

Similarly, it is preferable that the right mandibular second molar is arranged so that the central fossa thereof is positioned in a circle of a radius 0.12, which is on a vertical line to the line segment oq from the point t7 to the right buccal denture border 53 side, and has a central point at a distance of 0.08 from the point t7 to the right buccal denture border 53 side, and it is particularly preferable that it is arranged in a circle of a radius 0.08.

### (z) Arrangement Position of Left Mandibular Lateral incisor (left lower 2^{nd})

In Fig. 9, a point s2 is on the line segment op, and when a length of the line segment pq is set to be 1, is at a distance of 0.08 from the point o. A point t2 is on the line segment oq, and when a length of the line segment pq is set to be 1, is at a distance of 0.08 from the point o. It is preferable that the left mandibular lateral incisor is arranged so that the mesioincisal angle thereof is positioned in a circle of a radius 0.10, which is on a vertical line to the line segment op from the point s2 to the left buccal denture border 52 side, and has a central point at a distance of 0.10 from the point s2 to the left buccal denture border 52 side, and it is particularly preferable that it is arranged in a circle of a radius 0.08.

### (aa) Arrangement Position of Right Mandibular Lateral incisor (right lower 2^{nd})

Similarly, it is preferable that the right mandibular lateral incisor is arranged so that the mesioincisal angle thereof is positioned in a circle of a radius 0.10, which is on a vertical line to the line segment oq from the point t2 to the right buccal denture border 53 side, and has a central point at a distance of 0.10 from the point t2 to the right buccal denture border 53 side, and it is particularly preferable that it is arranged in a circle of a radius 0.08.

### (ab) Arrangement Height of Artificial Tooth

In Fig. 1(b), 21b is the left mandibular central incisor(left lower 1^{st}), and 21c is the centrolabial ridge of the left mandibular central incisor (left lower 1^{st}). 21d is an intersection between the centrolabial ridge 21c of the left mandibular central incisor (left lower 1^{st})and the gingival margin. 23c is the centrolabial ridge of the left mandibular canine (left lower 3^{rd}), and 23d is an intersection between the centrolabial ridge 23c of the left mandibular canine (left lower 3^{rd}) and the gingival margin. Fig. 11 is illustration showing an arrangement height of an artificial tooth which is arranged on the mandibular standard dental plate of the present invention. In Fig. 11, 21b is the left mandibular central incisor (left lower 1^{st}), and 21d is an intersection between the centrolabial ridge of the left mandibular central incisor (left lower 1^{st}) and the gingival margin. 21f is the right mandibular central incisor (right lower 1^{st}), and 21h is an intersection between the centrolabial ridge of the right mandibular central incisor (right lower 1^{st}) and the gingival margin. 23b is the left mandibular canine (left lower 3^{rd}), and 23d is an intersection between the centrolabial ridge of the left mandibular canine (left lower 3^{rd}) and the gingival margin. 23f is the right mandibular canine (right lower 3^{rd}), and 23h is an intersection between the centrolabial ridge of the right mandibular canine (right lower 3^{rd}) and the gingival margin. In the present invention, a planar surface connecting the intersections 21d, 21h, 23d, and 23h between the centrolabial ridge at each tooth and the gingival margin is defined as a standard planar surface 28.

In Fig. 11, 26b is the left mandibular first molar (left lower 6^{th}), and 26c is the distobuccal cusp tip of the left mandibular first molar (left lower 6^{th}). 26d is the right mandibular first molar (right lower 6^{th}), and 26e is the distobuccal cusp tip of the right mandibular first molar (right lower 6^{th}).

When a length of a line segment pq in Fig. 8 is set to be 1, a vertical distance 16 (L-6) to a standard planar surface 28 from the standard planar surface 28 to the distobuccal cusp tip 26c, and a vertical distance r6 to a standard planar surface 28 from the standard planar surface 28 to the distobuccal cusp tip 26e are preferably 0.12 to 0.20, and more preferably 0.14 to 0.18, respectively (see Figs. 7(a) and 7(b)). When the distance is outside a range of 0.12 to 0.20, an occlusal force is reduced in some cases.

In Fig. 11, 21b is the left mandibular central incisor (left lower 1^{st}) , and 21e is the center of incisal edge of the left mandibular central incisor (left lower 1^{st}). 21f is the right mandibular central incisor (right lower 1^{st}), and 21i is the center of incisal edge of the right mandibular central incisor (right lower 1^{st}).

When a length of the line segment pq in Fig. 8 is set to be 1, a vertical distance l1(L-1) to a standard planar surface 28 from the standard planar surface 28 to the center of incisal edge 21e of the left mandibular central incisor (left lower 1^{st}), and a vertical distance r1 to a standard planar surface 28 from the standard planar surface 28 to the center of incisal edge 21i of the right mandibular central incisor (right lower 1^{st}) are preferably 0.12 to 0.20, and more preferably 0.14 to 0.18, respectively (see Figs. 7(a) and 7(b)). When the distance is outside a range of 0.12 to 0.20, an occlusal force is reduced in some cases.

In Fig. 11, 23b is the left mandibular canine (left lower 3^{rd}), and 23e is the pointed cuspid of the left mandibular canine (left lower 3^{rd}). 23f is the right mandibular canine (right lower 3^{rd}), and 23i is the pointed cuspid of the right mandibular canine (right lower 3^{rd}).

When a length of the line segment pq in Fig. 8 is set to be 1, a vertical distance 13 (L-3) to a standard planar surface 28 from the standard planar surface 28 to the pointed cuspid 23e of the left mandibular canine (left lower 3^{rd}), and a vertical distance r3 to a standard planar surface 28 from the standard planar surface 28 to the pointed cuspid 23i of the right mandibular canine (right lower 3^{rd}) are preferably 0.12 to 0.20, and more preferably 0.14 to 0.18, respectively (see Figs. 7(a) and 7(b)). When the distance is outside a range of 0.12 to 0.20, an occlusal force is reduced in some cases.

### 4. With Regard to Mandibular Standard Denture

The mandibular standard denture of the present invention is such that artificial teeth of a whole teeth row have been arranged on the mandibular standard dental plate in advance. Positions at which these artificial teeth are arranged are not particularly limited, but it is preferable that they are arranged at the positions as described above.

### 5. With Regard to Denture Fabrication Kit

The denture fabrication kit of the present invention consists of the maxillary standard dental plate or the maxillary standard denture of the present invention, and/or the mandibular standard dental plate or the mandibular standard denture of the present invention, and may include a lining material and an artificial tooth. When the standard dental plate is used, a denture is fabricated by building up a lining material in conformity with the oral cavity size of a patient, and arranging an artificial tooth. When the standard denture is used, the denture is fabricated by building up a lining material in conformity with the oral cavity shape of a patient.

In the denture fabrication kit of the present invention, it is preferable that a plurality of maxillary standard dental plates or maxillary standard dentures and mandibular standard dental plates or mandibular standard dentures having the different sizes have been prepared in advance, respectively. Thereby, the matching rate to a patient can be further increased. In addition, as the size of the maxillary standard dental plate, the maxillary standard denture, the mandibular standard dental plate, and the mandibular standard denture, preferably 2 to 10 of kinds have been prepared, more preferably 2 to 5 kinds have been prepared, and particularly preferably 3 kinds have been prepared, respectively.

### (1) Material of Standard Dental Plate

As a material of the standard dental plate in the present invention, the known materials can be used without any limitation. Specific examples include poly(meth)acrylate-based resins, at least one of which is selected from homopolymers of methyl (meth) acryl ate, ethyl(meth)acrylate, isopropyl (meth)acrylate, n-propyl (meth)acrylate, and butyl (meth)acrylate, or copolymers of them; polyolefin-based resins (e.g. polypropylene); polyamide-based resins (e.g. nylon 66 (registered trademark)); polyester-based resins (e.g. polycarbonate); polyether-based resins (e.g. polyacetal, polysulfone); polynitrile-based resins (e.g. polyacrylonitrile); polyvinyl-based resins (e.g. polyvinyl acetate); cellulose-based resins (e.g. cellulose acetate); fluorine-based resins (e.g. polychlorofluoroethylene); imide-based resins (e.g. aromatic polyimide), and the like.

The standard dental plate in the present invention can also contain a filler, in order to improve the strength of the dental plate. The filler may be an organic filler or an inorganic filler. Alternatively, a particulate organic-inorganic composite filler which is obtained by adding a polymerizable monomer to an inorganic filler in advance, preparing the mixture into a paste, polymerizing the paste, and grinding the polymerization product may be used.

In the standard dental plate in the present invention, a part thereof may be formed of metal.

The standard dental plate in the present invention can be manufactured by the known molding method. Examples thereof include injection molding, compression molding, and the like.

### (2) Lining Material

As the lining material, the known material may be appropriately selected, depending on the use purpose. For example, when a patient himself/herself conducts an operation, and uses the denture in a relatively short term such as one day to a few days, a lining material which is a powdery, creamy or seal-like pasting material for fixing a dental plate (so-called denture adhesives) can be selected. Alternatively, it is also possible to select, as the lining material, a tissue conditioning material, a soft lining material, and a hard lining material which are used by a dentist for treatment. Usually, a material which is used for one week to a few weeks is a tissue conditioning material, and a material which is used for a long term of 6 months or longer is a soft lining material.

### (2-1) Lining Material Having Durometer A Hardness of 55 or Less

As a lining material constituting the denture fabrication kit of the present invention, a lining material in which the durometer A hardness after 24 hours measured in accordance with JIS T 6520 (Long-term soft lining materials for removable dentures) is 55 or less is preferable. The lining material having the durometer A hardness of 55 or less is used, until the oral cavity mucous membrane is restored to the healthy state while pain is alleviated, in the case where the ulcer or the inflammation exists in the oral cavity mucous membrane. Particularly, when alleviation of the burden of a patient and the like are considered, the lining material having the durometer A hardness of 50 or less is preferable, and the lining material having the durometer A hardness of 45 or less is more preferable.

Examples of the lining material having the durometer A hardness of 55 or less include a pasting material for stabilizing a dental plate (so-called denture adhesives), a tissue conditioning material, a soft lining material, and the like.

As the pasting material for stabilizing a dental plate, there are a sticky adhesive type containing a powder-type, cream-type, sheet-type or tape-type water-soluble polymer as a sticky adhesive component, and a water-insoluble paste type, and such pasting material is used by a patient himself/herself conducts an operation for alleviating pain, and is used for a very short term such as one day to a few days. In the present invention, a paste-like material can be suitably used.

As the tissue conditioning material and the soft lining material, there are a material which is used by mixing a powder material and a liquid material, and a paste-like material, and an operation is conducted by a dentist. The tissue conditioning material is used for one week to a few weeks until the oral cavity mucous membrane is restored to the healthy state while pain is alleviated, when the ulcer or the inflammation exists in the oral cavity mucous membrane, and the soft lining material is used after the ulcer or the inflammation of the oral cavity mucous membrane has finally disappeared as mentioned above, and the oral cavity mucous membrane becomes the healthy state, and is used for a long term such as 6 months or longer.

In addition, the pasting material for stabilizing a dental plate (so-called denture adhesives), the tissue conditioning material, and the soft lining material may be appropriately selected, depending on the objective utility and term.

These tissue conditioning material and soft lining material have fundamentally the same respective components to be used, have different amounts of the components, and can be classified into a tissue conditioning material for comparatively short term use and a soft lining material for long term use. In the present invention, the tissue conditioning material refers to those satisfying the depth of penetration and the depth of penetration ratio in accordance with JIS T 6519 (Short Term Resilient Lining Materials for removable dentures). Additionally, the soft lining material refers to those satisfying the durometer A hardness after 24 hours and the durometer A hardness after 28 days in accordance with JIS T 6520. A main component constituting the tissue conditioning material and the soft lining material is a polymer (resin). Additionally, a plasticizer, a monomer, a polymerizable initiator, and the like are contained, depending on the kind of the polymer (resin).

The main component constituting the tissue conditioning material and the soft lining material is a polymer (resin), and the known polymers can be used without any limitation. A specific example of polymer (resin) includes a silicone-based resin, a (meth)acrylate-based resin, a fluorine-based resin, a polyolefin-based resin, and the like. Inter alia, from a view point of easy handling, a silicone-based resin, and a (meth)acrylate-based resin are preferable.

Examples of the silicone-based resin include a condensation reaction-type or addition reaction-type silicone rubber. Since no byproduct such as an alcohol is generated, and polymerization heat production is not generated at curing, the lining material consisting of the addition-type silicone rubber is more preferable. The lining material consisting of the addition-type silicone rubber is usually composed of organopolysiloxane having two or more organic groups having an unsaturated bond at the terminal in a molecule, organohydrogenpolysiloxane having three or more SiH groups in a molecule, and hydrosilylation reaction catalyst substance.

When representative organopolysiloxane having an organic group having an unsaturated bond at the terminal is specifically exemplified,

examples thereof include organopolysiloxane represented by the following chemical formula: 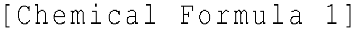 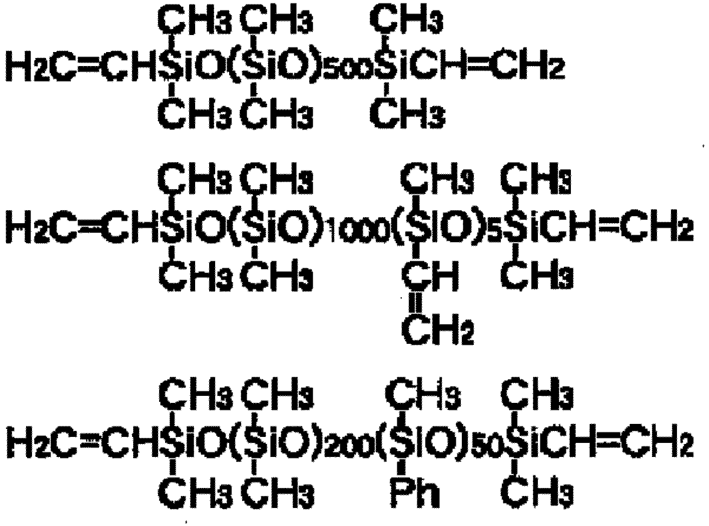 (wherein Ph denotes a phenyl group)
and the like. In addition, a binding order of each repetition constituent unit in the above compound is entirely arbitrary, and the number of repetition constituent units shown in the structural formula merely shows an average of a total amount of each constituent unit.

When representative organohydrogenpolysiloxane having three or more SiH groups in a molecule is specifically exemplified,

examples thereof include organohydrogenpolysiloxane represented by the following chemical formula: 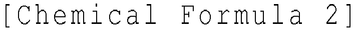 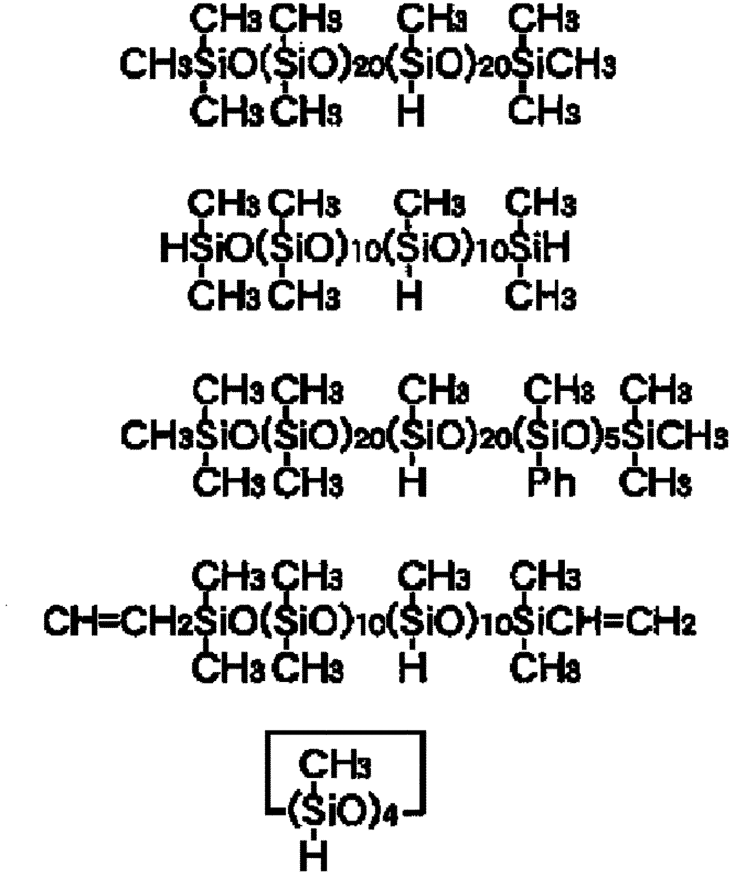 and the like. Also, in the above compound, a binding order of each repetition constituent unit in a molecule is entirely arbitrary.

Specific examples of the hydrosilylation reaction catalyst substance include chloroplatinic acid, an alcohol-modified product thereof, a vinylsiloxane complex of platinum, and the like.

By mixing the organopolysiloxane, the organohydrogenpolysiloxane, and the hydrosilylation reaction catalyst substance to react (addition-polymerize) them, the reaction product can be used as the lining material. In the above-mentioned addition-type silicone rubber, optionally, a silica-based powder such as silica, ground quartz, diatomaceous earth, and polyorganosilsesquioxane fine particles; inorganic particles such as aluminum oxide, aluminum silicate, iron oxide, zinc oxide, calcium carbonate, zirconium oxide, and carbon black; a coloring material such as a dye and a pigment; a perfume; an antibacterial agent; an antifungal agent, and the like may be incorporated. Inter alia, it is preferable to incorporate inorganic particles. Upon mixing, organopolysiloxane and organohydrogenpolysiloxane are divided into two or more combinations, the hydrosilylation reaction catalyst substance is incorporated into one of them, and those are mixed, thereby, the addition-type silicone rubber (silicone rubber-based lining material) can also be produced.

As such addition-type silicone rubber (silicone rubber-based lining material), those satisfying the durometer A hardness after 24 hours and the durometer A hardness after 28 days in accordance with JIS T 6520 can be suitably used as the soft lining material (long term use). In addition, naturally, by adjusting repetition and a use amount of organopolysiloxane and organohydrogenpolysiloxane, as well as a use amount of the hydrosilylation reaction catalyst substance, and adjusting those materials so that the depth of penetration and the depth of penetration ratio in accordance with JIS T 6519 (Short Term Resilient Lining Materials for removable dentures) are satisfied, the addition-type silicone rubber can also be used as the tissue conditioning material (short time use).

The lining material containing a (meth)acrylate-based resin as a main component is usually composed of a powder material consisting of a poly(meth)acrylate-based resin powder and a liquid material consisting of a plasticizer and a (meth)acrylate-based monomer.

Examples of the poly(meth)acrylate-based resin powder include homopolymers of methyl (meth)acrylate, ethyl (meth)acrylate, isopropyl (meth)acrylate, n-propyl (meth)acrylate, and butyl (meth)acrylate, copolymers of them, and the like. In order to further improve softness of the lining material, a poly(meth)acrylate-based resin powder having a glass transition temperature in a range of 0 to 60°C is preferable. Specific examples of the poly(meth)acrylate-based resin powder having a glass transition temperature in a range of 0 to 60°C include homopolymers such as poly(propyl methacrylate), poly(n-butyl methacrylate), poly(i-butyl methacrylate), poly(neopentyl methacrylate), poly(cyclohexyl methacrylate), poly(hexadecyl methacrylate), and poly(octadecyl methacrylate); copolymers such as poly(methyl methacrylate-n-butyl acrylate), poly(methyl methacrylate-n-butyl methacrylate), poly(ethyl methacrylate-n-butyl methacrylate), poly(propyl methacrylate-n-butyl methacrylate), and poly(styrene-n-butyl methacrylate); and the like. These are preferably a non-crosslinking polymer.

Examples of the plasticizer include phthalic acid esters such as ethyl phthalate, butyl phthalate, and octyl phthalate; sebacic acid esters such as ethyl sebacate, butyl sebacate, and octyl sebacate; water-insoluble liquid polymers; and the like. Among them, from a view point of prevention of deterioration due to dissolution or migration to the dental plate, the plasticizer is preferably the water-insoluble liquid polymer. Examples of the water-insoluble liquid polymer include homopolymers or copolymers obtained by polymerizing at least one monomer selected from ethyl (meth)acrylate, propyl (meth)acrylate, isopropyl (meth)acrylate, butyl (meth)acrylate, 2-ethylhexyl (meth)acrylate, methoxyethyl (meth)acrylate, ethoxymethyl (meth)acrylate, and glycidyl (meth)acrylate, having a mass average molecular weight of 1000 to 10000, and the ratio of an oligomer having a molecular weight of 500 or less of 10% by mass or less (plasticizer) .

Examples of the (meth)acrylate-based monomer include: monofunctional (meth)acrylate-based monomers such as methyl (meth)acrylate, ethyl (meth)acrylate, isopropyl (meth)acrylate, hydroxyethyl (meth)acrylate, β-methacryloyloxyethyl propionate, and perfluorooctylethyl methacrylate; difunctional (meth)acrylate-based monomers such as 2,2-bis(methacryloyloxyphenyl)propane, 2-hydroxyethyl (meth)acrylate, 3-chloro-2-hydroxypropyl (meth)acrylate, ethylene glycol di(meth)acrylate, triethylene glycol di(meth)acrylate, 1,6-hexanediol di(meth)acrylate, and 1,9-nonanediol di(meth)acrylate; and the like.

As the liquid material, in addition to the above-mentioned plasticizer and (meth)acrylate-based monomer, water-soluble organic solvents such as alcohols such as ethanol, isopropyl alcohol, and isobutyl alcohol; ketones such as methyl ethyl ketone and acetone; and amines such as diethanolamine and triethanolamine may be incorporated.

Furthermore, in addition to the above-mentioned components, optionally, a chemical polymerization initiator, a photopolymerization initiator, a coloring material such as a dye and a pigment, a perfume, an antibacterial agent, an antifungal agent, and the like may be incorporated.

The lining material containing the (meth)acrylate-based resin as a main component can be produced by mixing a powder material consisting of the poly(meth)acrylate-based resin powder, a liquid material consisting of the plasticizer and/or the (meth)acrylate-based monomer, and other components which are optionally incorporated, and curing (solidifying) the mixture. The lining material containing such (meth)acrylate-based resin as a main component can be prepared into a soft lining material (long term use) satisfying the durometer A hardness after 24 hours and the durometer A hardness after 28 days in accordance with JIS T 6520 by adjusting an incorporation amount of each component, or can also be prepared into a tissue conditioning material (short term use) satisfying the depth of penetration and the depth of penetration ratio in accordance with JIS T 6519 (Short Term Resilient Lining Materials for removable dentures) .

In the present invention, the soft lining material refers to those having the durometer A hardness of 55 or less, and satisfying the durometer A hardness after 24 hours and the durometer A hardness after 28 days in accordance with JIS T 6520. Inter alia, from a view point of long term use, the preferable durometer A hardness after 24 hours is 20 to 50, and the preferable durometer hardness after 28 days is 20 to 60.

It is preferable that this soft lining material is produced from a silicone rubber-based resin including organopolysiloxane having two or more organic groups having an unsaturated bond at the terminal in a molecule, organohydrogenpolysiloxane having three or more SiH groups in a molecule, and a hydrosilylation reaction catalyst substance.

The ratio of organopolysiloxane, organohydrogenpolysiloxane, and the hydrosilylation reaction catalyst substance is not particularly limited as long as the durometer A hardness after 24 hours and the durometer A hardness after 28 days in accordance with JIS T 6520 are satisfied, but it is preferable that the total number of hydrogen atoms constituting a SiH group of organohydrogenpolysiloxane is 0.5 to 5 times, based on the total number of terminal unsaturated bonds of organopolysiloxane. Additionally, it is preferable that the hydrosilylation reaction catalyst substance is in a range of 0.1 to 1000 ppm, based on a total mass of organopolysiloxane and organohydrogenpolysiloxane.

It is preferable that inorganic particles such as silica are incorporated at 1 to 60 parts by mass, based on a total of 100 mass of organopolysiloxane and organohydrogenpolysiloxane.

In the present invention, the tissue conditioning material refers to those having the durometer A hardness of 55 or less, and satisfying the depth of penetration and the depth of penetration ratio in accordance with JIS T 6519 (Short Term Resilient Lining Materials for removable dentures). Inter alia, the preferable durometer A hardness is 20 or less. It is preferable that the depth of penetration after 2 hours is 1.8 mm or less, the depth of penetration after 7 days is 0.18 mm or more, and the depth of penetration ratio is 5.0 or less.

This tissue conditioning material is a lining material which is packaged and stored by division into a powder material and a liquid material, and is used by mixing both materials upon use. It is preferable that the powder material includes a (meth) acryl-based powdery non-crosslinking polymer having a glass transition temperature in a range of 0 to 60°C, and it is preferable that the liquid material includes a liquid polymer having a mass average molecular weight in a range of 1000 to 10000, and the ratio of an oligomer having a molecular weight of 500 or less of 10% by mass or less.

The suitable ratio of the powder material and the liquid material is not particularly limited as long as the depth of penetration and the depth of penetration ratio in accordance with JIS T 6519 (Short Term Resilient Lining Materials for removable dentures) are satisfied, and when the non-crosslinking polymer is contained as the powder material, the liquid material is mixed preferably in a range of 50 to 300 parts by mass, and more preferably in a range of 80 to 200 parts by mass, based on 100 parts by mass of the non-crosslinking polymer. It is preferable that 0 to 30 parts by mass (more preferably, 3 to 20 parts by mass) of alcohols, and 0 to 40 parts by mass (more preferably, 10 to 30 parts by mass) of a plasticizer are contained, based on 100 parts by mass of the non-crosslinking polymer.

### (2-2) Hard Lining Material

As the lining material constituting the denture fabrication kit of the present invention, a lining material having the Knoop hardness measured according to JIST6521 (Denture base hard relining materials) of 7 Hk or more (hereinafter, simply referred to as hard lining material) can be used. Particularly, it is preferable to use the hard lining material having the Knoop hardness of 7 Hk or more and 30 Hk or less.

Such a hard lining material is generally composed of a liquid containing, as a main component, a polymerizable monomer having a radical polymerizable group such as a (meth) acrylate group (methacrylic group, or acrylic group); and a solid (powder) containing, as a main component, a polymer consisting of a polymerizable monomer having a radical polymerizable group such as a (meth)acrylate group, and including a radical polymerization initiator such as organic peroxide.

As the polymerizable monomer having a radical polymerizable group (hereinafter, also referred to as radical polymerizable monomer), the known ones can be used without particular limitation.

Examples thereof include radical polymerizable monomers having a radical polymerizable group such as a vinyl group, and a vinyl group having a substituent (e.g. styryl group, vinyl chloride group, vinyl acetate group, (meth)acrylate group, (meth)acrylamide group). Among them, the radical polymerizable monomer having a (meth)acrylate group is preferable. As the (meth)acrylate-based polymerizable monomer, the known (meth) acrylate-based polymerizable monomer which can be generally used in a dental restorative material can be used without any limitation.

Specific examples of the (meth)acrylate-based polymerizable monomer include n-octadecyl (meth)acrylate, n-dodecyl (meth)acrylate, n-tridecyl (meth)acrylate, n-hexyl (meth)acrylate, cyclohexyl (meth)acrylate, 2-methacryloxyethyl propionate, methoxyethylene glycol (meth)acrylate, ethoxyethylene glycol (meth)acrylate, ethylene glycol di(meth)acrylate, diethylene glycol di(meth)acrylate, triethylene glycol di(meth)acrylate, tetraethylene glycol di(meth)acrylate, pentaethylene glycol di(meth)acrylate, propylene glycol di(meth)acrylate, hexamethylene glycol di(meth)acrylate, acetoacetoxyethyl (meth)acrylate, 1,3-butanediol di(meth)acrylate, 1,4-butanediol di(meth)acrylate, 1,6-hexanediol di(meth)acrylate, 1,9-nonanediol di(meth)acrylate, 1,10-decanediol di(meth)acrylate, 1,12-stearyl di(meth)acrylate, neopentyl glycol di(meth)acrylate, dimer diol di(meth)acrylate, bisphenol A di(meth)acrylate, 2,2-bis[(meth)acryloyloxypolyethoxyphenyl]propane, trimethylolpropane tri(meth)acrylate, trimethylolethane tri(meth)acrylate, tetramethylolmethane tetra(meth)acrylate, urethane di(meth)acrylate, a reaction product of hydroxyethyl (meth)acrylate, 2,2,4-trimethylhexyl-1,6-diisocyanate, and the like.

In the hard lining material used in the present invention, a polymerizable monomer having a radical polymerizable group can be used alone, or two or more thereof can be used in combination.

As a polymer which is produced from a radical polymerizable monomer, a polymer which can be swollen or dissolved in the radical polymerizable monomer can be used. As the polymer, a polymer which is produced from a polymerizable monomer having a (meth)acrylate group ((meth) acrylate-based polymer) is preferably used. When the (meth)acrylate-based polymer is exemplified, examples thereof include polymethyl (meth)acrylate, polyethyl (meth)acrylate, methyl (meth)acrylate-ethyl (meth)acrylate-copolymer, methyl (meth)acrylate-butyl (meth)acrylate copolymer, ethyl (meth)acrylate-butyl (meth)acrylate, crosslinking-type polymethyl (meth)acrylate, crosslinking-type polyethyl (meth)acrylate, and the like. Additionally, since it is enough that the polymer is swollen or dissolved in the radical polymerizable monomer, an ethylene-vinyl acetate copolymer, a styrene-butadiene copolymer, an acrylonitrile-styrene copolymer, an acrylonitrile-styrene-butadiene copolymer, and the like can also be used.

In the hard lining material used in the present invention, the above-mentioned polymer can be used alone, or two or more thereof can be used in combination.

As the radical polymerization initiator, any radical polymerization initiator can be used without any limitation, as long as it generates a radical by heating or photoirradiation. Additionally, a radical polymerization initiator which can generate a radical under the room temperature condition by coming in contact with a tertiary amine or the like may be used.

As a substance which generates a radical by heating or coming into contact with a tertiary amine, organic peroxide is exemplified. Examples of the organic peroxide include benzoyl peroxide, 2,4-dichlorobenzoyl peroxide, dilauroyl peroxide, and the like.

As the tertiary amine which generates a radical by coming into contact with these organic peroxides, the known compounds are used without particular limitation. Examples of the tertiary amine compound which is suitably used include anilines such as N,N-dimethylaniline, N,N-diethylaniline, N,N-dipropylaniline, N,N-dibutylaniline, and N-methyl,N-β-hydroxyethylaniline; toluidines such as N,N-dimethyl-p-toluidine, N,N-diethyl-p-toluidine, N,N-dipropyl-p-toluidine, N,N-dibutyl-p-toluidine, p-tolyldiethanolamine, and p-tolyldipropanolamine; anisidines such as N,N-dimethyl-anisidine, N,N-diethyl-p-anisidine, N,N-dipropyl-p-anisidine, and N,N-dibutyl-p-anisidine; morpholines such as N-phenylmorpholine and N-tolylmorpholine; bis(N,N-dimethylaminophenyl)methane, bis(N,N-dimethylaminophenyl) ether, and the like. These tertiary amine compounds may be a salt with an organic acid such as hydrochloric acid, phosphoric acid, acetic acid, and propionic acid.

As the photopolymerization initiator, the known initiator systems such as an α-diketone-reducing agent, a ketal-reducing agent, and a thioxanthone-reducing agent are preferably used. Examples of the α-diketone include camphorquinone, benzil, 2,3-pentadione, 3,4-heptadione, and the like. Examples of the ketal include benzyldimethylketal, benzyldiethylketal, benzyl (2-methoxyethylketal), and the like. Examples of the thioxanthone include thioxanthone, 2,4-dimethylthioxanthone, 2,4-diethylthioxanthone, 2-chlorothioxanthone, and the like. Examples of the reducing agent which is one component of the photopolymerization initiator include tertiary amines such as 2-(dimethylamino)ethyl methacrylate, ethyl 4-dimethylaminobenzoate, and N-methyldiethanolamine; aldehydes such as laurylaldehyde, dimethylaminobenzaldehyde, and terephthalaldehyde; 2-mercaptobenzoxazole, 1-decanethiol, thiosalicylic acid, thiobenzoic acid, and the like.

The hard lining material used in the present invention may contain a filler in order to improve the strength. The filler may be an organic filler or an inorganic filler.

As the organic filler, for example, generally available resin polymers such as poly(meth)acrylate, polycarbonate, and polyester can be used without limitation.

A kind of the inorganic filler is not particularly limited, but can be selected from a reinforcing material and a filler which are added to a general resin composition. Specifically, examples thereof include calcium carbonate, calcium sulfate, magnesium oxide, barium carbonate, barium sulfate, titanium oxide, potassium titanate, barium titanate, aluminum hydroxide, magnesium hydroxide, quartzite powder, glass powder, diatomaceous earth, silica, calcium silicate, talc, alumina, bentonite, zeolite, kaolin clay, mica, and quartz glass.

The hard lining material in the present invention is not particularly limited in its form, and any of a powder-liquid type, a paste/paste type, and a single paste type can be used. A mainstream of a usual hard lining material is a powder-liquid type, and it is composed of a powder component containing, as a main component, the polymer, the radical polymerization initiator, and an optionally incorporated organic or inorganic filler, and a liquid component containing, as a main component, the radical polymerizable monomer. These are used by mixing and kneading a predetermined amount of the powder component and the liquid component upon use.

The hard lining material is not particularly limited as long as it has the Knoop hardness of 7 Hk or more, and it is preferable that it includes 50 to 350 parts by mass of a polymer which is produced from the radical polymerizable monomer, 0.1 to 20 parts by mass of the radical polymerization initiator, and 0 to 20 parts by mass of the organic or inorganic filler, based on 100 parts by mass of the radical polymerizable monomer which is the liquid component.

### (3) Artificial Tooth

In the present invention, as an artificial tooth, the known artificial tooth made of a resin or made of a ceramic can be used. Examples of the artificial tooth made of a resin include artificial teeth formed of a material such as the poly (meth) acrylate-based resin, the polyolefin-based resin, the polyamide-based resin, the polyester-based resin, the polyether-based resin, the polynitrile-based resin, the polyvinyl-based resin, the cellulose-based resin, the fluorine-based resin, the imide-based resin, and the silicone-based resin which are described above as the material of the standard dental plate. As the artificial tooth, connected artificial teeth in which two or more artificial teeth are connected and integrated can also be used. As a method of fixing the artificial tooth, the previously known method such as fitting and adhesion can be used without any limitation.

As the artificial tooth, it is preferable to use connected artificial teeth in which two or more artificial teeth are connected and integrated. The connected artificial teeth for an upper jaw have preferably a tooth row arch shape which satisfies two or more defined in (a) to (m) when arranged on the maxillary standard dental plate of the present invention, more preferably have a tooth row shape which satisfies three or more thereof, and particularly preferably have a tooth row shape which satisfies all of them. The connected artificial teeth for a lower jaw preferably have a tooth row arch shape which satisfies two or more defined in (o) to (aa) when arranged on the mandibular standard dental plate of the present invention, more preferably have a tooth row shape which satisfies three or more thereof, and particularly preferably have a tooth row shape which satisfies all of them.

It is preferable that in artificial teeth constituting the first premolar to the second molar, these four teeth are connected. Similarly, it is preferable that in artificial teeth constituting the right canine to the left canine, these six teeth are connected. Additionally, it is particularly preferable that teeth in the whole teeth row are connected. This is because the arranging time can be shortened.

### 6. Denture Fabrication Method

A method of fabricating a denture using the denture fabrication kit of the present invention will be described below.

First, a maxillary standard dental plate or a maxillary standard denture, and/or a mandibular standard dental plate or a mandibular standard denture matching the oral cavity size of a patient are (is) selected, respectively. Selection of such standard dental plate or standard denture can be determined based on measurement of the oral cavity size. As a method of measuring the oral cavity size, the known method can be used. Examples thereof include measurement by visual observation and palpation, measurement of the size by try-in of an impression tray, and the like. When the standard dental plate is selected, artificial teeth are arranged on these selected maxillary standard dental plate base and mandibular standard dental plate, respectively, to fabricate a maxillary denture and a mandibular denture. Furthermore, a lining material is built up on the maxillary denture and the mandibular denture, respectively, to completely fit to the oral cavity shape of a patient. Either of arrangement of the artificial teeth and building up of the lining material may be performed first. Then, after try-in in the oral cavity of a patient, optionally, occlusion is adjusted. Thereby, the denture fitting an oral cavity shape of a patient is fabricated.

### Examples

### (Matching Rate)

In the present Examples, the matching rate was evaluated by the ratio of the number of matching models upon try-in in 20 kinds of edentulous jaw full mouth models having different shapes, after lining of the standard dental plate or the standard denture.

### (Arrangement Time)

In the present Examples, the arrangement time was evaluated by the ratio of the time required for arranging artificial teeth on the standard dental plate of each Example, when the time required for arranging artificial teeth of the whole teeth row upon fabrication of a custom-made denture in Reference Example (previous method) is set to be 100%.

### (Occlusion Adjustment Time)

In the present Examples, the occlusion adjustment time was evaluated by the ratio of the time required for adjusting occlusion in the standard dental plate of each Example, when the time required for adjusting occlusion upon fabrication of a custom-made denture (previous method) is set to be 100%.

### (Mucous Membrane Matching Rate)

In the present Examples, the mucous membrane matching rate was evaluated by the ratio of the number of models in which a maximum thickness of a palate-covering part can be in a range of 1 to 3 mm, when the palate-covering part is formed on the standard dental plate so as to match 20 kinds of edentulous jaw full mouth models having different shapes.

### (Plate Posterior Border Cutting Out Time)

In the present Examples, the plate posterior border cutting out time was evaluated by the ratio of the time required for cutting out the plate posterior border of the standard dental plate of each Example, when the plate posterior border cutting out time required upon fabrication of the custom-made denture (Reference Example) is set to be 100%.

### (Cleanability)

Into 10 g of a salad oil, 0.5 g of green dried seaweed was mixed to prepare artificial food debris. This artificial food debris was coated on a denture with a brush. 1.0 g of toothpaste (White & White, manufactured by Lion Corporation) was attached to a tooth brush, and a denture artificial tooth part was brushed while water was appropriately poured thereon. The green dried seaweed removal time in a custom-made denture was set to be 100%, and the time required until removal was represented by the percentage.

### (Occlusion Degree)

Maxillary/mandibular standard dentures were set on a model, and foods which can be masticated were scored, letting French bread to be score 5, glutinous rice to be score 4, boiled rice to be score 3, soybean curd to be score 2, and soup to be score 1.

### (Durometer A Hardness)

With regard to the durometer A hardness which is an index of softness of the lining material, the hardness after 24 hours from mixing was measured based on JIS-T6520 (Long-term soft lining materials for removable dentures).

### (tanδ: Evaluation of Reduction in Pain)

An index for buffering impact of an occlusal or chewing pressure (i.e. evaluation of reduction in pain) was evaluated by tanδ. With regard to dentures after lining of each Example and Comparative Example, tanδ was measured using a repeated load testing machine (ElectroPuls, manufactured by Instron) under the conditions of maximum load pressure: 13.6 kg, minimum load pressure: 0 kg, frequency: 1.2 Hz, temperature: 37°C (in water), and cycle: 3000 times. This evaluation was performed using 20 kinds of edentulous jaw full mouth models. This tanδ is in the proportional relationship with an amount of the lining material capable of buffering impact, and as the numerical value is larger, more impact can be buffered, and as a result, pain can be reduced. For that reason, larger numerical value of tanδ is better, and when tanδ is 0.01 or more, and more preferably 0.03 or more, impact can be sufficiently buffered, and pain at fitting can be reduced.

### (Durability)

With regard to durability, a load (maximum load pressure: 13.6 kg, minimum load pressure: 0 kg, frequency: 1.2 Hz, temperature: 37°C (in water)) was applied to dentures which had been lined in each Example and Comparative Example using a repeated load testing machine (ElectroPalace, manufactured by Instron). When damage of the dentures, such as cracking, breaking away, peeling, etc. was generated at predetermined times (300000 times, 350000 times, 950000 times), it was evaluated as ×, and when the damage was not generated, it was evaluated as ○. This durability was evaluated by adapting the denture to 20 kinds of edentulous jaw full mouth models. When among dentures matching edentulous jaw full mouth models, one or more teeth were damaged, it was evaluated as ×, and when all matched dentures were not damaged, it was evaluated as ○.

### (Examples 1 to 20, Comparative Examples 1 to 4)

Maxillary standard dental plates having shapes described in Table 1 were fabricated, and the matching rate, the arrangement time, and the occlusion adjustment time were evaluated. The evaluation results are shown in Table 1. In addition, the shape of the denture is bilaterally symmetric.

### (Examples 21 to 40, Comparative Examples 5 to 8)

Mandibular standard dental plates having shapes described in Table 2 were fabricated, and the matching rate, the arrangement time, and the occlusion adjustment time were evaluated. The evaluation results are shown in Table 2. In addition, a shape of the denture is bilaterally symmetric.

In Tables, 6^{th} is the first molar, 1^{st} is the central incisor and 3^{rd} is the canine. Additionally, "0" in the case of the first molar (6^{th}) or the canine (3^{rd}) means that the central fossa (in the case of the first molar) or the pointed cuspid (in the case of the canine) of the artificial tooth is arranged so as to be a center in the predetermined circle defined in the present application. Additionally, for example, "0.14" in the first molar (6^{th}) means a distance from a center in the predetermined circle as defined in the present application to the central fossa. Other numerical values have the same meaning. Additionally, for example, "0.12" in the canine (3^{rd}) means a distance from a center in the predetermined circle as defined in the present application to the pointed cuspid. Other numerical values have the same meaning. In the case of the maxillary central incisor, "0.95" refers to that teeth are arranged so as to have an interdental part between the left maxillary central incisor and the right maxillary central incisor at a distance of 0.95 from a point M to a labial side on a straight line OM, and "1.14" and "0.76" refer to that teeth are arranged so that an interdental part between the left maxillary central incisor and the right maxillary central incisor is positioned at a distance of the described value, respectively. In the case of the mandibular central incisor, "0.84" refers to that teeth are arranged so that an interdental part between the left mandibular central incisor and the right mandibular central incisor is positioned at a distance of 0.84 from a point m to the labial side on a straight line om, and "0.94" and "0.74" refer to that teeth are arranged so that an interdental part between the left mandibular central incisor and the right mandibular central incisor is positioned at a distance of the described value, respectively.

### [Table 1]

**(Table 1)**

| | Shape of dental plate | | | | Arrangement of artificial teeth | | | | Matching rate (%) | Arrangement time (%) | Occlusion adjustment time (%) |
|---|---|---|---|---|---|---|---|---|---|---|---|
| | OM | P1D1 | P2D2 | P3D3 | 6^{th} | 1^{st} | 3^{rd} | Other tooth rows | | | |
| Example 1 | 0.87 | 0.24 | 0.32 | 0.31 | Unarranged | | | | 90 | 100 | 100 |
| Example 2 | 0.98 | 0.24 | 0.32 | 0.31 | | | | | 50 | 100 | 100 |
| Example 3 | 0.76 | 0.24 | 0.32 | 0.31 | | | | | 50 | 100 | 100 |
| Example 4 | 0.87 | 0.36 | 0.45 | 0.45 | | | | | 60 | 100 | 100 |
| Example 5 | 0.87 | 0.11 | 0.19 | 0.16 | | | | | 60 | 100 | 100 |
| Example 6 | 0.87 | 0.08 | 0.15 | 0.10 | | | | | 40 | 100 | 100 |
| Example 7 | 0.87 | 0.42 | 0.51 | 0.51 | | | | | 40 | 100 | 100 |
| Example 8 | 0.87 | 0.24 | 0.32 | 0.31 | 0 | Unarranged | Unarranged | Unarranged | 90 | 90 | 90 |
| Example 9 | 0.87 | 0.24 | 0.32 | 0.31 | 0 | 0.95 | Unarranged | Unarranged | 90 | 70 | 80 |
| Example 10 | 0.87 | 0.24 | 0.32 | 0.31 | 0 | 0.95 | 0 | Unarranged | 90 | 50 | 70 |
| Example 11 | 0.87 | 0.24 | 0.32 | 0.31 | 0.14 | 0.95 | 0 | Unarranged | 90 | 50 | 75 |
| Example 12 | 0.87 | 0.24 | 0.32 | 0.31 | 0.08 | 0.95 | 0 | Unarranged | 90 | 50 | 70 |
| Example 13 | 0.87 | 0.24 | 0.32 | 0.31 | 0 | 1.14 | 0 | Unarranged | 90 | 50 | 75 |
| Example 14 | 0.87 | 0.24 | 0.32 | 0.31 | 0 | 0.76 | 0 | Unarranged | 90 | 50 | 75 |
| Example 15 | 0.87 | 0.24 | 0.32 | 0.31 | 0 | 0.95 | 0.12 | Unarranged | 90 | 50 | 75 |
| Example 16 | 0.87 | 0.24 | 0.32 | 0.31 | 0 | 0.95 | 0.07 | Unarranged | 90 | 50 | 70 |
| Example 17 | 0.87 | 0.24 | 0.32 | 0.31 | 0 | 0.95 | 0 | Unarranged | 90 | 0 | 50 |
| Example 18 | 0.87 | 0.24 | 0.32 | 0.31 | 0.16 | Unarranged | Unarranged | Unarranged | 90 | 90 | 100 |
| Example 19 | 0.87 | 0.24 | 0.32 | 0.31 | 0.16 | 0.70 | Unarranged | Unarranged | 90 | 70 | 105 |
| Example 20 | 0.87 | 0.24 | 0.32 | 0.31 | 0.16 | 0.70 | 0.14 | Unarranged | 90 | 50 | 115 |
| Comp. Ex 1 | 0.70 | 0.24 | 0.32 | 0.31 | Unarranged | | | | 10 | 100 | 100 |
| Comp. Ex 2 | 1.05 | 0.24 | 0.32 | 0.31 | | | | | 10 | 100 | 100 |
| Comp. Ex 3 | 0.70 | 0.08 | 0.15 | 0.10 | | | | | 5 | 100 | 100 |
| Comp. Ex 4 | 1.05 | 0.42 | 0.51 | 0.51 | | | | | 5 | 100 | 100 |
| Ref. Ex | Custom-made | | | | | | | | 10 | 100 | 100 |

### [Table 2]

**(Table 2)**

| | Shape of dental plate | | | | | | | Arrangement of artificial teeth | | | | Matching rate (%) | Arrangement time (%) | Occlusion adjustment time (%) |
|---|---|---|---|---|---|---|---|---|---|---|---|---|---|---|
| | om | p1d1 | p2d2 | p3d3 | b1d1 | b2d2 | b3d3 | 6^{th} | 1^{st} | 3^{rd} | Other tooth rows | | | |
| Example 21 | 0.85 | 0.22 | 0.24 | 0.24 | 0.27 | 0.30 | 0.32 | Unarranged | | | | 90 | 100 | 100 |
| Example 22 | 0.94 | 0.22 | 0.24 | 0.24 | 0.27 | 0.30 | 0.32 | | | | | 50 | 100 | 100 |
| Example 23 | 0.76 | 0.22 | 0.24 | 0.24 | 0.27 | 0.30 | 0.32 | | | | | 50 | 100 | 100 |
| Example 24 | 0.85 | 0.32 | 0.34 | 0.33 | 0.40 | 0.41 | 0.42 | | | | | 60 | 100 | 100 |
| Example 25 | 0.85 | 0.11 | 0.13 | 0.14 | 0.14 | 0.19 | 0.21 | | | | | 60 | 100 | 100 |
| Example 26 | 0.85 | 0.07 | 0.08 | 0.08 | 0.08 | 0.10 | 0.14 | | | | | 40 | 100 | 100 |
| Example 27 | 0.85 | 0.38 | 0.40 | 0.40 | 0.48 | 0.50 | 0.50 | | | | | 40 | 100 | 100 |
| Example 28 | 0.85 | 0.22 | 0.24 | 0.24 | 0.27 | 0.30 | 0.32 | 0 | Unarranged | Unarranged | Unarranged | 90 | 90 | 90 |
| Example 29 | 0.85 | 0.22 | 0.24 | 0.24 | 0.27 | 0.30 | 0.32 | 0 | 0.84 | Unarranged | Unarranged | 90 | 70 | 80 |
| Example 30 | 0.85 | 0.22 | 0.24 | 0.24 | 0.27 | 0.30 | 0.32 | 0 | 0.84 | 0 | Unarranged | 90 | 50 | 70 |
| Example 31 | 0.85 | 0.22 | 0.24 | 0.24 | 0.27 | 0.30 | 0.32 | 0.12 | 0.84 | 0 | Unarranged | 90 | 50 | 75 |
| Example 32 | 0.85 | 0.22 | 0.24 | 0.24 | 0.27 | 0.30 | 0.32 | 0.08 | 0.84 | 0 | Unarranged | 90 | 50 | 70 |
| Example 33 | 0.85 | 0.22 | 0.24 | 0.24 | 0.27 | 0.30 | 0.32 | 0 | 0.94 | 0 | Unarranged | 90 | 50 | 75 |
| Example 34 | 0.85 | 0.22 | 0.24 | 0.24 | 0.27 | 0.30 | 0.32 | 0 | 0.74 | 0 | Unarranged | 90 | 50 | 75 |
| Example 35 | 0.85 | 0.22 | 0.24 | 0.24 | 0.27 | 0.30 | 0.32 | 0 | 0.84 | 0.11 | Unarranged | 90 | 50 | 75 |
| Example 36 | 0.85 | 0.22 | 0.24 | 0.24 | 0.27 | 0.30 | 0.32 | 0 | 0.84 | 0.08 | Unarranged | 90 | 50 | 70 |
| Example 37 | 0.85 | 0.22 | 0.24 | 0.24 | 0.27 | 0.30 | 0.32 | 0 | 0.84 | 0 | Unarranged | 90 | 0 | 50 |
| Example 38 | 0.85 | 0.22 | 0.24 | 0.24 | 0.27 | 0.30 | 0.32 | 0.14 | Unarranged | Unarranged | Unarranged | 90 | 90 | 100 |
| Example 39 | 0.85 | 0.22 | 0.24 | 0.24 | 0.27 | 0.30 | 0.32 | 0.14 | 0.70 | Unarranged | Unarranged | 90 | 70 | 105 |
| Example 40 | 0.85 | 0.22 | 0.24 | 0.24 | 0.27 | 0.30 | 0.32 | 0.14 | 0.70 | 0.13 | Unarranged | 90 | 50 | 115 |
| Comp. Ex 5 | 0.65 | 0.22 | 0.24 | 0.24 | 0.27 | 0.30 | 0.32 | Unarranged | | | | 10 | 100 | 100 |
| Comp. Ex 6 | 1.05 | 0.22 | 0.24 | 0.24 | 0.27 | 0.30 | 0.32 | | | | | 10 | 100 | 100 |
| Comp. Ex 7 | 0.65 | 0.07 | 0.08 | 0.08 | 0.08 | 0.10 | 0.14 | | | | | 5 | 100 | 100 |
| Comp. Ex 8 | 1.05 | 0.38 | 0.40 | 0.40 | 0.48 | 0.50 | 0.50 | | | | | 5 | 100 | 100 |
| Ref. Ex | Custom-made | | | | | | | | | | | 10 | 100 | 100 |

### (Examples 41 to 60, Comparative Example 9)

Maxillary standard dental plates having shapes described in Table 3 were fabricated, and the matching rate, the arrangement time, and the occlusion adjustment time were evaluated. The evaluation results are shown in Table 3. In addition, the shape of the denture is bilaterally symmetric.

### (Examples 61 to 80, Comparative Example 10)

Mandibular standard dental plates having shapes described in Table 4 were fabricated, and the matching rate, the arrangement time, and the occlusion adjustment time were evaluated. The evaluation results are shown in Table 4. In addition, a shape of the denture is bilaterally symmetric.

In Tables, 6-7^{th} is connected artificial teeth in which two teeth of the first molar and the second molar are connected, 4-7^{th} is connected artificial teeth in which four teeth from the first premolar to the second molar are connected, 1-1^{st} is connected artificial teeth in which two teeth of left and right central incisors are connected, 1-3^{rd} is connected artificial teeth in which three teeth from the central incisor to the canine are connected, 3-3^{rd} is connected artificial teeth in which six teeth from the left canine to the right canine are connected, and 7-7^{th} is connected artificial teeth in which all 14 teeth are connected.

### [Table 3]

**(Table 3)**

| | Shape of dental plate | | | | Shape of connected artificial teeth | | | Arrangement of artificial teeth | | | | Matching rate (%) | Arrange ment time (%) | Occlusion Adjustment time (%) |
|---|---|---|---|---|---|---|---|---|---|---|---|---|---|---|
| | OM | P1D1 | P2D2 | P3D3 | Connection place | | Number of teeth (right/left) | 6^{th} | 1^{st} | 3^{rd} | Other tooth rows | | | |
| Example 41 | 0.87 | 0.24 | 0.32 | 0.31 | Molar | 6-7^{th} | 2 teeth/2 teeth | 0 | - | - | 5-5^{th} unarranged | 90 | 60 | 80 |
| Example 42 | 0.87 | 0.24 | 0.32 | 0.31 | Molar | 4-7^{th} | 4 teeth/4 teeth | 0 | - | - | 3-3^{rd} unarranged | 90 | 50 | 70 |
| Example 43 | 0.87 | 0.24 | 0.32 | 0.31 | Anterior teeth | 1-1^{st} Se | 2 teeth | - | 0.95 | - | 2-7^{th} unarranged | 90 | 70 | 90 |
| Example 44 | 0.87 | 0.24 | 0.32 | 0.31 | Anterior teeth | 1-3^{rd} | 3 teeth/3 teeth | - | 0.95 | 0 | 4-7^{th} unarranged | 90 | 60 | 85 |
| Example 45 | 0.87 | 0.24 | 0.32 | 0.31 | Anterior teeth | 3-3^{rd} | 6 teeth | - | 0.95 | 0 | 4-7^{th} unarranged | 90 | 50 | 85 |
| Example 46 | 0.87 | 0.24 | 0.32 | 0.31 | Molar Anterior teeth | 4-7^{th} 3-3^{rd} | 4 teeth/4 teeth 6 teeth | 0 | 0.95 | 0 | Whole teeth row | 90 | 25 | 45 |
| Example 47 | 0.87 | 0.24 | 0.32 | 0.31 | All teeth | 7-7^{th} | 14 teeth | 0 | 0.95 | 0 | Whole teeth row | 90 | 10 | 50 |
| Example 48 | 0.87 | 0.24 | 0.32 | 0.31 | All teeth | 7-7^{th} | 14 teeth | 0.14 | 0.95 | 0 | Whole teeth row | 90 | 10 | 80 |
| Example 49 | 0.87 | 0.24 | 0.32 | 0.31 | All teeth | 7-7^{th} | 14 teeth | 0.08 | 0.95 | 0 | Whole teeth row | 90 | 10 | 80 |
| Example 50 | 0.87 | 0.24 | 0.32 | 0.31 | All teeth | 7-7^{th} | 14 teeth | 0 | 1.14 | 0 | Whole teeth row | 90 | 10 | 75 |
| Example 51 | 0.87 | 0.24 | 0.32 | 0.31 | All teeth | 7-7^{th} | 14 teeth | 0 | 0.76 | 0 | Whole teeth row | 90 | 10 | 75 |
| Example 52 | 0.87 | 0.24 | 0.32 | 0.31 | All teeth | 7-7^{th} | 14 teeth | 0 | 0.95 | 0.12 | Whole teeth row | 90 | 10 | 75 |
| Example 53 | 0.87 | 0.24 | 0.32 | 0.31 | All teeth | 7-7^{th} | 14 teeth | 0 | 0.95 | 0.07 | Whole teeth row | 90 | 10 | 75 |
| Example 54 | 0.87 | 0.24 | 0.32 | 0.31 | All teeth | 7-7^{th} | 14 teeth | 0 | 0.70 | 0.14 | Whole teeth row | 90 | 10 | 105 |
| Example 55 | 0.98 | 0.24 | 0.32 | 0.31 | All teeth | 7-7^{th} | 14 teeth | 0 | 0.95 | 0 | Whole teeth row | 50 | 10 | 50 |
| Example 56 | 0.76 | 0.24 | 0.32 | 0.31 | All teeth | 7-7^{th} | 14 teeth | 0 | 0.95 | 0 | Whole teeth row | 50 | 10 | 50 |
| Example 57 | 0.87 | 0.36 | 0.45 | 0.45 | All teeth | 7-7^{th} | 14 teeth | 0 | 0.95 | 0 | Whole teeth row | 60 | 10 | 50 |
| Example 58 | 0.87 | 0.11 | 0.19 | 0.16 | All teeth | 7-7^{th} | 14 teeth | 0 | 0.95 | 0 | Whole teeth row | 60 | 10 | 50 |
| Example 59 | 0.87 | 0.08 | 0.15 | 0.10 | All teeth | 7-7^{th} | 14 teeth | 0 | 0.95 | 0 | Whole teeth row | 40 | 10 | 50 |
| Example 60 | 0.87 | 0.42 | 0.51 | 0.51 | All teeth | 7-7^{th} | 14 teeth | 0 | 0.95 | 0 | Whole teeth row | 40 | 10 | 50 |
| Comp. Ex 9 | 0.871 | 0.32 | 0.24 | 0.31 | - | | | Unarranged | Unarranged | Unarranged | Unarranged | 90 | 100 | 100 |
| Ref.Ex | Custom-made | | | | | | | | | | | 10 | 100 | 100 |

### [Table 4]

**(Table 4)**

| | Shape of dental plate | | | | | | | Shape of connected artificial teeth | | | Arrangement of artificial teeth | | | | Matching rate (%) | Arrange ment time (%) | Occlusion Adjustment time (%) |
|---|---|---|---|---|---|---|---|---|---|---|---|---|---|---|---|---|---|
| | om | p1d1 | p2d2 | p3d3 | d1e1 | d2e2 | d3e3 | Connection place | | Number of teeth (right/left) | 6^{th} | 1^{st} | 3^{rd} | Other tooth rows | | | |
| Example 61 | 0.85 | 0.22 | 0.24 | 0.24 | 0.27 | 0.30 | 0.32 | Molar | 6-7^{th} | 2 teeth/2 teeth | 0 | - | - | 5-5^{th} unarranged | 90 | 60 | 80 |
| Example 62 | 0.85 | 0.22 | 0.24 | 0.24 | 0.27 | 0.30 | 0.32 | Molar | 4-7^{th} | 4 teeth/4 teeth | 0 | - | - | 3-3^{rd} unarranged | 90 | 50 | 70 |
| Example 63 | 0.85 | 0.22 | 0.24 | 0.24 | 0.27 | 0.30 | 0.32 | Anterior teeth | 1-1^{st} | 2 teeth | - | 0.84 | - | 2-7^{th} unarranged | 90 | 70 | 90 |
| Example 64 | 0.85 | 0.22 | 0.24 | 0.24 | 0.27 | 0.30 | 0.32 | Anterior teeth | 1-3^{rd} | 3 teeth/3 teeth | - | 0.84 | 0 | 4-7^{th} unarranged | 90 | 60 | 85 |
| Example 65 | 0.85 | 0.22 | 0.24 | 0.24 | 0.27 | 0.30 | 0.32 | Anterior teeth | 3-3^{rd} | 6 teeth | - | 0.84 | 0 | 4-7^{th} unarranged | 90 | 50 | 85 |
| Example 66 | 0.85 | 0.22 | 0.24 | 0.24 | 0.27 | 0.30 | 0.32 | Molar Anterior teeth | 4-7^{th} 3-3^{rd} | 4 teeth/4 teeth 6 teeth | 0 | 0.84 | 0 | All teeth | 90 | 25 | 45 |
| Example 67 | 0.85 | 0.22 | 0.24 | 0.24 | 0.27 | 0.30 | 0.32 | All teeth | 7-7^{th} | 14 teeth | 0 | 0.84 | 0 | All teeth | 90 | 10 | 50 |
| Example 68 | 0.85 | 0.22 | 0.24 | 0.24 | 0.27 | 0.30 | 0.32 | All teeth | 7-7^{th} | 14 teeth | 0.12 | 0.84 | 0 | All teeth | 90 | 10 | 80 |
| Example 69 | 0.85 | 0.22 | 0.24 | 0.24 | 0.27 | 0.30 | 0.32 | All teeth | 7-7^{th} | 14 teeth | 0.08 | 0.84 | 0 | All teeth | 90 | 10 | 80 |
| Example 70 | 0.85 | 0.22 | 0.24 | 0.24 | 0.27 | 0.30 | 0.32 | All teeth | 7-7^{th} | 14 teeth | 0 | 0.94 | 0 | All teeth | 90 | 10 | 75 |
| Example 71 | 0.85 | 0.22 | 0.24 | 0.24 | 0.27 | 0.30 | 0.32 | All teeth | 7-7^{th} | 14 teeth | 0 | 0.74 | 0 | All teeth | 90 | 10 | 75 |
| Example 72 | 0.85 | 0.22 | 0.24 | 0.24 | 0.27 | 0.30 | 0.32 | All teeth | 7-7^{th} | 14 teeth | 0 | 0.84 | 0.11 | All teeth | 90 | 10 | 75 |
| Example 73 | 0.85 | 0.22 | 0.24 | 0.24 | 0.27 | 0.30 | 0.32 | All teeth | 7-7^{th} | 14 teeth | 0 | 0.84 | 0.08 | All teeth | 90 | 10 | 75 |
| Example 74 | 0.85 | 0.22 | 0.24 | 0.24 | 0.27 | 0.30 | 0.32 | All teeth | 7-7^{th} | 14 teeth | 0 | 0.70 | 0.13 | All teeth | 90 | 10 | 105 |
| Example 75 | 0.94 | 0.22 | 0.24 | 0.24 | 0.27 | 0.30 | 0.32 | All teeth | 7-7^{th} | 14 teeth | 0 | 0.84 | 0 | All teeth | 50 | 10 | 50 |
| Example 76 | 0.76 | 0.22 | 0.24 | 0.24 | 0.27 | 0.30 | 0.32 | All teeth | 7-7^{th} | 14 teeth | 0 | 0.84 | 0 | All teeth | 50 | 10 | 50 |
| Example 77 | 0.85 | 0.32 | 0.34 | 0.33 | 0.40 | 0.41 | 0.42 | All teeth | 7-7^{th} | 14 teeth | 0 | 0.84 | 0 | All teeth | 60 | 10 | 50 |
| Example 78 | 0.85 | 0.11 | 0.13 | 0.14 | 0.14 | 0.19 | 0.21 | All teeth | 7-7^{th} | 14 teeth | 0 | 0.84 | 0 | All teeth | 60 | 10 | 50 |
| Example 79 | 0.85 | 0.07 | 0.08 | 0.08 | 0.08 | 0.10 | 0.14 | All teeth | 7-7^{th} | 14 teeth | 0 | 0.84 | 0 | All teeth | 40 | 10 | 50 |
| Example 80 | 0.85 | 0.38 | 0.40 | 0.40 | 0.48 | 0.50 | 0.50 | All teeth | 7-7^{th} | 14 teeth | 0 | 0.84 | 0 | All teeth | 40 | 10 | 50 |
| Comp. Ex 10 | 0.85 | 0.22 | 0.24 | 0.24 | 0.27 | 0.30 | 0.32 | - | | | Unarra nged | Unarra nged | Unarra nged | Unarranged | 90 | 100 | 100 |
| Ref.Ex | Custom-made | | | | | | | | | | | | | | 10 | 100 | 100 |

### (Examples 81 to 103, Comparative Example 11)

Mandibular standard dental plates with a guide having shapes described in Table 5 were fabricated, and the matching rate, the arrangement time, and the occlusion adjustment time were evaluated. The evaluation results are shown in Table 5. In addition, a shape of the denture is bilaterally symmetric.

### (Examples 104 to 126, Comparative Example 12)

Mandibular standard dental plates with a guide having shapes described in Table 6 were fabricated, and the matching rate, the arrangement time, and the occlusion adjustment time were evaluated. The evaluation results are shown in Table 6. In addition, a shape of the denture is bilaterally symmetric.

In Tables, 7^{th} is the second molar.

In addition, "0" in the case of the first molar (6^{th}) or the canine (3^{rd}) means that the guide is formed at a center in the predetermined circle as defined in the present application. Additionally, for example, "0.14" in the first molar (6^{th}) means a distance from a center of the predetermined circle as defined in the present application to the guide. Other numerical values have the same meaning. For example, "0.12" in the canine (3^{rd}) means a distance from a center in the predetermined circle as defined in the present application to the guide, and other numerical values have the same meaning. In the case of the maxillary central incisor, "0.95" refers to that the guide is formed at a center in two predetermined circles coming into contact at a point U1 at a distance of 0.95 from a point M to the labial side on a straight line OM, and "1.14" and "0.76" refer to that the guide is formed at a center in two predetermined circles coming into contact with a point U1 at a distance of the described value, respectively.

In the case of the mandibular central incisor, "0.84" refers to that the guide is formed at a center in two predetermined circles coming in contact at a point u1 at a distance of 0.84 from a point m to the labial side on a straight line om, and "0.94" and "0.74" refer to that the guide is formed at a center in two predetermined circles coming into contact with a point u1 at a distance of the described value, respectively.

Additionally, artificial teeth are arranged so that the central fossa (in the case of the first molar), the pointed cuspid (in the case of the canine), or the central mamelon (in the case of the central incisor) of the artificial teeth is positioned at a center of the guide.

### [Table 5]

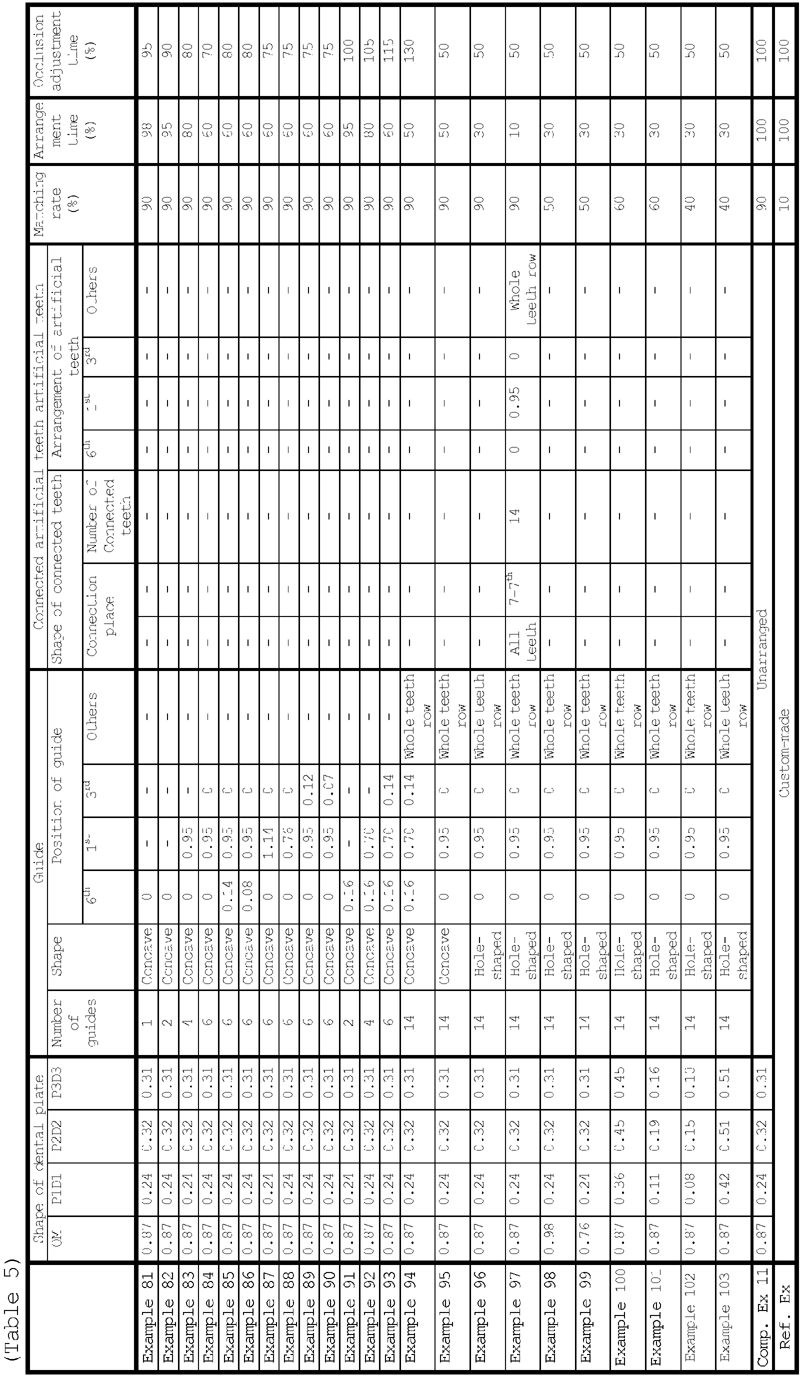

### [Table 6]

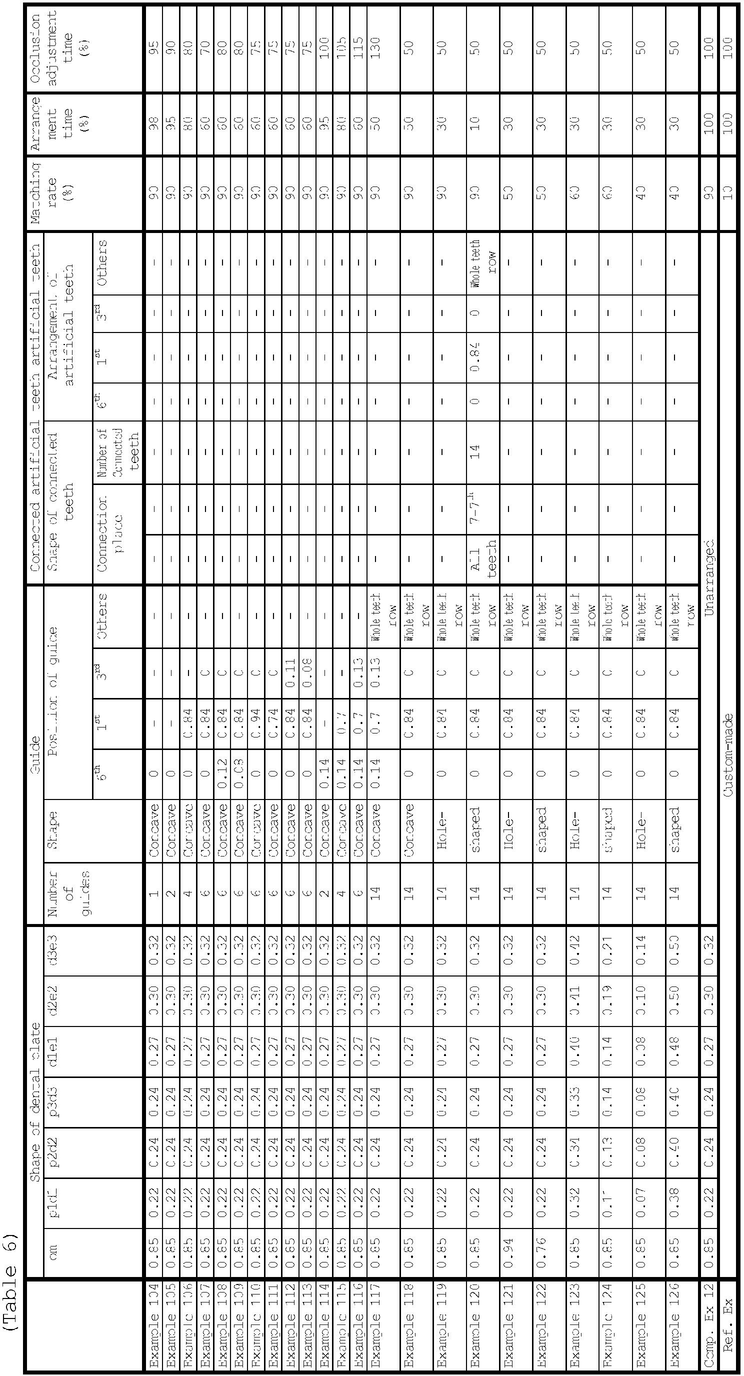

### (Examples 127 to 138, Comparative Examples 13 to 16

Non-palate maxillary standard dental plates having shapes described in Table 7 were fabricated, and the matching rate, the occlusion adjustment time, and the mucous membrane matching rate were evaluated. The evaluation results are shown in Table 7. In addition, a shape of the denture is bilaterally symmetric.

### [Table 7]

**(Table 7)**

| | Shape of dental plate | | | | | | Arrangement of artificial teeth | | | | Matching rate (%) | Arrangement time (%) | Occlusion adjustment time (%) | Mucous membrane matching rate (%) |
|---|---|---|---|---|---|---|---|---|---|---|---|---|---|---|
| | OM | NM | XY | P1D1 | P2D2 | P3D3 | 6^{th} | 1^{st} | 3^{rd} | Other tooth rows | | | | |
| Example 127 | 0.87 | 0.34 | 0.55 | 0.24 | 0.32 | 0.31 | Unarranged | | | | 90 | 100 | 100 | 60 |
| Example 128 | 0.87 | 0.70 | 0.55 | 0.24 | 0.32 | 0.31 | | | | | 90 | 100 | 100 | 100 |
| Example 129 | 0.87 | 0.40 | 0.55 | 0.24 | 0.32 | 0.31 | | | | | 90 | 100 | 100 | 80 |
| Example 130 | 0.87 | 0.85 | 0.55 | 0.24 | 0.32 | 0.31 | | | | | 90 | 100 | 100 | 85 |
| Example 131 | 0.87 | 0.70 | 0.35 | 0.24 | 0.32 | 0.31 | | | | | 90 | 100 | 100 | 70 |
| Example 132 | 0.87 | 0.70 | 0.75 | 0.24 | 0.32 | 0.31 | | | | | 90 | 100 | 100 | 70 |
| Example 133 | 0.87 | 0.70 | 0.40 | 0.24 | 0.32 | 0.31 | | | | | 90 | 100 | 100 | 85 |
| Example 134 | 0.87 | 0.70 | 0.70 | 0.24 | 0.32 | 0.31 | | | | | 90 | 100 | 100 | 90 |
| Example 135 | 0.87 | 0.70 | 0.55 | 0.24 | 0.32 | 0.31 | 0 | Unarranged | Unarranged | Unarranged | 90 | 90 | 90 | 100 |
| Example 136 | 0.87 | 0.70 | 0.55 | 0.24 | 0.32 | 0.31 | 0 | 0.95 | Unarranged | Unarranged | 90 | 70 | 80 | 100 |
| Example 137 | 0.87 | 0.70 | 0.55 | 0.24 | 0.32 | 0.31 | 0 | 0.95 | 0 | Unarranged | 90 | 50 | 70 | 100 |
| Example 138 | 0.87 | 0.70 | 0.55 | 0.24 | 0.32 | 0.31 | 0 | 0.95 | 0 | Whole teeth row | 90 | 0 | 50 | 100 |
| Comp. Ex 13 | 0.87 | 0 | 0 | 0.24 | 0.32 | 0.31 | Unarranged | | | | 90 | 100 | 100 | 40 |
| Comp. Ex 14 | 0.70 | 0.60 | 0.55 | 0.24 | 0.32 | 0.31 | | | | | 10 | 100 | 100 | 55 |
| Comp. Ex 15 | 1.05 | 0.70 | 0.55 | 0.24 | 0.32 | 0.31 | | | | | 10 | 100 | 100 | 50 |
| Comp. Ex 16 | 0.87 | 0 | 0 | 0.24 | 0.32 | 0.31 | 0 | 0.95 | 0 | Whole teeth row | 90 | 0 | 50 | 40 |
| Ref. Ex | Custom-made | | | | | | | | | | | 10 | 100 | 100 |

### (Examples 139 to 145, Comparative Examples 17 and 18)

Maxillary standard dental plates having shapes described in Table 8 were fabricated, and the matching rate, the arrangement time, the occlusion adjustment time, and the plate posterior border cutting out time were evaluated. The evaluation results are shown in Table 8. In addition, a shape of the denture is bilaterally symmetric.

### [Table 8]

**(Table 8)**

| | Shape of dental plate | | | | | Arrangement of artificial teeth | | | | Matching rate (%) | Arrangement time (%) | Occlusion adjustment time (%) | Plate posterior border cutting out time (%) |
|---|---|---|---|---|---|---|---|---|---|---|---|---|---|
| | OM | NM | P1D1 | P2D2 | P3D3 | 6^{th} | 1^{st} | 3^{rd} | Other tooth rows | | | | |
| Example 139 | 0.87 | 0.07 | 0.24 | 0.32 | 0.31 | Unarranged | | | | 90 | 100 | 100 | 60 |
| Example 140 | 0.87 | 0.05 | 0.24 | 0.32 | 0.31 | | | | | 90 | 100 | 100 | 80 |
| Example 141 | 0.87 | 0.10 | 0.24 | 0.32 | 0.31 | | | | | 90 | 100 | 100 | 80 |
| Example 142 | 0.87 | 0.07 | 0.24 | 0.32 | 0.31 | 0 | Unarranged | Unarranged | Unarranged | 90 | 100 | 100 | 60 |
| Example 143 | 0.87 | 0.07 | 0.24 | 0.32 | 0.31 | 0 | 0.95 | Unarranged | Unarranged | 90 | 100 | 100 | 60 |
| Example 144 | 0.87 | 0.07 | 0.24 | 0.32 | 0.31 | 0 | 0.95 | 0 | Unarranged | 90 | 100 | 100 | 60 |
| Example 145 | 0.87 | 0.07 | 0.24 | 0.32 | 0.31 | 0 | 0.95 | 0 | Whole teeth row | 90 | 100 | 100 | 60 |
| Comp. Ex 17 | 0.87 | 0 | 0.24 | 0.32 | 0.31 | Unarranged | | | | 90 | 100 | 100 | 100 |
| Comp. Ex 18 | 0.87 | 0 | 0.24 | 0.32 | 0.31 | 0 | 0.95 | 0 | Whole teeth row | 90 | 90 | 90 | 100 |
| Ref. Ex | Custom-made | | | | | | | | | 10 | 100 | 100 | 100 |

### (Examples 146 to 162, Comparative Examples 19 to 21)

Maxillary standard dentures having shapes described in Table 9 were fabricated, and the matching rate, the occlusion adjustment time, and the cleanability were evaluated. The evaluation results are shown in Table 9. In addition, a shape of the denture is bilaterally symmetric.

### (Examples 163 to 179, Comparative Examples 22 to 24)

Mandibular standard dentures having shapes described in Table 10 were fabricated, and the matching rate, the occlusion adjustment time, and the cleanability were evaluated. The evaluation results are shown in Table 10. In addition, a shape of the denture is bilaterally symmetric.

### [Table 9]

**(Table 9)**

| | Shape of dental plate | | | | Arrangement of artificial teeth | | | | | | | Matching rate (%) | Occlusion adjustment time (%) | Cleanability (%) |
|---|---|---|---|---|---|---|---|---|---|---|---|---|---|---|
| | OM | P1D1 | P2D2 | P3D3 | 6^{th} | 1^{st} | 3^{rd} | 4^{th} | 5^{th} | 7^{th} | 2^{nd} | | | |
| Example 146 | 0.87 | 0.24 | 0.32 | 0.31 | 0 | 0.95 | 0 | 0 | 0.15 | 0.14 | 0.13 | 90 | 45 | 170 |
| Example 147 | 0.87 | 0.24 | 0.32 | 0.31 | 0 | 0.95 | 0 | 0.07 | 0.15 | 0.14 | 0.13 | 90 | 47 | 179 |
| Example 148 | 0.87 | 0.24 | 0.32 | 0.31 | 0 | 0.95 | 0 | 0.13 | 0.15 | 0.14 | 0.13 | 90 | 49 | 191 |
| Example 149 | 0.87 | 0.24 | 0.32 | 0.31 | 0 | 0.95 | 0 | 0 | 0 | 0.14 | 0.13 | 90 | 40 | 145 |
| Example 150 | 0.87 | 0.24 | 0.32 | 0.31 | 0 | 0.95 | 0 | 0 | 0.06 | 0.14 | 0.13 | 90 | 42 | 156 |
| Example 151 | 0.87 | 0.24 | 0.32 | 0.31 | 0 | 0.95 | 0 | 0 | 0.13 | 0.14 | 0.13 | 90 | 44 | 163 |
| Example 152 | 0.87 | 0.24 | 0.32 | 0.31 | 0 | 0.95 | 0 | 0 | 0 | 0 | 0.13 | 90 | 35 | 122 |
| Example 153 | 0.87 | 0.24 | 0.32 | 0.31 | 0 | 0.95 | 0 | 0 | 0 | 0.06 | 0.13 | 90 | 37 | 128 |
| Example 154 | 0.87 | 0.24 | 0.32 | 0.31 | 0 | 0.95 | 0 | 0 | 0 | 0.12 | 0.13 | 90 | 39 | 135 |
| Example 155 | 0.87 | 0.24 | 0.32 | 0.31 | 0 | 0.95 | 0 | 0 | 0 | 0 | 0 | 90 | 30 | 98 |
| Example 156 | 0.87 | 0.24 | 0.32 | 0.31 | 0 | 0.95 | 0 | 0 | 0 | 0 | 0.06 | 90 | 32 | 111 |
| Example 157 | 0.87 | 0.24 | 0.32 | 0.31 | 0 | 0.95 | 0 | 0 | 0 | 0 | 0.11 | 90 | 34 | 117 |
| Example 158 | 0.87 | 0.24 | 0.32 | 0.31 | 0 | 0.95 | 0 | 0 | 0.15 | 0 | 0.13 | 90 | 40 | 120 |
| Example 159 | 0.87 | 0.24 | 0.32 | 0.31 | 0 | 0.95 | 0 | 0 | 0.15 | 0.14 | 0 | 90 | 40 | 121 |
| Example 160 | 0.87 | 0.24 | 0.32 | 0.31 | 0 | 0.95 | 0 | 0 | 0.15 | 0 | 0 | 90 | 35 | 111 |
| Example 161 | 0.87 | 0.24 | 0.32 | 0.31 | 0 | 0.95 | 0 | 0 | 0 | 0.14 | 0 | 90 | 35 | 111 |
| Example 162 | 0.87 | 0.24 | 0.32 | 0.31 | 0 | 0.95 | 0 | 0 | 0 | 0 | 0.13 | 90 | 35 | 110 |
| Comp. Ex 19 | 0.70 | 0.08 | 0.15 | 0.10 | 0 | 0.95 | 0 | 0 | 0 | 0 | 0 | 5 | 115 | 132 |
| Comp. Ex 20 | 1.05 | 0.42 | 0.51 | 0.51 | 0 | 0.95 | 0 | 0 | 0 | 0 | 0 | 5 | 116 | 130 |
| Comp. Ex 21 | 0.87 | 0.24 | 0.32 | 0.31 | 0 | 0.95 | 0 | 0.15 | 0.15 | 0.14 | 0.13 | 90 | 50 | 202 |
| Ref. Ex | Custom-made | | | | | | | | | | | 10 | 100 | 100 |

### [Table 10]

**(Table 10)**

| | Shape of dental plate | | | | | | | Arrangement of artificial teeth | | | | | | | Matching rate (%) | Occlusion adjustment time (%) | Cleanability (%) |
|---|---|---|---|---|---|---|---|---|---|---|---|---|---|---|---|---|---|
| | om | p1d1 | p2d2 | p3d3 | d1b1 | d2b2 | d3b3 | 6^{th} | 1^{st} | 3^{rd} | 4^{th} | 5^{th} | 7^{th} | 2^{nd} | | | |
| Example 163 | 0.85 | 0.22 | 0.24 | 0.24 | 0.27 | 0.30 | 0.32 | 0 | 0.84 | 0 | 0 | 0.14 | 0.14 | 0.12 | 90 | 44 | 169 |
| Example 164 | 0.85 | 0.22 | 0.24 | 0.24 | 0.27 | 0.30 | 0.32 | 0 | 0.84 | 0 | 0.06 | 0.14 | 0.14 | 0.12 | 90 | 46 | 181 |
| Example 165 | 0.85 | 0.22 | 0.24 | 0.24 | 0.27 | 0.30 | 0.32 | 0 | 0.84 | 0 | 0.12 | 0.14 | 0.14 | 0.12 | 90 | 48 | 189 |
| Example 166 | 0.85 | 0.22 | 0.24 | 0.24 | 0.27 | 0.30 | 0.32 | 0 | 0.84 | 0 | 0 | 0 | 0.14 | 0.12 | 90 | 39 | 143 |
| Example 167 | 0.85 | 0.22 | 0.24 | 0.24 | 0.27 | 0.30 | 0.32 | 0 | 0.84 | 0 | 0 | 0.05 | 0.14 | 0.12 | 90 | 42 | 157 |
| Example 168 | 0.85 | 0.22 | 0.24 | 0.24 | 0.27 | 0.30 | 0.32 | 0 | 0.84 | 0 | 0 | 0.12 | 0.14 | 0.12 | 90 | 44 | 165 |
| Example 169 | 0.85 | 0.22 | 0.24 | 0.24 | 0.27 | 0.30 | 0.32 | 0 | 0.84 | 0 | 0 | 0 | 0 | 0.12 | 90 | 36 | 122 |
| Example 170 | 0.85 | 0.22 | 0.24 | 0.24 | 0.27 | 0.30 | 0.32 | 0 | 0.84 | 0 | 0 | 0 | 0.06 | 0.12 | 90 | 37 | 127 |
| Example 171 | 0.85 | 0.22 | 0.24 | 0.24 | 0.27 | 0.30 | 0.32 | 0 | 0.84 | 0 | 0 | 0 | 0.12 | 0.12 | 90 | 39 | 138 |
| Example 172 | 0.85 | 0.22 | 0.24 | 0.24 | 0.27 | 0.30 | 0.32 | 0 | 0.84 | 0 | 0 | 0 | 0 | 0 | 90 | 30 | 95 |
| Example 173 | 0.85 | 0.22 | 0.24 | 0.24 | 0.27 | 0.30 | 0.32 | 0 | 0.84 | 0 | 0 | 0 | 0 | 0.04 | 90 | 33 | 110 |
| Example 174 | 0.85 | 0.22 | 0.24 | 0.24 | 0.27 | 0.30 | 0.32 | 0 | 0.84 | 0 | 0 | 0 | 0 | 0.10 | 90 | 35 | 116 |
| Example 175 | 0.85 | 0.22 | 0.24 | 0.24 | 0.27 | 0.30 | 0.32 | 0 | 0.84 | 0 | 0 | 0.14 | 0 | 0.12 | 90 | 40 | 125 |
| Example 176 | 0.85 | 0.22 | 0.24 | 0.24 | 0.27 | 0.30 | 0.32 | 0 | 0.84 | 0 | 0 | 0.14 | 0.14 | 0 | 90 | 39 | 124 |
| Example 177 | 0.85 | 0.22 | 0.24 | 0.24 | 0.27 | 0.30 | 0.32 | 0 | 0.84 | 0 | 0 | 0.14 | 0 | 0 | 90 | 36 | 110 |
| Example 178 | 0.85 | 0.22 | 0.24 | 0.24 | 0.27 | 0.30 | 0.32 | 0 | 0.84 | 0 | 0 | 0 | 0.14 | 0 | 90 | 35 | 110 |
| Example 179 | 0.85 | 0.22 | 0.24 | 0.24 | 0.27 | 0.30 | 0.32 | 0 | 0.84 | 0 | 0 | 0 | 0 | 0.12 | 90 | 35 | 111 |
| Comp. Ex 22 | 0.65 | 0.07 | 0.08 | 0.08 | 0.08 | 0.10 | 0.14 | 0 | 0.84 | 0 | 0 | 0 | 0 | 0 | 5 | 116 | 133 |
| Comp. Ex 23 | 1.05 | 0.38 | 0.40 | 0.40 | 0.48 | 0.50 | 0.50 | 0 | 0.84 | 0 | 0 | 0 | 0 | 0 | 5 | 117 | 134 |
| Comp. Ex 24 | 0.85 | 0.22 | 0.24 | 0.24 | 0.27 | 0.30 | 0.32 | 0 | 0.84 | 0 | 0.14 | 0.14 | 0.14 | 0.12 | 90 | 50 | 210 |
| Ref. Ex | Custom-made | | | | | | | | | | | | | | 10 | 100 | 100 |

### (Manufacturing Examples 1 to 3, Comparative Manufacturing Examples 1 to 4)

Maxillary standard dentures having shapes described in Table 11 were fabricated, and the matching rate, the arrangement time, and the occlusion adjustment time were evaluated. The evaluation results are shown in Table 11. In addition, a shape of the denture is bilaterally symmetric.

### (Manufacturing Examples 4 to 6, Comparative Manufacturing Examples 5 to 8)

Mandibular standard dentures having shapes described in Table 12 were fabricated, and the matching rate, the arrangement time, and the occlusion adjustment time were evaluated. The evaluation results are shown in Table 12. In addition, a shape of the denture is bilaterally symmetric.

### (Examples 180 to 182, Comparative Examples 25 to 28, Reference Example 2)

Using the maxillary standard dentures and the mandibular standard dentures described in Table 13, an occlusion degree was measured. The results are shown in Table 13.

### [Table 11]

**(Table 11)**

| | Shape of dental plate | | | | Arrangement of artificial teeth | | | | | | | Matching rate (%) | Arrangement time (%) | Occlusion adjustment time (%) |
|---|---|---|---|---|---|---|---|---|---|---|---|---|---|---|
| | OM | P1D1 | P2D2 | P3D3 | 6^{th} | | 1^{st} | | 3^{rd} | | Other tooth rows | | | |
| | | | | | Position | L6 | Position | L1 | Position | L3 | | | | |
| Manufacturing Example 1 | 0.87 | 0.24 | 0.32 | 0.31 | 0 | 0.20 | 0.95 | 0.20 | 0 | 0.20 | Whole teeth row | 90 | 0 | 40 |
| Manufacturing Example 2 | 0.87 | 0.24 | 0.32 | 0.31 | 0 | 0.16 | 0.95 | 0.16 | 0 | 0.16 | Whole teeth row | 90 | 0 | 40 |
| Manufacturing Example 3 | 0.87 | 0.24 | 0.32 | 0.31 | 0 | 0.24 | 0.95 | 0.24 | 0 | 0.24 | Whole teeth row | 90 | 0 | 40 |
| Comparative Manufacturing Example 1 | 0.87 | 0.24 | 0.32 | 0.31 | 0 | 0.12 | 0.95 | 0.12 | 0 | 0.12 | Whole teeth row | 90 | 0 | 40 |
| Comparative Manufacturing Example 2 | 0.87 | 0.24 | 0.32 | 0.31 | 0 | 0.28 | 0.95 | 0.28 | 0 | 0.28 | Whole teeth row | 90 | 0 | 40 |
| Comparative Manufacturing Example 3 | 0.70 | 0.08 | 0.15 | 0.10 | 0 | 0.20 | 0.95 | 0.20 | 0 | 0.20 | Whole teeth row | 5 | 0 | 50 |
| Comparative Manufacturing Example 4 | 1.05 | 0.42 | 0.51 | 0.51 | 0 | 0.20 | 0.95 | 0.20 | 0 | 0.20 | Whole teeth row | 5 | 0 | 50 |
| Reference Manufacturing Example 1 | Custom-made | | | | | | | | | | | 10 | 100 | 100 |

### [Table 12]

**(Table 12)**

| | Shape of dental plate | | | | | | | Arrangement of artificial teeth | | | | | | | Matching rate (%) | Arrangement time (%) | Occlusion adjustment time (%) |
|---|---|---|---|---|---|---|---|---|---|---|---|---|---|---|---|---|---|
| | om | p1d1 | p2d2 | p3d3 | b1d1 | b2d2 | b3d3 | 6^{th} | | 1^{st} | | 3^{rd} | | Other tooth rows | | | |
| | | | | | | | | Position | 16 | Position | 11 | Position | 13 | | | | |
| Manufacturing Example 4 | 0.85 | 0.22 | 0.24 | 0.24 | 0.27 | 0.30 | 0.32 | 0 | 0.16 | 0.84 | 0.16 | 0 | 0.16 | Whole teeth row | 90 | 0 | 40 |
| Manufacturing Example 5 | 0.85 | 0.22 | 0.24 | 0.24 | 0.27 | 0.30 | 0.32 | 0 | 0.12 | 0.84 | 0.12 | 0 | 0.12 | Whole teeth row | 90 | 0 | 50 |
| Manufacturing Example 6 | 0.85 | 0.22 | 0.24 | 0.24 | 0.27 | 0.30 | 0.32 | 0 | 0.20 | 0.84 | 0.20 | 0 | 0.20 | Whole teeth row | 90 | 0 | 50 |
| Comparative Manufacturing Example 5 | 0.85 | 0.22 | 0.24 | 0.24 | 0.27 | 0.30 | 0.32 | 0 | 0.08 | 0.84 | 0.08 | 0 | 0.08 | Whole teeth row | 90 | 0 | 100 |
| Comparative Manufacturing Example 6 | 0.85 | 0.22 | 0.24 | 0.24 | 0.27 | 0.30 | 0.32 | 0 | 0.24 | 0.84 | 0.24 | 0 | 0.24 | Whole teeth row | 90 | 0 | 100 |
| Comparative Manufacturing Example 7 | 0.65 | 0.07 | 0.08 | 0.08 | 0.08 | 0.10 | 0.14 | 0 | 0.12 | 0.84 | 0.12 | 0 | 0.12 | Whole teeth row | 5 | 0 | 100 |
| Comparative Manufacturing Example 8 | 1.05 | 0.38 | 0.40 | 0.40 | 0.48 | 0.50 | 0.50 | 0 | 0.12 | 0.84 | 0.12 | 0 | 0.12 | Whole teeth row | 5 | 0 | 100 |
| Reference Manufacturing Example 2 | Custom-made | | | | | | | | | | | | | | 10 | 100 | 100 |

### [Table 13]

**(Table 13)**

| | Upper jaw | Lower jaw | Occlusion degree |
|---|---|---|---|
| Example 180 | Manufacturing Example 1 | Manufacturing Example 4 | 4 |
| Example 181 | Manufacturing Example 2 | Manufacturing Example 5 | 3 |
| Example 182 | Manufacturing Example 3 | Manufacturing Example 6 | 3 |
| Comparative Example 25 | Comparative Manufacturing Example 1 | Comparative Manufacturing Example 5 | 2 |
| Comparative Example 26 | Comparative Manufacturing Example 2 | Comparative Manufacturing Example 6 | 2 |
| Comparative Example 27 | Comparative Manufacturing Example 3 | Comparative Manufacturing Example 7 | 3 |
| Comparative Example 28 | Comparative Manufacturing Example 4 | Comparative Manufacturing Example 8 | 3 |
| Reference Example 2 | Reference Manufacturing Example 1 | Reference Manufacturing Example 2 | 4 |

### (Soft Lining Material, Tissue conditioning Material)

Lining materials used in Examples and Comparative Examples are as follows.

Lining material A; This lining material consists of:
a paste 1 consisting of 60 parts by mass of organopolysiloxane a, and 40 parts by mass of organopolysiloxane b, which are shown in Table 14, 200 ppm of a vinylsiloxane complex of platinum, a structure of which is shown later, and 30 parts by mass of silica, and
a paste 2 consisting of 55 parts by mass of organopolysiloxane a, 37 parts by mass of organopolysiloxane b, 2 parts by mass of organopolysiloxane c, and 6 parts by mass of organopolysiloxane d, which are shown in Table 14, and 30 parts by mass of silica, and was used by mixing upon use. The durometer A hardness is 43, and this lining material corresponds to a soft lining material.

### [Table 14]

**(Table 14)**

| Organopolysiloxane | x | y | R |
|---|---|---|---|
| a | 400 | 0 | -CH=CH₂ |
| b | 1000 | 0 | -CH=CH₂ |
| c | 20 | 20 | -CH₃ |
| d | 10 | 0 | -H |
| 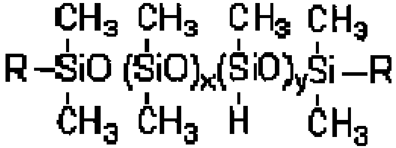 | | | |

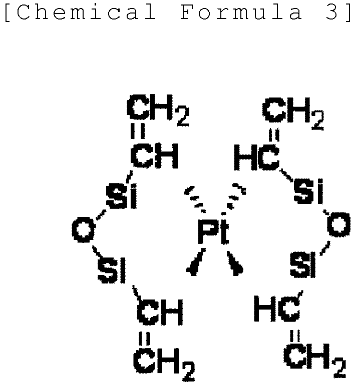

Lining material B; This lining material consists of a powder material consisting of 100 parts by mass of polybutyl methacrylate (glass transition temperature 20°C), and a liquid material consisting of 90 parts by mass of polybutyl acrylate (molecular weight 6000, molecular weight less than 500; 1% by mass or less), and 10 parts by mass of ethanol, and was used by mixing before use. The durometer A hardness is 10, and this lining material corresponds to a tissue conditioning material.

### (Examples 183 to 204, Comparative Examples 29 to 32)

Maxillary standard dental plates having shapes described in Table 15 were fabricated, the lining material was lined, and thereafter, the matching rate, the arrangement time, the occlusion adjustment time, the durometer A hardness, and tanδ were evaluated. The evaluation results are shown in Table 15. In addition, a shape of the denture is bilaterally symmetric.

### (Examples 205 to 226, Comparative Examples 33 to 36)

Mandibular standard dental plates having shapes described in Table 16 were fabricated, the lining material was lined, and thereafter, the matching rate, the arrangement time, the occlusion adjustment time, the durometer A hardness, and tanδ were evaluated. The evaluation results are shown in Table 16. In addition, a shape of the denture is bilaterally symmetric.

### [Table 15]

**(Table 15)**

| | Shape of dental plate | | | | Arrangement of artificial teeth | | | | Lining material | Matching rate (%) | Arrangement time (%) | Occlusion adjustment time (%) | Durometer A hardness | tanδ |
|---|---|---|---|---|---|---|---|---|---|---|---|---|---|---|
| | OM | P1D1 | P2D2 | P3D3 | 6^{th} | 1^{st} | 3^{rd} | Other tooth rows | | | | | | |
| Example 183 | 0.87 | 0.24 | 0.32 | 0.31 | Unarranged | | | | A | 90 | 100 | 100 | 43 | 0.06 |
| Example 184 | 0.87 | 0.24 | 0.32 | 0.31 | | | | | B | 90 | 100 | 100 | 10 | 0.10 |
| Example 185 | 0.98 | 0.24 | 0.32 | 0.31 | | | | | A | 50 | 100 | 100 | 43 | 0.06 |
| Example 186 | 0.76 | 0.24 | 0.32 | 0.31 | | | | | A | 50 | 100 | 100 | 43 | 0.06 |
| Example 187 | 0.87 | 0.36 | 0.45 | 0.45 | | | | | A | 60 | 100 | 100 | 43 | 0.06 |
| Example 188 | 0.87 | 0.11 | 0.19 | 0.16 | | | | | A | 60 | 100 | 100 | 43 | 0.06 |
| Example 189 | 0.87 | 0.08 | 0.15 | 0.10 | | | | | A | 40 | 100 | 100 | 43 | 0.06 |
| Example 190 | 0.87 | 0.42 | 0.51 | 0.51 | | | | | A | 40 | 100 | 100 | 43 | 0.06 |
| Example 191 | 0.87 | 0.24 | 0.32 | 0.31 | 0 | Unarranged | Unarranged | Unarranged | A | 90 | 90 | 90 | 43 | 0.06 |
| Example 192 | 0.87 | 0.24 | 0.32 | 0.31 | 0 | 0.95 | Unarranged | Unarranged | A | 90 | 70 | 80 | 43 | 0.06 |
| Example 193 | 0.87 | 0.24 | 0.32 | 0.31 | 0 | 0.95 | 0 | Unarranged | A | 90 | 50 | 70 | 43 | 0.06 |
| Example 194 | 0.87 | 0.24 | 0.32 | 0.31 | 0.1 | 0.95 | 0 | Unarranged | A | 90 | 50 | 75 | 43 | 0.06 |
| Example 195 | 0.87 | 0.24 | 0.32 | 0.31 | 0.1 | 0.95 | 0 | Unarranged | A | 90 | 50 | 70 | 43 | 0.06 |
| Example 196 | 0.87 | 0.24 | 0.32 | 0.31 | 0 | 0.14 | 0 | Unarranged | A | 90 | 50 | 75 | 43 | 0.06 |
| Example 197 | 0.87 | 0.24 | 0.32 | 0.31 | 0 | 0.76 | 0 | Unarranged | A | 90 | 50 | 75 | 43 | 0.06 |
| Example 198 | 0.87 | 0.24 | 0.32 | 0.31 | 0 | 0.95 | 0.12 | Unarranged | A | 90 | 50 | 75 | 43 | 0.06 |
| Example 199 | 0.87 | 0.24 | 0.32 | 0.31 | 0 | 0.95 | 0.07 | Unarranged | A | 90 | 50 | 75 | 43 | 0.06 |
| Example 200 | 0.87 | 0.24 | 0.32 | 0.31 | 0 | 0.95 | 0 | Whole teeth row | A | 90 | 0 | 50 | 43 | 0.06 |
| Example 201 | 0.87 | 0.24 | 0.32 | 0.31 | 0 | 0.95 | 0 | Whole teeth row | B | 90 | 0 | 50 | 10 | 0.10 |
| Example 202 | 0.87 | 0.24 | 0.32 | 0.31 | 0.2 | Unarranged | Unarranged | Unarranged | A | 90 | 90 | 100 | 43 | 0.06 |
| Example 203 | 0.87 | 0.24 | 0.32 | 0.31 | 0.2 | 0.16 | Unarranged | Unarranged | A | 90 | 70 | 105 | 43 | 0.06 |
| Example 204 | 0.87 | 0.24 | 0.32 | 0.31 | 0.2 | 0.16 | 0.16 | Unarranged | A | 90 | 50 | 115 | 43 | 0.06 |
| Comp. Ex 29 | 0.70 | 0.24 | 0.32 | 0.31 | Unarranged | | | | A | 10 | 100 | 100 | 43 | 0.06 |
| Comp. Ex 30 | 1.05 | 0.24 | 0.32 | 0.31 | | | | | A | 10 | 100 | 100 | 43 | 0.06 |
| Comp. Ex 31 | 0.70 | 0.08 | 0.15 | 0.10 | | | | | A | 5 | 100 | 100 | 43 | 0.06 |
| Comp. Ex 32 | 1.05 | 0.42 | 0.51 | 0.51 | | | | | A | 5 | 100 | 100 | 43 | 0.06 |
| Ref. Ex | Custom-made | | | | | | | | - | 10 | 100 | 100 | - | - |

### [Table 16]

**(Table 16)**

| | Shape of dental plate | | | | | | | Arrangement of artificial teeth | | | | Lining material | Matching rate (%) | Arrangement time (%) | Occlusion adjustment time (%) | Durometer A hardness | tanδ |
|---|---|---|---|---|---|---|---|---|---|---|---|---|---|---|---|---|---|
| | om | p1d1 | p2d2 | p3d3 | d1e1 | d2e2 | d3e3 | 6^{th} | 1^{st} | 3^{rd} | Other tooth rows | | | | | | |
| Example 205 | 0.85 | 0.22 | 0.24 | 0.24 | 0.27 | 0.30 | 0.32 | Unarranged | | | | A | 90 | 100 | 100 | 43 | 0.06 |
| Example 206 | 0.85 | 0.22 | 0.24 | 0.24 | 0.27 | 0.30 | 0.32 | | | | | B | 90 | 100 | 100 | 10 | 0.10 |
| Example 207 | 0.94 | 0.22 | 0.24 | 0.24 | 0.27 | 0.30 | 0.32 | | | | | A | 50 | 100 | 100 | 43 | 0.06 |
| Example 208 | 0.76 | 0.22 | 0.24 | 0.24 | 0.27 | 0.30 | 0.32 | | | | | A | 50 | 100 | 100 | 43 | 0.06 |
| Example 209 | 0.85 | 0.32 | 0.34 | 0.33 | 0.40 | 0.41 | 0.42 | | | | | A | 60 | 100 | 100 | 43 | 0.06 |
| Example 210 | 0.85 | 0.11 | 0.13 | 0.14 | 0.14 | 0.19 | 0.21 | | | | | A | 60 | 100 | 100 | 43 | 0.06 |
| Example 211 | 0.85 | 0.07 | 0.08 | 0.08 | 0.08 | 0.10 | 0.14 | | | | | A | 40 | 100 | 100 | 43 | 0.06 |
| Example 212 | 0.85 | 0.38 | 0.40 | 0.40 | 0.48 | 0.50 | 0.50 | | | | | A | 40 | 100 | 100 | 43 | 0.06 |
| Example 213 | 0.85 | 0.22 | 0.24 | 0.24 | 0.27 | 0.30 | 0.32 | 0 | Unarranged | Unarranged | Unarranged | A | 90 | 90 | 90 | 43 | 0.06 |
| Example 214 | 0.85 | 0.22 | 0.24 | 0.24 | 0.27 | 0.30 | 0.32 | 0 | 0.84 | Unarranged | Unarranged | A | 90 | 70 | 80 | 43 | 0.06 |
| Example 215 | 0.85 | 0.22 | 0.24 | 0.24 | 0.27 | 0.30 | 0.32 | 0 | 0.84 | 0 | Unarranged | A | 90 | 50 | 70 | 43 | 0.06 |
| Example 216 | 0.85 | 0.22 | 0.24 | 0.24 | 0.27 | 0.30 | 0.32 | 0.1 | 0.84 | 0 | Unarranged | A | 90 | 50 | 75 | 43 | 0.06 |
| Example 217 | 0.85 | 0.22 | 0.24 | 0.24 | 0.27 | 0.30 | 0.32 | 0.1 | 0.84 | 0 | Unarranged | A | 90 | 50 | 70 | 43 | 0.06 |
| Example 218 | 0.85 | 0.22 | 0.24 | 0.24 | 0.27 | 0.30 | 0.32 | 0 | 0.94 | 0 | Unarranged | A | 90 | 50 | 75 | 43 | 0.06 |
| Example 219 | 0.85 | 0.22 | 0.24 | 0.24 | 0.27 | 0.30 | 0.32 | 0 | 0.74 | 0 | Unarranged | A | 90 | 50 | 75 | 43 | 0.06 |
| Example 220 | 0.85 | 0.22 | 0.24 | 0.24 | 0.27 | 0.30 | 0.32 | 0 | 0.84 | 0.11 | Unarranged | A | 90 | 50 | 75 | 43 | 0.06 |
| Example 221 | 0.85 | 0.22 | 0.24 | 0.24 | 0.27 | 0.30 | 0.32 | 0 | 0.84 | 0.08 | Unarranged | A | 90 | 50 | 70 | 43 | 0.06 |
| Example 222 | 0.85 | 0.22 | 0.24 | 0.24 | 0.27 | 0.30 | 0.32 | 0 | 0.84 | 0 | Whole teeth row | A | 90 | 0 | 50 | 43 | 0.06 |
| Example 223 | 0.85 | 0.22 | 0.24 | 0.24 | 0.27 | 0.30 | 0.32 | 0 | 0.84 | 0 | Whole teeth row | B | 90 | 0 | 50 | 10 | 0.10 |
| Example 224 | 0.85 | 0.22 | 0.24 | 0.24 | 0.27 | 0.30 | 0.32 | 0.1 | Unarranged | Unarranged | Unarranged | A | 90 | 90 | 100 | 43 | 0.06 |
| Example 225 | 0.85 | 0.22 | 0.24 | 0.24 | 0.27 | 0.30 | 0.32 | 0.1 | 0.70 | Unarranged | Unarranged | A | 90 | 70 | 105 | 43 | 0.06 |
| Example 226 | 0.85 | 0.22 | 0.24 | 0.24 | 0.27 | 0.30 | 0.32 | 0.1 | 0.70 | 0.13 | Unarranged | A | 90 | 50 | 115 | 43 | 0.06 |
| Comp. Ex 33 | 0.65 | 0.22 | 0.24 | 0.24 | 0.27 | 0.30 | 0.32 | Unarranged | | | | A | 10 | 100 | 100 | 43 | 0.06 |
| Comp. Ex 34 | 1.05 | 0.22 | 0.24 | 0.24 | 0.27 | 0.30 | 0.32 | | | | | A | 10 | 100 | 100 | 43 | 0.06 |
| Comp. Ex 35 | 0.65 | 0.07 | 0.08 | 0.08 | 0.08 | 0.10 | 0.14 | | | | | A | 5 | 100 | 100 | 43 | 0.06 |
| Comp. Ex 36 | 1.05 | 0.38 | 0.40 | 0.40 | 0.48 | 0.50 | 0.50 | | | | | A | 5 | 100 | 100 | 43 | 0.06 |
| Ref. Ex | Custom-made | | | | | | | | | | | - | 10 | 100 | 100 | - | - |

### (Hard Lining Material)

Abbreviations of the hard lining materials used in the present Examples are as follows.
R1: Hard lining material consisting of a liquid of 25 parts by mass of acetoacetoxyethyl (meth)acrylate and 25 parts by mass of 1,9-nonanediol di(meth)acrylate, and a powder of 100 parts by mass of polymethyl (meth) acrylate and 2 parts by mass of benzoyl peroxide, and having the Knoop harness of 10.0 Hk.
R2: Hard lining material consisting of a liquid of 25 parts by mass of hexamethylene glycol di(meth)acrylate and 25 parts by mass of 2-methacryloyloxyethyl propionate, and a powder of 100 parts by mass of polyethyl (meth) acrylate and 2 parts by mass of camphorquinone, and having the Knoop hardness of 11.0 Hk.

### (Examples 227 to 247, Comparative Examples 37 to 41)

Maxillary standard dental plates having shapes described in Table 17 were fabricated, and the matching rate, the arrangement time, the occlusion adjustment time, and the durability were evaluated. The evaluation results are shown in Table 17. In addition, a shape of the denture is bilaterally symmetric.

### (Examples 248 to 268, Comparative Examples 42 to 46)

Mandibular standard dental plates having shapes described in Table 18 were fabricated, and the matching rate, the arrangement time, the occlusion adjustment time, and the durability were evaluated. The evaluation results are shown in Table 18. In addition, a shape of the denture is bilaterally symmetric.

### [Table 17]

**(Table 17)**

| | Shape of dental plate | | | | Arrangement of artificial teeth | | | | Lining material | Matching rate (%) | Arrangement time (%) | Occlusion adjustment time (%) | Durability/times | | |
|---|---|---|---|---|---|---|---|---|---|---|---|---|---|---|---|
| | OM | P1D1 | P2D2 | P3D3 | 6^{th} | 1^{st} | 3^{rd} | Other tooth rows | | | | | 300000 | 350000 | 950000 |
| Example 227 | 0.87 | 0.24 | 0.32 | 0.31 | Unarranged | | | | R1 | 90 | 100 | 100 | ○ | ○ | ○ |
| Example 228 | 0.98 | 0.24 | 0.32 | 0.31 | | | | | R1 | 50 | 100 | 100 | ○ | ○ | ○ |
| Example 229 | 0.76 | 0.24 | 0.32 | 0.31 | | | | | R1 | 50 | 100 | 100 | ○ | ○ | ○ |
| Example 230 | 0.87 | 0.36 | 0.45 | 0.45 | | | | | R1 | 60 | 100 | 100 | ○ | ○ | ○ |
| Example 231 | 0.87 | 0.11 | 0.19 | 0.16 | | | | | R1 | 60 | 100 | 100 | ○ | ○ | ○ |
| Example 232 | 0.87 | 0.08 | 0.15 | 0.10 | | | | | R1 | 40 | 100 | 100 | ○ | ○ | ○ |
| Example 233 | 0.87 | 0.42 | 0.51 | 0.51 | | | | | R1 | 40 | 100 | 100 | ○ | ○ | ○ |
| Example 234 | 0.87 | 0.24 | 0.32 | 0.31 | 0 | Unarranged | Unarranged | Unarranged | R1 | 90 | 90 | 90 | ○ | ○ | ○ |
| Example 235 | 0.87 | 0.24 | 0.32 | 0.31 | 0 | 0.95 | Unarranged | Unarranged | R1 | 90 | 70 | 80 | ○ | ○ | ○ |
| Example 236 | 0.87 | 0.24 | 0.32 | 0.31 | 0 | 0.95 | 0 | Unarranged | R1 | 90 | 50 | 70 | ○ | ○ | ○ |
| Example 237 | 0.87 | 0.24 | 0.32 | 0.31 | 0.14 | 0.95 | 0 | Unarranged | R1 | 90 | 50 | 75 | ○ | ○ | ○ |
| Example 238 | 0.87 | 0.24 | 0.32 | 0.31 | 0.08 | 0.95 | 0 | Unarranged | R1 | 90 | 50 | 70 | ○ | ○ | ○ |
| Example 239 | 0.87 | 0.24 | 0.32 | 0.31 | 0 | 0.14 | 0 | Unarranged | R1 | 90 | 50 | 75 | ○ | ○ | ○ |
| Example 240 | 0.87 | 0.24 | 0.32 | 0.31 | 0 | 0.76 | 0 | Unarranged | R1 | 90 | 50 | 75 | ○ | ○ | ○ |
| Example 241 | 0.87 | 0.24 | 0.32 | 0.31 | 0 | 0.95 | 0.12 | Unarranged | R1 | 90 | 50 | 75 | ○ | ○ | ○ |
| Example 242 | 0.87 | 0.24 | 0.32 | 0.31 | 0 | 0.95 | 0.07 | Unarranged | R1 | 90 | 50 | 70 | ○ | ○ | ○ |
| Example 243 | 0.87 | 0.24 | 0.32 | 0.31 | 0 | 0.95 | 0 | Whole teeth row | R1 | 90 | 0 | 50 | ○ | ○ | ○ |
| Example 244 | 0.87 | 0.24 | 0.32 | 0.31 | 0 | 0.95 | 0 | Whole teeth row | R2 | 90 | 0 | 50 | ○ | ○ | ○ |
| Example 245 | 0.87 | 0.24 | 0.32 | 0.31 | 0.16 | Unarranged | Unarranged | Unarranged | R1 | 90 | 90 | 100 | ○ | ○ | ○ |
| Example 246 | 0.87 | 0.24 | 0.32 | 0.31 | 0.16 | 0.70 | Unarranged | Unarranged | R1 | 90 | 70 | 105 | ○ | ○ | ○ |
| Example 247 | 0.87 | 0.24 | 0.32 | 0.31 | 0.16 | 0.70 | 0.14 | Unarranged | R1 | 90 | 50 | 115 | ○ | ○ | ○ |
| Comp. Ex 37 | 0.70 | 0.24 | 0.32 | 0.31 | Unarranged | | | | R1 | 10 | 100 | 100 | ○ | ○ | ○ |
| Comp. Ex 38 | 1.05 | 0.24 | 0.32 | 0.31 | | | | | R1 | 10 | 100 | 100 | ○ | ○ | ○ |
| Comp. Ex 39 | 0.70 | 0.08 | 0.15 | 0.10 | | | | | R1 | 5 | 100 | 100 | ○ | ○ | ○ |
| Comp. Ex 40 | 1.05 | 0.42 | 0.51 | 0.51 | | | | | R1 | 5 | 100 | 100 | ○ | ○ | ○ |
| Comp. Ex 41 | 0.87 | 0.24 | 0.32 | 0.31 | | | | | None | 90 | 100 | 100 | ○ | × | - |
| Ref. Ex | Custom-made | | | | | | | | - | 10 | 100 | 100 | ○ | ○ | ○ |

### [Table 18]

**(Table 18)**

| | Shape of dental plate | | | | | | | Arrangement of artificial teeth | | | | Lining material | Matching rate (%) | Arrangement time (%) | Occlusion adjustment time (%) | Durability/times | | |
|---|---|---|---|---|---|---|---|---|---|---|---|---|---|---|---|---|---|---|
| | om | p1d1 | p2d2 | p3d3 | d1e1 | d2e2 | d3e3 | 6^{th} | 1^{st} | 3^{rd} | Other tooth rows | | | | | 300000 | 350000 | 950000 |
| Example 248 | 0.85 | 0.22 | 0.24 | 0.24 | 0.27 | 0.30 | 0.32 | Unarranged | | | | R1 | 90 | 100 | 50 | ○ | ○ | ○ |
| Example 249 | 0.94 | 0.22 | 0.24 | 0.24 | 0.27 | 0.30 | 0.32 | | | | | R1 | 50 | 100 | 50 | ○ | ○ | ○ |
| Example 250 | 0.76 | 0.22 | 0.24 | 0.24 | 0.27 | 0.30 | 0.32 | | | | | R1 | 50 | 100 | 100 | ○ | ○ | ○ |
| Example 251 | 0.85 | 0.32 | 0.34 | 0.33 | 0.40 | 0.41 | 0.42 | | | | | R1 | 60 | 100 | 100 | ○ | ○ | ○ |
| Example 252 | 0.85 | 0.11 | 0.13 | 0.14 | 0.14 | 0.19 | 0.21 | | | | | R1 | 60 | 100 | 100 | ○ | ○ | ○ |
| Example 253 | 0.85 | 0.07 | 0.08 | 0.08 | 0.08 | 0.10 | 0.14 | | | | | R1 | 40 | 100 | 100 | ○ | ○ | ○ |
| Example 254 | 0.85 | 0.38 | 0.40 | 0.40 | 0.48 | 0.50 | 0.50 | | | | | R1 | 40 | 100 | 100 | ○ | ○ | ○ |
| Example 255 | 0.85 | 0.22 | 0.24 | 0.24 | 0.27 | 0.30 | 0.32 | 0 | Unarranged | Unarranged | Unarranged | R1 | 90 | 90 | 90 | ○ | ○ | ○ |
| Example 256 | 0.85 | 0.22 | 0.24 | 0.24 | 0.27 | 0.30 | 0.32 | 0 | 0.84 | Unarranged | Unarranged | R1 | 90 | 70 | 80 | ○ | ○ | ○ |
| Example 257 | 0.85 | 0.22 | 0.24 | 0.24 | 0.27 | 0.30 | 0.32 | 0 | 0.84 | 0 | Unarranged | R1 | 90 | 50 | 70 | ○ | ○ | ○ |
| Example 258 | 0.85 | 0.22 | 0.24 | 0.24 | 0.27 | 0.30 | 0.32 | 0.12 | 0.84 | 0 | Unarranged | R1 | 90 | 50 | 75 | ○ | ○ | ○ |
| Example 259 | 0.85 | 0.22 | 0.24 | 0.24 | 0.27 | 0.30 | 0.32 | 0.08 | 0.84 | 0 | Unarranged | R1 | 90 | 50 | 70 | ○ | ○ | ○ |
| Example 260 | 0.85 | 0.22 | 0.24 | 0.24 | 0.27 | 0.30 | 0.32 | 0 | 0.94 | 0 | Unarranged | R1 | 90 | 50 | 75 | ○ | ○ | ○ |
| Example 261 | 0.85 | 0.22 | 0.24 | 0.24 | 0.27 | 0.30 | 0.32 | 0 | 0.74 | 0 | Unarranged | R1 | 90 | 50 | 75 | ○ | ○ | ○ |
| Example 262 | 0.85 | 0.22 | 0.24 | 0.24 | 0.27 | 0.30 | 0.32 | 0 | 0.84 | 0.11 | Unarranged | R1 | 90 | 50 | 75 | ○ | ○ | ○ |
| Example 263 | 0.85 | 0.22 | 0.24 | 0.24 | 0.27 | 0.30 | 0.32 | 0 | 0.84 | 0.08 | Unarranged | R1 | 90 | 50 | 70 | ○ | ○ | ○ |
| Example 264 | 0.85 | 0.22 | 0.24 | 0.24 | 0.27 | 0.30 | 0.32 | 0 | 0.84 | 0 | Whole teeth row | R1 | 90 | 0 | 50 | ○ | ○ | ○ |
| Example 265 | 0.85 | 0.22 | 0.24 | 0.24 | 0.27 | 0.30 | 0.32 | 0 | 0.84 | 0 | Whole teeth row | R2 | 90 | 0 | 50 | ○ | ○ | ○ |
| Example 266 | 0.85 | 0.22 | 0.24 | 0.24 | 0.27 | 0.30 | 0.32 | 0.14 | Unarranged | Unarranged | Unarranged | R1 | 90 | 90 | 100 | ○ | ○ | ○ |
| Example 267 | 0.85 | 0.22 | 0.24 | 0.24 | 0.27 | 0.30 | 0.32 | 0.14 | 0.70 | Unarranged | Unarranged | R1 | 90 | 70 | 105 | ○ | ○ | ○ |
| Example 268 | 0.85 | 0.22 | 0.24 | 0.24 | 0.27 | 0.30 | 0.32 | 0.14 | 0.70 | 0.13 | Unarranged | R1 | 90 | 50 | 115 | ○ | ○ | ○ |
| Comp. Ex 42 | 0.65 | 0.22 | 0.24 | 0.24 | 0.27 | 0.30 | 0.32 | Unarranged | | | | R1 | 10 | 100 | 100 | ○ | ○ | ○ |
| Comp. Ex 43 | 1.05 | 0.22 | 0.24 | 0.24 | 0.27 | 0.30 | 0.32 | | | | | R1 | 10 | 100 | 100 | ○ | ○ | ○ |
| Comp. Ex 44 | 0.65 | 0.07 | 0.08 | 0.08 | 0.08 | 0.10 | 0.14 | | | | | R1 | 5 | 100 | 100 | ○ | ○ | ○ |
| Comp. Ex 45 | 1.05 | 0.38 | 0.40 | 0.40 | 0.48 | 0.50 | 0.50 | | | | | R1 | 5 | 100 | 100 | ○ | ○ | ○ |
| Comp. Ex 46 | 0.85 | 0.22 | 0.24 | 0.24 | 0.27 | 0.30 | 0.32 | | | | | None | 90 | 100 | 100 | ○ | × | - |
| Ref. Ex | Custom-made | | | | | | | | | | | - | 10 | 100 | 100 | ○ | ○ | ○ |

### Reference Signs List

11 Maxillary central incisor
11a Interdental part between left maxillary central incisor and right maxillary central incisor
11b Left maxillary central incisor
11d, 11h, 13d, 13h Intersection
11f Right maxillary central incisor
11c, 13c Centrolabial ridge
11e, 11i center of incisal edge
12 Maxillary lateral incisor
12a Mesioincisal angle
13 Maxillary canine
13a, 13e, 13iPointed cuspid
13b Left maxillary canine
13f Right maxillary canine
14 Maxillary first premolar
14a Intersection between central groove and central ridge
15 Maxillary second premolar
15a Intersection between central groove and central ridge
16 Maxillary first molar
16a Central fossa of maxillary first molar
16b Left maxillary first molar
16d Right maxillary first molar
16c, 16e Distobuccal cusp tip
17 Maxillary second molar
17a Central fossa of maxillary second molar
18, 28 Standard planar surface
21 Mandibular central incisor
21a Interdental part between left mandibular central incisor and right mandibular central incisor
21b Left mandibular central incisor
21c, 23c Centrolabial ridge
21d, 21h, 23d, 23h Intersection
21f Right mandibular central incisor
21e, 21i center of incisal edge
21f Right mandibular central incisor
22 Mandibular lateral incisor
22a Mesioincisal angle
23 Mandibular canine
23b Left mandibular canine
23e, 23i Pointed cuspid
23f Right mandibular canine
23a Pointed cuspid
24 Mandibular first premolar
24a Intersection between central groove and central ridge
25 Mandibular second premolar
25a Intersection between central groove and central ridge
26 Mandibular first molar
26a Central fossa of mandibular first molar
26c, 26e Distobuccal cusp tip
26b Left mandibular first molar
26d Right mandibular first molar
27 Mandibular second molar
27a Central fossa of mandibular second molar
50, 52 Left buccal denture border
51, 53 Right buccal denture border
100 Maxillary standard dental plate
100a None-palate maxillary standard dental plate
100b Notched part
101 Artificial teeth
200 Mandibular standard dental plate

## Claims

1. A maxillary standard dental plate (100) **characterized in that**
when a length of a line segment PQ connecting a point P corresponding to the left pterygomaxillary notch and a point Q corresponding to the right pterygomaxillary notch of the plate posterior border is set to be 1,
a length of a line segment OM connecting a point O corresponding to the superior labial frenulum which comes under the median of the labial denture border and a middle point M of the line segment PQ is 0.76 to 0.98, and
when respective points dividing a line segment OP connecting the point O and the point P into four equal portions are defined as a point P1, a point P2, and a point P3 from the point O side, points at which vertical lines from the point P1, the point P2, and the point P3 on the line segment OP are intersected with the left buccal denture border (50) are defined as a point D1, a point D2, and a point D3,
respective points dividing a line segment OQ connecting the point O and the point Q into four equal portions are defined as a point Q1, a point Q2, and a point Q3 from the point O side, points at which vertical lines from the point Q1, the point Q2, and the point Q3 on the line segment OQ are intersected with the right buccal denture border (51) are defined as a point E1, a point E2, and a point E3, and
lengths of a line segment P1D1 and a line segment Q1E1 are 0.11 to 0.36, respectively,
lengths of a line segment P2D2 and a line segment Q2E2 are 0.19 to 0.45, respectively, and
lengths of a line segment P3D3 and a line segment Q3E3 are 0.16 to 0.45, respectively.

2. The maxillary standard dental plate (100) according to claim 1, wherein
when a length of the line segment PQ is set to be 1,
a length of a line segment NM connecting a point N corresponding to the plate posterior border on the line segment OM and the middle point M is 0.05 to 0.10.

3. The maxillary standard dental plate (100a) according to claim 1, wherein the maxillary standard dental plate has a notched part (100b) at a part covering the palate.

4. The maxillary standard dental plate (100) according to claim 1, wherein a guide showing an arrangement position of an artificial tooth is formed.

5. The maxillary standard dental plate (100) according to claim 1, wherein at least one artificial tooth has been arranged in advance.

6. A maxillary standard denture **characterized in that** artificial teeth of a whole teeth row have been arranged on the maxillary standard dental plate as defined in claim 1 in advance.

7. A maxillary denture fabrication kit consisting of:
the maxillary standard dental plate (100) as defined in claim 1; and
a lining material.

8. A maxillary denture fabrication kit consisting of:
the maxillary standard dental plate (100) as defined in claim 1;
connected artificial teeth in which two or more artificial teeth are connected and integrated; and
a lining material.

9. A maxillary denture fabrication kit consisting of:
the maxillary standard denture as defined in claim 6; and
a lining material.

10. The maxillary denture fabrication kit according to any one of claims 7 to 9, wherein said lining material is a lining material having the durometer A hardness of 55 or less.

11. The maxillary denture fabrication kit according to any one of claims 7 to 9, wherein said lining material is a hard lining material.

12. A maxillary denture fabrication method **characterized by** having:
a step of selecting a maxillary standard dental plate (100) in conformity with the size of the oral cavity of a patient, from the maxillary denture fabrication kit as defined in claim 7 or 8;
a step of arranging an artificial tooth on said maxillary standard dental plate (100); and
a step of building up a lining material on said maxillary standard dental plate (100).

13. A maxillary denture fabrication method **characterized by** having:
a step of selecting a maxillary standard denture in conformity with the size of the oral cavity of a patient, from the maxillary denture fabrication kit as defined in claim 9; and
a step of building up a lining material on said maxillary standard denture.

14. A mandibular standard dental plate (200) **characterized in that**
when a length of a line segment pq connecting a point p corresponding to the left retromolar pad and a point q corresponding to the right retromolar pad of the plate posterior border is set to be 1,
a length of a line segment om connecting a point o corresponding to the inferior labial frenulum which comes under the median of the labial denture border and a middle point m of the line segment pq is 0.74 to 0.94,
when respective points dividing a line segment op connecting the point o and the point p into four equal portions are defined as a point p1, a point p2, and a point p3 from the point ο side, points at which vertical lines from the point p1, the point p2, and the point p3 on the line segment op are intersected with the left buccal denture border are defined as a point d1, a point d2, and a point d3,
respective points dividing a line segment oq connecting the point o and the point q into four equal portions are defined as a point q1, a point q2, and a point q3 from the point o side, points at which vertical lines from the point q1, the point q2, and the point q3 on the line segment oq are intersected with the right buccal denture border are defined as a point e1, a point e2 and a point e3, and
lengths of a line segment p1d1 and a line segment q1e1 are 0.11 to 0.32, respectively,
lengths of a line segment p2d2 and a line segment q2e2 are 0.13 to 0.34, respectively, and
lengths of a line segment p3d3 and a line segment q3e3 are 0.14 to 0.33, respectively, and
respective line segment d1p1, d2p2, d3p3, e1q1, e2q2 ,and e3q3 are intersected with the lingual denture border are defined as a point b1, a point b2, point b3, point c1, a point c2 and a point c3, and
lengths of a line segment d1b1 and a line segment e1c1 are 0.14 to 0.40, respectively,
lengths of a line segment d2b2 and a line segment e2c2 are 0.19 to 0.41, respectively, and
lengths of a line segment d3b3 and a line segment e3c3 are 0.21 to 0.42, respectively.

15. The mandibular standard dental plate (200) according to claim 14, wherein a guide showing an arrangement position of an artificial tooth is formed.

16. The mandibular standard dental plate (200) according to claim 14, wherein at least one artificial tooth has been arranged in advance.

17. A mandibular standard denture **characterized in that** artificial teeth of a whole teeth row have been arranged on the mandibular standard dental plate as defined in claim 14 in advance.

18. A mandibular denture fabrication kit consisting of:
the mandibular standard dental plate (200) as defined in claim 14; and
a lining material.

19. A mandibular denture fabrication kit consisting of:
the mandibular standard dental plate (200) as defined in claim 13;
connected artificial teeth in which two or more artificial teeth are connected and integrated; and
a lining material.

20. A mandibular denture fabrication kit consisting of:
the mandibular standard denture as defined in claim 17; and
a lining material.

21. The mandibular denture fabrication kit according to any one of claims 18 to 20, wherein said lining material is a lining material having the durometer A hardness of 55 or less.

22. The mandibular denture fabrication kit according to any one of claims 18 to 20, wherein said lining material is a hard lining material.

23. A mandibular denture fabrication method **characterized by** having:
a step of selecting a mandibular standard dental plate (200) in conformity with the size of the oral cavity of a patient, from the mandibular denture fabrication kit as defined in claim 18 or 19;
a step of arranging an artificial tooth on said mandibular standard dental plate (200); and
a step of building up a lining material on said mandibular standard dental plate (200).

24. A mandibular denture fabrication method **characterized by** having:
a step of selecting a mandibular standard denture in conformity with the size of the oral cavity of a patient, from the mandibular denture fabrication kit as defined in claim 20; and
a step of building up a lining material on said mandibular standard denture.

## Patentansprüche

1. Maxillare Standard-Zahnplatte (100), **dadurch gekennzeichnet, dass**
wenn eine Länge eines Liniensegments PQ, das einen Punkt P, welcher der linken pterygomaxillaren Fissur entspricht, und einen Punkt Q, welcher der rechten pterygomaxillaren Fissur des hinteren Plattenrandes entspricht, verbindet, auf 1 festgelegt ist,
eine Länge eines Liniensegments OM, das einen Punkt O, der dem oberen Lippenbändchen, das unter dem Median der labialen Prothesengrenze liegt, entspricht, mit einem Mittelpunkt M des Liniensegments PQ verbindet, 0,76 bis 0,98 beträgt, und
wenn Punkte, die ein Liniensegment OP, das den Punkt O und den Punkt P verbindet, in vier gleiche Abschnitte teilen, ausgehend von der Seite des Punktes O jeweils als ein Punkt P1, ein Punkt P2 und ein Punkt P3 definiert sind, Punkte, an denen vertikale Linien von dem Punkt P1, dem Punkt P2 und dem Punkt P3 auf dem Liniensegment OP sich mit der linken bukkalen Prothesengrenze (50) schneiden, als ein Punkt D1, ein Punkt D2 und ein Punkt D3 definiert sind,
Punkte, die ein Liniensegment OQ, das den Punkt O und den Punkt Q verbindet, in vier gleiche Abschnitte teilen, ausgehend von der Seite des Punktes O jeweils als ein Punkt Q1, ein Punkt Q2 und ein Punkt Q3 definiert sind, Punkte, an denen vertikale Linien von dem Punkt Q1, dem Punkt Q2 und dem Punkt Q3 auf dem Liniensegment OQ sich mit der rechten bukkalen Prothesengrenze (51) schneiden, als ein Punkt E1, ein Punkt E2 und ein Punkt E3 definiert sind, und
die jeweilige Länge eines Liniensegments P1D1 und eines Liniensegments Q1E1 0,11 bis 0,36 beträgt,
die jeweilige Länge eines Liniensegments P2D2 und eines Liniensegments Q2E2 0,19 bis 0,45 beträgt und
die jeweilige Länge eines Liniensegments P3D3 und eines Liniensegments Q3E3 0,16 bis 0,45 beträgt.

2. Maxillare Standard-Zahnplatte (100) nach Anspruch 1, wobei,
wenn eine Länge des Liniensegments PQ auf 1 festgelegt ist,
eine Länge eines Liniensegments NM, das einen Punkt N, welcher der hinteren Plattengrenze auf dem Liniensegment OM entspricht, mit dem Mittelpunkt M verbindet, 0,05 bis 0,10 beträgt.

3. Maxillare Standard-Zahnplatte (100a) nach Anspruch 1, wobei die maxillare Standard-Zahnplatte einen eingekerbten Teil (100b) an einem den Gaumen bedeckenden Teil aufweist.

4. Maxillare Standard-Zahnplatte (100) nach Anspruch 1, wobei eine Führung, die eine Anordnungsposition eines künstlichen Zahnes zeigt, ausgebildet ist.

5. Maxillare Standard-Zahnplatte (100) nach Anspruch 1, wobei mindestens ein künstlicher Zahn im Voraus angeordnet wurde.

6. Maxillare Standard-Zahnplatte, **dadurch gekennzeichnet, dass** künstliche Zähne einer ganzen Zahnreihe auf der maxillaren Standard-Zahnplatte, wie in Anspruch 1 definiert, im Voraus angeordnet wurden.

7. Maxillares Zahnprothesenherstellungskit, bestehend aus:
der maxillaren Standard-Zahnplatte (100) wie in Anspruch 1 definiert; und
einem Auskleidungsmaterial.

8. Maxillares Zahnprothesenherstellungskit, bestehend aus:
der maxillaren Standard-Zahnplatte (100) wie in Anspruch 1 definiert;
verbundene künstliche Zähne, bei denen zwei oder mehr künstliche Zähne verbunden und integriert sind; und
einem Auskleidungsmaterial.

9. Maxillares Zahnprothesenherstellungskit, bestehend aus:
der maxillaren Standard-Zahnprothese wie in Anspruch 6 definiert; und
einem Auskleidungsmaterial.

10. Maxillares Zahnprothesenherstellungskit nach einem der Ansprüche 7 bis 9, wobei das Auskleidungsmaterial ein Auskleidungsmaterial mit einer Durometer A-Härte von 55 oder weniger ist.

11. Maxillares Zahnprothesenherstellungskit nach einem der Ansprüche 7 bis 9, wobei das Auskleidungsmaterial ein hartes Auskleidungsmaterial ist.

12. Maxillares Zahnprothesenherstellungsverfahren, **gekennzeichnet durch**:
einen Schritt zum Auswählen einer maxillaren Standard-Zahnplatte (100) aus dem maxillaren Zahnprothesenherstellungskit wie in Anspruch 7 oder 8 definiert entsprechend der Größe der Mundhöhle eines Patienten;
einen Schritt zum Anordnen eines künstlichen Zahnes auf der maxillaren Standard-Zahnplatte (100); und
einen Schritt zum Aufbauen eines Auskleidungsmaterials auf der maxillaren Standard-Zahnplatte (100).

13. Maxillares Zahnprothesenherstellungsverfahren, **gekennzeichnet durch**
einen Schritt zum Auswählen einer maxillaren Standard-Zahnprothese aus dem maxillaren Zahnprothesenherstellungskit wie in Anspruch 9 definiert entsprechend der Größe der Mundhöhle eines Patienten; und
einen Schritt zum Aufbauen eines Auskleidungsmaterials auf der maxillaren Standard-Zahnprothese.

14. Mandibulare Standard-Zahnplatte (200), **dadurch gekennzeichnet, dass**
wenn die Länge eines Liniensegments pq, das einen Punkt p, der dem linken retromolaren Pad entspricht, mit einem Punkt q, der dem rechten retromolaren Pad der hinteren Plattengrenze entspricht, verbindet, auf 1 festgelegt ist,
eine Länge eines Liniensegments om, das einen Punkt o, der dem unteren Lippenbändchen, das unter dem Median der labialen Prothesengrenze liegt, entspricht, mit einem Mittelpunkt m des Liniensegments pq verbindet, 0,74 bis 0,94 beträgt,
wenn Punkte, die ein Liniensegment op, das den Punkt o und den Punkt p verbindet, in vier gleiche Abschnitte teilen, ausgehend von der Seite des Punktes o jeweils als ein Punkt p1, ein Punkt p2 und ein Punkt p3 definiert sind, Punkte, an denen vertikale Linien von dem Punkt p1, dem Punkt p2 und dem Punkt p3 auf dem Liniensegment op sich mit der linken bukkalen Prothesengrenze schneiden, als ein Punkt d1, ein Punkt d2 und ein Punkt d3 definiert sind,
Punkte, die ein Liniensegment oq, das den Punkt o und den Punkt q verbindet, in vier gleiche Abschnitte teilen, ausgehend von der Seite des Punktes o jeweils als ein Punkt q1, ein Punkt q2 und ein Punkt q3 definiert sind, Punkte, an denen vertikale Linien von dem Punkt q1, dem Punkt q2 und dem Punkt q3 auf dem Liniensegment oq sich mit der rechten bukkalen Prothesengrenze schneiden, als ein Punkt e1, ein Punkt e2 und ein Punkt e3 definiert sind, und
die jeweilige Länge eines Liniensegments p1d1 und eines Liniensegments q1e1 0,11 bis 0,32 beträgt,
die jeweilige Länge eines Liniensegments p2d2 und eines Liniensegments q2e2 0,13 bis 0,34 beträgt und
die jeweilige Länge eines Liniensegments p3d3 und eines Liniensegments q3e3 0,14 bis 0,33 beträgt und
die Liniensegmente d1p1, d2p2, d3p3, e1q1, e2q2 und e3q3 jeweils sich mit der lingualen Prothesengrenze schneiden und als ein Punkt b1, ein Punkt b2, ein Punkt b3, ein Punkt c1, ein Punkt c2 und ein Punkt c3 definiert sind, und
die jeweilige Länge eines Liniensegments d1b1 und eines Liniensegments e1c1 0,14 bis 0,40 beträgt,
die jeweilige Länge eines Liniensegments d2b2 und eines Liniensegments e2c2 0,19 bis 0,41 beträgt und
die jeweilige Länge eines Liniensegments d3b3 und eines Liniensegments e3c3 0,21 bis 0,42 beträgt.

15. Mandibulare Standard-Zahnplatte (200) nach Anspruch 14, wobei eine Führung, die eine Anordnungsposition eines künstlichen Zahnes zeigt, ausgebildet ist.

16. Mandibulare Standard-Zahnplatte (200) nach Anspruch 14, wobei mindestens ein künstlicher Zahn im Voraus angeordnet wurde.

17. Mandibulare Standard-Zahnprothese, **dadurch gekennzeichnet, dass** künstliche Zähne einer ganzen Zahnreihe auf der mandibularen Standard-Zahnplatte, wie in Anspruch 14 definiert, im Voraus angeordnet wurden.

18. Mandibulares Zahnprothesenherstellungskit, bestehend aus:
der mandibularen Standard-Zahnplatte (200) wie in Anspruch 14 definiert; und
einem Auskleidungsmaterial.

19. Mandibulares Zahnprothesenherstellungskit, bestehend aus:
der mandibularen Standard-Zahnplatte (200) wie in Anspruch 13 definiert;
verbundenen künstlichen Zähnen, in denen zwei oder mehr künstliche Zähne verbunden und integriert sind; und
einem Auskleidungsmaterial.

20. Mandibulares Zahnprothesenherstellungskit, bestehend aus:
der mandibularen Standard-Zahnprothese wie in Anspruch 17 definiert; und einem Auskleidungsmaterial.

21. Mandibulares Zahnprothesenherstellungskit nach einem der Ansprüche 18 bis 20, wobei das Auskleidungsmaterial ein Auskleidungsmaterial mit einer Durometer A-Härte von 55 oder weniger ist.

22. Manibulares Zahnprothesenherstellungskit nach einem der Ansprüche 18 bis 20, wobei das Auskleidungsmaterial ein hartes Auskleidungsmaterial ist.

23. Manibulares Zahnprothesenherstellungsverfahren, **gekennzeichnet durch**
einen Schritt zum Auswählen einer manibularen Standard-Zahnplatte (200) aus dem manibularen Zahnprothesenherstellungskit wie in Anspruch 18 oder 19 definiert entsprechend der Größe der Mundhöhle eines Patienten;
einen Schritt zum Anordnen eines künstlichen Zahnes auf der manibularen Standard-Zahnplatte (200); und
einen Schritt zum Aufbauen eines Auskleidungsmaterials auf der manibularen Standard-Zahnplatte (200).

24. Manibulares Zahnprothesenherstellungsverfahren, **gekennzeichnet durch**
einen Schritt zum Auswählen einer manibularen Standard-Zahnprothese aus dem manibularen Zahnprothesenherstellungskit wie in Anspruch 20 definiert entsprechend der Größe der Mundhöhle eines Patienten; und
einen Schritt zum Aufbauen eines Auskleidungsmaterials auf der manibularen Standard-Zahnprothese.

## Revendications

1. Plaque dentaire standard maxillaire (100), **caractérisée en ce que**
lorsqu'une longueur d'un segment de ligne PQ connectant un point P correspondant à la fosse ptérygo-maxillaire gauche et un point Q correspondant à la fosse ptérygo-maxillaire droite de la bordure postérieure de la plaque est fixée à 1,
une longueur d'un segment de ligne OM connectant un point O correspondant au frein labial supérieur qui vient sous la médiane de la bordure de prothèse dentaire labiale et un point milieu M du segment de ligne PQ est de 0,76 à 0,98, et
lorsque des points divisant un segment de ligne OP connectant le point O et le point P en quatre portions égales sont respectivement définis comme un point P1, un point P2 et un point P3 du côté du point O, les points auxquels des lignes verticales provenant du point P1, d point P2 et du point P3 sur le segment de ligne OP sont intersectées avec la bordure de prothèse dentaire buccale gauche (50) sont définis comme un point D1, un point D2 et un point D3,
des points divisant un segment de ligne OQ connectant le point O et le point Q en quatre portions égales sont respectivement définis comme un point Q1, un point Q2 et un point Q3 du côté du point O, les points auxquels des lignes verticales provenant du point Q1, d point Q2 et du point Q3 sur le segment de ligne OQ sont intersectées avec la bordure de prothèse dentaire buccale droite (51) sont définis comme un point E1, un point E2 et un point E3, et
des longueurs d'un segment de ligne P1D1 et d'un segment de ligne Q1E1 sont de 0,11 à 0,36, respectivement,
des longueurs d'un segment de ligne P2D2 et d'un segment de ligne Q2E2 sont de 0,19 à 0,45, respectivement, et
des longueurs d'un segment de ligne P3D3 et d'un segment de ligne Q3E3 sont de 0,16 à 0,45, respectivement.

2. Plaque dentaire standard maxillaire (100) selon la revendication 1, où
lorsqu'une longueur du segment de ligne PQ est fixée à 1,
une longueur d'un segment de ligne NM connectant un point N correspondant à la bordure postérieure de la plaque sur le segment de ligne OM et le point milieu M est de 0,05 à 0,10.

3. Plaque dentaire standard maxillaire (100a) selon la revendication 1, la plaque dentaire standard maxillaire ayant une partie entaillée (100b) au niveau d'une partie couvrant le palais.

4. Plaque dentaire standard maxillaire (100) selon la revendication 1, un guide montrant une position d'agencement d'une dent artificielle étant formé.

5. Plaque dentaire standard maxillaire (100) selon la revendication 1, au moins une dent artificielle ayant été agencée à l'avance.

6. Prothèse dentaire standard maxillaire, **caractérisée en ce que** des dents artificielles d'une rangée de dents complètes ont été agencées sur la plaque dentaire standard maxillaire telle que définie dans la revendication 1 à l'avance.

7. Kit de fabrication de prothèse dentaire maxillaire constitué par :
la plaque dentaire standard maxillaire (100) telle que définie dans la revendication 1 ; et
un matériau de revêtement.

8. Kit de fabrication de prothèse dentaire maxillaire constitué par :
la plaque dentaire standard maxillaire (100) telle que définie dans la revendication 1 ;
des dents artificielles connectées parmi lesquelles deux ou davantage de dents artificielles sont connectées et intégrées ; et
un matériau de revêtement.

9. Kit de fabrication de prothèse dentaire maxillaire constitué par :
la prothèse dentaire standard maxillaire telle que définie dans la revendication 6 ; et
un matériau de revêtement.

10. Kit de fabrication de prothèse dentaire maxillaire selon l'une quelconque des revendications 7 à 9, ledit matériau de revêtement étant un matériau de revêtement ayant la dureté A au duromètre de 55 ou moins.

11. Kit de fabrication de prothèse dentaire maxillaire selon l'une quelconque des revendications 7 à 9, ledit matériau de revêtement étant un matériau de revêtement dur.

12. Procédé de fabrication de prothèse dentaire maxillaire **caractérisé en ce qu'**il comprend :
une étape de sélection d'une plaque dentaire standard maxillaire (100) conforme à la taille de la cavité orale d'un patient, à partir du kit de fabrication de prothèse dentaire maxillaire tel que défini dans la revendication 7 ou 8 ;
une étape d'agencement d'une dent artificielle sur ladite plaque dentaire standard maxillaire (100) ; et
une étape de formation d'un matériau de revêtement sur ladite plaque dentaire standard maxillaire (100).

13. Procédé de fabrication de prothèse dentaire maxillaire **caractérisé en ce qu'**il comprend :
une étape de sélection d'une prothèse dentaire standard maxillaire conforme à la taille de la cavité orale d'un patient, à partir du kit de fabrication de prothèse dentaire maxillaire tel que défini dans la revendication 9 ; et
une étape de formation d'un matériau de revêtement sur ladite prothèse dentaire standard maxillaire.

14. Plaque dentaire standard mandibulaire (200), **caractérisée en ce que**
lorsqu'une longueur d'un segment de ligne pq connectant un point p correspondant à la papille rétromolaire gauche et un point q correspondant à la papille rétromolaire droite de la bordure postérieure de la plaque est fixée à 1,
une longueur d'un segment de ligne om connectant un point o correspondant au frein labial inférieur qui vient sous la médiane de la bordure de prothèse dentaire labiale et un point milieu m du segment de ligne pq est de 0,74 à 0,94,
lorsque des points divisant un segment de ligne op connectant le point o et le point p en quatre portions égales sont respectivement définis comme un point p1, un point p2 et un point p3 du côté du point o, les points auxquels des lignes verticales provenant du point p1, du point p2 et du point p3 sur le segment de ligne op sont intersectées avec la bordure de prothèse dentaire buccale gauche sont définis comme un point d1, un point d2 et un point d3,
des points divisant un segment de ligne oq connectant le point o et le point q en quatre portions égales sont respectivement définis comme un point q1, un point q2 et un point q3 du côté du point o, les points auxquels des lignes verticales provenant du point q1, du point q2 et du point q3 sur le segment de ligne oq sont intersectées avec la bordure de prothèse dentaire buccale droite sont définis comme un point e1, un point e2 et un point e3, et
des longueurs d'un segment de ligne p1d1 and et d'un segment de ligne q1e1 sont de 0,11 à 0,32, respectivement,
des longueurs d'un segment de ligne p2d2 et d'un segment de ligne q2e2 sont de 0,13 à 0,34, respectivement, et
des longueurs d'un segment de ligne p3d3 et d'un segment de ligne q3e3 sont de 0,14 à 0,33, respectivement, et
des segments de ligne d1p1, d2p2, d3p3, e1q1, e2q2 et e3q3 sont respectivement intersectés avec la bordure de prothèse dentaire linguale et définis comme un point b1, un point b2, un point b3, un point c1, un point c2 et un point c3, et
des longueurs d'un segment de ligne d1b1 et d'un segment de ligne e1c1 sont de 0,14 à 0,40, respectivement,
des longueurs d'un segment de ligne d2b2 et d'un segment de ligne e2c2 sont de 0,19 à 0,41, respectivement, et
des longueurs d'un segment de ligne d3b3 et d'un segment de ligne e3c3 sont de 0,21 à 0,42, respectivement.

15. Plaque dentaire standard mandibulaire (200) selon la revendication 14, un guide montrant une position d'agencement d'une dent artificielle étant formé.

16. Plaque dentaire standard mandibulaire (200) selon la revendication 14, au moins une dent artificielle ayant été agencée à l'avance.

17. Prothèse dentaire standard mandibulaire, **caractérisée en ce que** des dents artificielles d'une rangée de dents complètes ont été agencées sur la plaque dentaire standard mandibulaire telle que définie dans la revendication 14 à l'avance.

18. Kit de fabrication de prothèse dentaire mandibulaire constitué par :
la plaque dentaire standard mandibulaire (200) telle que définie dans la revendication 14 ; et un matériau de revêtement.

19. Kit de fabrication de prothèse dentaire mandibulaire constitué par :
la plaque dentaire standard mandibulaire (200) telle que définie dans la revendication 13 ; des dents artificielles connectées parmi lesquelles deux ou davantage de dents artificielles sont connectées et intégrées ; et
un matériau de revêtement.

20. Kit de fabrication de prothèse dentaire mandibulaire constitué par :
la prothèse dentaire standard mandibulaire telle que définie dans la revendication 17 ; et un matériau de revêtement.

21. Kit de fabrication de prothèse dentaire mandibulaire selon l'une quelconque des revendications 18 à 20, ledit matériau de revêtement étant un matériau de revêtement ayant la dureté A au duromètre de 55 ou moins.

22. Kit de fabrication de prothèse dentaire mandibulaire selon l'une quelconque des revendications 18 à 20, ledit matériau de revêtement étant un matériau de revêtement dur.

23. Procédé de fabrication de prothèse dentaire mandibulaire **caractérisé en ce qu'**il comprend :
une étape de sélection d'une plaque dentaire standard mandibulaire (200) conforme à la taille de la cavité orale d'un patient, à partir du kit de fabrication de prothèse dentaire mandibulaire tel que défini dans la revendication 18 ou 19 ;
une étape d'agencement d'une dent artificielle sur ladite plaque dentaire standard mandibulaire (200) ; et
une étape de formation d'un matériau de revêtement sur ladite plaque dentaire standard mandibulaire(200).

24. Procédé de fabrication de prothèse dentaire mandibulaire **caractérisé en ce qu'**il comprend :
une étape de sélection d'une prothèse dentaire standard mandibulaire conforme à la taille de la cavité orale d'un patient, à partir du kit de fabrication de prothèse dentaire mandibulaire tel que défini dans la revendication 20 ; et
une étape de formation d'un matériau de revêtement sur ladite prothèse dentaire standard mandibulaire.
